# EUROPÄISCHE PATENTSCHRIFT

(11) **EP 1 395 834 B1**
(45) Veröffentlichungstag und Bekanntmachung des Hinweises auf die Patenterteilung: **06.06.2007**
(21) Anmeldenummer: 02751022.1
(22) Anmeldetag: 13.06.2002
(51) Int. Cl.: G01N 33/94, A61P 29/00

(54) **SCREENINGVERFAHREN MIT BNPI UND DNPI**
SCREENING METHOD WHICH USES BNPI AND DNPI
PROCEDE DE CRIBLAGE A L'AIDE DES PROTEINES BNPI ET DNPI

(30) Priorität: 13.06.2001 DE 10128541
(43) Veröffentlichungstag der Anmeldung: 10.03.2004
(73) Patentinhaber: Grünenthal GmbH, 52078 Aachen (DE)
(72) Erfinder: WEIHE, Eberhard, 35037 Marburg (DE); SCHÄFER, Martin, K.-H., 35041 Marburg (DE); BIELLER, Annette, 35091 Cölbe-Bürgeln (DE)
(74) Vertreter: Hiebl, Inge Elisabeth
(86) Internationale Anmeldenummer: PCT/EP2002/006484
(87) Internationale Veröffentlichungsnummer: WO 2002/101394

(56) Entgegenhaltungen:
- WO-A-96/34288
- WO-A-98/26054
- AIHARA YASUO ET AL: "Molecular cloning of a novel brain-type Na+-dependent inorganic phosphate cotransporter." JOURNAL OF NEUROCHEMISTRY, Bd. 74, Nr. 6, Juni 2000 (2000-06), Seiten 2622-2625, XP001151843 June, 2000 ISSN: 0022-3042 in der Anmeldung erwähnt -& DATABASE EMBL [Online] "Brain-specific Na-dependent inorganic phosphate cotransporter, BNPI" Database accession no. q9p2u7 XP002240796 -& DATABASE EMBL [Online] "Differentiation-associated Na-dependent inorganic phosphate cotransporter" Database accession no. q9p2u8 XP002240797
- BAI LIQUN ET AL: "Molecular and functional analysis of a novel neuronal vesicular glutamate transporter." JOURNAL OF BIOLOGICAL CHEMISTRY, Bd. 276, Nr. 39, 28. September 2001 (2001-09-28), Seiten 36764-36769, XP001151946 ISSN: 0021-9258 -& DATABASE EMBL [Online] "Vesicular glutamate transporter 2" Database accession no. q920B7 XP002240798
- LI ET AL: THE JOURNAL OF COMPARATIVE NEUROLOGY, Bd. 457, Nr. 3, 22. Januar 2003 (2003-01-22), Seiten 236-249,
- HWANG ET AL: JOURNAL OF NEUROCYTOLOGY, Bd. 33, 2004, Seiten 321-329,

### SEQUENZPROTOKOLL

<110> GRÜNENTHAL GmbH
<120> Screeningverfahren mit BNPI und DNPI
<130> GRA 3069-PCT
<140> PCT/EP 02/06484
   <141> 2002-06-13
<150> DE10128541.8
   <151> 2001-06-13
<160> 14
<170> Patent In Ver. 2.1
<210> 1
   <211> 2366
   <212> DNA
   <213> Homo Sapiens
<400> 1
<210> 2
   <211> 560
   <212> PRT
   <213> Homo Sapiens
<400> 2
   Met Glu Phe Arg Gln Glu Glu Phe Arg Lys Leu Ala Gly Arg Ala Leu
    1               5                  10                  15
   Gly Lys Leu His Arg Leu Leu Glu Lys Arg Gln Glu Gly Ala Glu Thr
               20                  25                  30
   Leu Glu Leu Ser Ala Asp Gly Arg Pro Val Thr Thr Gln Thr Arg Asp
           35                  40                  45
   Pro Pro Val Val Asp Cys Thr Cys Phe Gly Leu Pro Arg Arg Tyr Ile
       50                  55                  60
   Ile Ala Ile Met Ser Gly Leu Gly Phe Cys Ile Ser Phe Gly Ile Arg
   65                  70                  75                  80
   Cys Asn Leu Gly Val Ala Ile Val Ser Met Val Asn Asn Ser Thr Thr
                   85                  90                  95
   His Arg Gly Gly His Val Val Val Gln Lys Ala Gln Phe Ser Trp Asp
               100                 105                 110
   Pro Glu Thr Val Gly Leu Ile His Gly Ser Phe Phe Trp Gly Tyr Ile
           115                 120                 125
   Val Thr Gln Ile Pro Gly Gly Phe Ile Cys Gln Lys Phe Ala Ala Asn
   130 135 140
   Arg Val Phe Gly Phe Ala Ile Val Ala Thr Ser Thr Leu Asn Met Leu
   145 150 155 160
   Ile Pro Ser Ala Ala Arg Val His Tyr Gly Cys Val Ile Phe Val Arg
   165 170 175
   Ile Leu Gln Gly Leu Val Glu Gly Val Thr Tyr Pro Ala Cys His Gly
   180 185 190
   Ile Trp Ser Lys Trp Ala Pro Pro Leu Glu Arg Ser Arg Leu Ala Thr
   195 200 205
   Thr Ala Phe Cys Gly Ser Tyr Ala Gly Ala Val Val Ala Met Pro Leu
   210 215 220
   Ala Gly Val Leu Val Gln Tyr Ser Gly Trp Ser Ser Val Phe Tyr Val
   225 230 235 240
   Tyr Gly Ser Phe Gly Ile Phe Trp Tyr Leu Phe Trp Leu Leu Val Ser
   245 250 255
   Tyr Glu Ser Pro Ala Leu His Pro Ser Ile Ser Glu Glu Glu Arg Lys
   260 265 270
   Tyr Ile Glu Asp Ala Ile Gly Glu Ser Ala Lys Leu Met Asn Pro Leu
   275 280 285
   Thr Lys Phe Ser Thr Pro Trp Arg Arg Phe Phe Thr Ser Met Pro Val
   290 295 300
   Tyr Ala Ile Ile Val Ala Asn Phe Cys Arg Ser Trp Thr Phe Tyr Leu
   305 310 315 320
   Leu Leu Ile Ser Gln Pro Ala Tyr Phe Glu Glu Val Phe Gly Phe Glu
   325 330 335
   Ile Ser Lys Val Gly Leu Val Ser Ala Leu Pro His Leu Val Met Thr
   340 345 350
   Ile Ile Val Pro Ile Gly Gly Gln Ile Ala Asp Phe Leu Arg Ser Arg
   355 360 365
   Arg Ile Met Ser Thr Thr Asn Val Arg Lys Leu Met Asn Cys Gly Gly
   370 375 380
   Phe Gly Met Glu Ala Thr Leu Leu Leu Val Val Gly Tyr Ser His Ser
   385 390 395 400
   Lys Gly Val Ala Ile Ser Phe Leu Val Leu Ala Val Gly Phe Ser Gly
   405 410 415
   Phe Ala Ile Ser Gly Phe Asn Val Asn His Leu Asp Ile Ala Pro Arg
   420 425 430
   Tyr Ala Ser Ile Leu Met Gly Ile Ser Asn Gly Val Gly Thr Leu Ser
   435 440 445
   Gly Met Val Cys Pro Ile Ile Val Gly Ala Met Thr Lys His Lys Thr
   450 455 460
   Arg Glu Glu Trp Gln Tyr Val Phe Leu Ile Ala Ser Leu Val His Tyr
   465 470 475 480
   Gly Gly Val Ile Phe Tyr Gly Val Phe Ala Ser Gly Glu Lys Gln Pro
   485 490 495
   Trp Ala Glu Pro Glu Glu Met Ser Glu Glu Lys Cys Gly Phe Val Gly
   500 505 510
   His Asp Gln Leu Ala Gly Ser Asp Asp Ser Glu Met Glu Asp Glu Ala
   515 520 525
   Glu Pro Pro Gly Ala Pro Pro Ala Pro Pro Pro Ser Tyr Gly Ala Thr
   530 535 540
   His Ser Thr Phe Gln Pro Pro Arg Pro Pro Pro Pro Val Arg Asp Tyr
   545 550 555 560
<210> 3
   <211> 2716
   <212> DNA
   <213> Homo Sapiens
<400> 3
   cgataagctt gatatcgaat tccggactct tgctcgggcg ccttaacccg gcgttcggtt 60
   catcccgcag cgccagttct gcttaccaaa agtggcccac taggcactcg cattccacgc 120
   ccggctccac gccagcgagc cgggcttctt acccatttaa agtttgagaa taggttgaga 180
   tcgtttcggc cccaagacct ctaatcattc gctttaccgg ataaaactgc gtggcggggg 240
   tgcgtcgggt ctgcgagagc gccagctatc ctgagggaaa cttcggaggg aaccagctac 300
   tagatggttc gattagtctt tcgcccctat acccaggtcg gacgaccgat ttgcacgtca 360
   ggaccgctac ggacctccac cagagtttcc tctggcttcg ccctgcccag gcgatcggcg 420
   ggggggaccc gcggggtgac cggcggcagg agccgccacc atggagttcc gccaggagga 480
   gtttcggaag ctagcgggtc gtgctctcgg gaagctgcac cgccttctgg agaagcggca 540
   ggaaggcgcg gagacgctgg agctgagtgc ggatgggcgc ccggtgacca cgcagacccg 600
   ggacccgccg gtggtggact gcacctgctt cggcctccct cgccgctaca ttatcgccat 660
   catgagtggt ctgggcttct gcatcagctt tggcatccgc tgcaacctgg gcgtggccat 720
   cgtctccatg gtcaataaca gcacgaccca ccgcgggggc cacgtggtgg tgcagaaagc 780
   ccagttcagc tgggatccag agactgtcgg cctcatacac ggctcctttt tctggggcta 840
   cattgtcact cagattccag gaggatttat ctgtcaaaaa tttgcagcca acagagtttt 900
   cggctttgct attgtggcaa catccactct aaacatgctg atcccctcag ctgcccgcgt 960
   ccactatggc tgtgtcatct tcgtgaggat cctgcagggg ttggtagagg gggtcacata 1020
   ccccgcctgc catgggatct ggagcaaatg ggccccaccc ttagaacgga gtcgcctggc 1080
   gacgacagcc ttttgtggtt cctatgctgg ggcggtggtc gcgatgcccc tcgccggggt 1140
   ccttgtgcag tactcaggat ggagctctgt tttctacgtc tacggcagct tcgggatctt 1200
   ctggtacctg ttctggctgc tcgtctccta cgagtccccc gcgctgcacc ccagcatctc 1260
   ggaggaggag cgcaagtaca tcgaggacgc catcggagag agcgcgaaac tcatgaaccc 1320
   cctcacgaag tttagcactc cctggcggcg cttcttcacg tctatgccag tctatgccat 1380
   catcgtggcc aacttctgcc gcagctggac gttctacctg ctgctcatct cccagcccga 1440
   ctacttcgaa gaagtgttcg gcttcgagat cagcaaggta ggcctggtgt ccgcgctgcc 1500
   ccacctggtc atgaccatca tcgtgcccat cggcggccag atcgcggact tcctgcggag 1560
   ccgccgcatc atgtccacca ccaacgtgcg caagttgatg aactgcggag gcttcggcat 1620
   ggaagccacg ctgctgttgg tggtcggcta ctcgcactcc aagggcgtgg ccatctcctt 1680
   cctggtccta gccgtgggct tcagcggctt cgccatctct gggttcaacg tgaaccacct 1740
   ggacatagcc ccgcgctacg ccagcatcct catgggcatc tccaacggcg tgggcacact 1800
   gtcgggcatg gtgtgcccca tcatcgtggg ggccatgact aagcacaaga ctcgggagga 1860
   gtggcagtac gtgttcctaa ttgcctccct ggtgcactat ggaggtgtca tcttctacgg 1920
   ggtctttgct tctggagaga agcagccgtg ggcagagcct gaggagatga gcgaggagaa 1980
   gtgtggcttc gttggccatg accagctggc tggcagtgac gacagcgaaa tggaggatga 2040
   ggctgagccc ccgggggcac cccctgcacc cccgccctcc tatggggcca cacacagcac 2100
   atttcagccc cccaggcccc caccccctgt ccgggactac tgaccatgtg cctcccactg 2160
   aatggcagtt tccaggacct ccattccact catctctggc ctgagtgaca gtgtcaagga 2220
   accctgctcc tctctgtcct gcctcaggcc taagaagcac tctcccttgt tcccagtgct 2280
   gtcaaatcct ctttccttcc caattgcctc tcaggggtag tgaagctgca gactgacagt 2340
   ttcaaggata cccaaattcc cctaaaggtt ccctctccac ccgttctgcc tcagtggttt 2400
   caaatctctc ctttcagggc tttatttgaa tggacagttc gacctcttac tctctcttgt 2460
   ggttttgagg cacccacacc ccccgctttc ctttatctcc agggactctc aggctaacct 2520
   ttgagatcac tcagctccca tctcctttca gaaaaattca aggtcctcct ctagaagttt 2580
   caaatctctc ccaactctgt tctgcatctt ccagattggt ttaaccaatt actcgtcccc 2640
   gccattccag ggattgattc tcaccagcgt ttctgatgga aaatggcggg aattcctgca 2700
   gcccggggga tccact                                                 2716
<210> 4
   <211> 560
   <212> PRT
   <213> Homo Sapiens
<400> 4
   Met Glu Phe Arg Gln Glu Glu Phe Arg Lys Leu Ala Gly Arg Ala Leu
   1 5 10 15
   Gly Lys Leu His Arg Leu Leu Glu Lys Arg Gln Glu Gly Ala Glu Thr
   20 25 30
   Leu Glu Leu Ser Ala Asp Gly Arg Pro Val Thr Thr Gln Thr Arg Asp
   35 40 45
   Pro Pro Val Val Asp Cys Thr Cys Phe Gly Leu Pro Arg Arg Tyr Ile
   50 55 60
   Ile Ala Ile Met Ser Gly Leu Gly Phe Cys Ile Ser Phe Gly Ile Arg
   65 70 75 80
   Cys Asn Leu Gly Val Ala Ile Val Ser Met Val Asn Asn Ser Thr Thr
   85 90 95
   His Arg Gly Gly His Val Val Val Gln Lys Ala Gln Phe Ser Trp Asp
   100 105 110
   Pro Glu Thr Val Gly Leu Ile His Gly Ser Phe Phe Trp Gly Tyr Ile
   115 120 125
   Val Thr Gln Ile Pro Gly Gly Phe Ile Cys Gln Lys Phe Ala Ala Asn
   130 135 140
   Arg Val Phe Gly Phe Ala Ile Val Ala Thr Ser Thr Leu Asn Met Leu
   145 150 155 160
   Ile Pro Ser Ala Ala Arg Val His Tyr Gly Cys Val Ile Phe Val Arg
   165 170 175
   Ile Leu Gln Gly Leu Val Glu Gly Val Thr Tyr Pro Ala Cys His Gly
   180 185 190
   Ile Trp Ser Lys Trp Ala Pro Pro Leu Glu Arg Ser Arg Leu Ala Thr
   195 200 205
   Thr Ala Phe Cys Gly Ser Tyr Ala Gly Ala Val Val Ala Met Pro Leu
   210 215 220
   Ala Gly Val Leu Val Gln Tyr Ser Gly Trp Ser Ser Val Phe Tyr Val
   225 230 235 240
   Tyr Gly Ser Phe Gly Ile Phe Trp Tyr Leu Phe Trp Leu Leu Val Ser
   245 250 255
   Tyr Glu Ser Pro Ala Leu His Pro Ser Ile Ser Glu Glu Glu Arg Lys
   260 265 270
   Tyr Ile Glu Asp Ala Ile Gly Glu Ser Ala Lys Leu Met Asn Pro Leu
   275 280 285
   Thr Lys Phe Ser Thr Pro Trp Arg Arg Phe Phe Thr Ser Met Pro Val
   290 295 300
   Tyr Ala Ile Ile Val Ala Asn Phe Cys Arg Ser Trp Thr Phe Tyr Leu
   305 310 315 320
   Leu Leu Ile Ser Gln Pro Asp Tyr Phe Glu Glu Val Phe Gly Phe Glu
   325 330 335
   Ile Ser Lys Val Gly Leu Val Ser Ala Leu Pro His Leu Val Met Thr
   340 345 350
   Ile Ile Val Pro Ile Gly Gly Gln Ile Ala Asp Phe Leu Arg Ser Arg
   355 360 365
   Arg Ile Met Ser Thr Thr Asn Val Arg Lys Leu Met Asn Cys Gly Gly
   370 375 380
   Phe Gly Met Glu Ala Thr Leu Leu Leu Val Val Gly Tyr Ser His Ser
   385 390 395 400
   Lys Gly Val Ala Ile Ser Phe Leu Val Leu Ala Val Gly Phe Ser Gly
   405 410 415
   Phe Ala Ile Ser Gly Phe Asn Val Asn His Leu Asp Ile Ala Pro Arg
   420 425 430
   Tyr Ala Ser Ile Leu Met Gly Ile Ser Asn Gly Val Gly Thr Leu Ser
   435 440 445
   Gly Met Val Cys Pro Ile Ile Val Gly Ala Met Thr Lys His Lys Thr
   450 455 460
   Arg Glu Glu Trp Gln Tyr Val Phe Leu Ile Ala Ser Leu Val His Tyr
   465 470 475 480
   Gly Gly Val Ile Phe Tyr Gly Val Phe Ala Ser Gly Glu Lys Gln Pro
   485 490 495
   Trp Ala Glu Pro Glu Glu Met Ser Glu Glu Lys Cys Gly Phe Val Gly
   500 505 510
   His Asp Gln Leu Ala Gly Ser Asp Asp Ser Glu Met Glu Asp Glu Ala
   515 520 525
   Glu Pro Pro Gly Ala Pro Pro Ala Pro Pro Pro Ser Tyr Gly Ala Thr
   530 535 540
   His Ser Thr Phe Gln Pro Pro Arg Pro Pro Pro Pro Val Arg Asp Tyr
   545 550 555 560
<210> 5
   <211> 2024
   <212> DNA
   <213> Rattus Norvegicus
<400> 5
<210> 6
   <211> 560
   <212> PRT
   <213> Rattus Norvegicus
<400> 6
   Met Glu Phe Arg Gln Glu Glu Phe Arg Lys Leu Ala Gly Arg Ala Leu
   1 5 10 15
   Gly Arg Leu His Arg Leu Leu Glu Lys Arg Gln Glu Gly Ala Glu Thr
   20 25 30
   Leu Glu Leu Ser Ala Asp Gly Arg Pro Val Thr Thr His Thr Arg Asp
   35 40 45
   Pro Pro Val Val Asp Cys Thr Cys Phe Gly Leu Pro Arg Arg Tyr Ile
   50 55 60
   Ile Ala Ile Met Ser Gly Leu Gly Phe Cys Ile Ser Phe Gly Ile Arg
   65 70 75 80
   Cys Asn Leu Gly Val Ala Ile Val Ser Met Val Asn Asn Ser Thr Thr
   85 90 95
   His Arg Gly Gly His Val Val Val Gln Lys Ala Gln Phe Asn Trp Asp
   100 105 110
   Pro Glu Thr Val Gly Leu Ile His Gly Ser Phe Phe Trp Gly Tyr Ile
   115 120 125
   Val Thr Gln Ile Pro Gly Gly Phe Ile Cys Gln Lys Phe Ala Ala Asn
   130 135 140
   Arg Val Phe Gly Phe Ala Ile Val Ala Thr Ser Thr Leu Asn Met Leu
   145 150 155 160
   Ile Pro Ser Ala Ala Arg Val His Tyr Gly Cys Val Ile Phe Val Arg
   165 170 175
   Ile Leu Gln Gly Leu Val Glu Gly Val Thr Tyr Pro Ala Cys His Gly
               180                 185                 190
   Ile Trp Ser Lys Trp Ala Pro Pro Leu Glu Arg Ser Arg Leu Ala Thr
           195                 200                 205
   Thr Ala Phe Cys Gly Ser Tyr Ala Gly Ala Val Val Ala Met Pro Leu
       210                 215                 220
   Ala Gly Val Leu Val Gln Tyr Ser Gly Trp Ser Ser Val Phe Tyr Val
   225                 230                 235                 240
   Tyr Gly Ser Phe Gly Ile Phe Trp Tyr Leu Phe Trp Leu Leu Val Ser
                   245                 250                 255
   Tyr Glu Ser Pro Ala Leu His Pro Ser Ile Ser Glu Glu Glu Arg Lys
               260                 265                 270
   Tyr Ile Glu Asp Ala Ile Gly Glu Ser Ala Lys Leu Met Asn Pro Val
           275                 280                 285
   Thr Lys Phe Asn Thr Pro Trp Arg Arg Phe Phe Thr Ser Met Pro Val
       290                 295                 300
   Tyr Ala Ile Ile Val Ala Asn Phe Cys Arg Ser Trp Thr Phe Tyr Leu
   305                 310                 315                 320
   Leu Leu Ile Ser Gln Pro Ala Tyr Phe Glu Glu Val Phe Gly Phe Glu
                   325                 330                 335
   Ile Ser Lys Val Gly Leu Val Ser Ala Leu Pro His Leu Val Met Thr
               340                 345                 350
   Ile Ile Val Pro Ile Gly Gly Gln Ile Ala Asp Phe Leu Arg Ser Arg
           355                 360                 365
   His Ile Met Ser Thr Thr Asn Val Arg Lys Leu Met Asn Cys Gly Gly
       370                 375                 380
   Phe Gly Met Glu Ala Thr Leu Leu Leu Val Val Gly Tyr Ser His Ser
   385                 390                 395                 400
   Lys Gly Val Ala Ile Ser Phe Leu Val Leu Ala Val Gly Phe Ser Gly
                   405                 410                 415
   Phe Ala Ile Ser Gly Phe Asn Val Asn His Leu Asp Ile Ala Pro Arg
               420                 425                 430
   Tyr Ala Ser Ile Leu Met Gly Ile Ser Asn Gly Val Gly Thr Leu Ser
   435 440 445
   Gly Met Val Cys Pro Ile Ile Val Gly Ala Met Thr Lys His Lys Thr
   450 455 460
   Arg Glu Glu Trp Gln Tyr Val Phe Leu Ile Ala Ser Leu Val His Tyr
   465 470 475 480
   Gly Gly Val Ile Phe Tyr Gly Val Phe Ala Ser Gly Glu Lys Gln Pro
   485 490 495
   Trp Ala Glu Pro Glu Glu Met Ser Glu Glu Lys Cys Gly Phe Val Gly
   500 505 510
   His Asp Gln Leu Ala Gly Ser Asp Glu Ser Glu Met Glu Asp Glu Val
   515 520 525
   Glu Pro Pro Gly Ala Pro Pro Ala Pro Pro Pro Ser Tyr Gly Ala Thr
   530 535 540
   His Ser Thr Val Gln Pro Pro Arg Pro Pro Pro Pro Val Arg Asp Tyr
   545 550 555 560
<210> 7
   <211> 3946
   <212> DNA
   <213> Homo Sapiens
<400> 7
<210> 8
   <211> 582
   <212> PRT
   <213> Homo Sapiens
<400> 8
   Met Glu Ser Val Lys Gln Arg Ile Leu Ala Pro Gly Lys Glu Gly Leu
   1               5                   10                  15
   Lys Asn Phe Ala Gly Lys Ser Leu Gly Gln Ile Tyr Arg Val Leu Glu
               20                  25                  30
   Lys Lys Gln Asp Thr Gly Glu Thr Ile Glu Leu Thr Glu Asp Gly Lys
           35                  40                  45
   Pro Leu Glu Val Pro Glu Arg Lys Ala Pro Leu Cys Asp Cys Thr Cys
       50                  55                  60
   Phe Gly Leu Pro Arg Arg Tyr Ile Ile Ala Ile Met Ser Gly Leu Gly
   65                  70                  75                  80
   Phe Cys Ile Ser Phe Gly Ile Arg Cys Asn Leu Gly Val Ala Ile Val
                   85                  90                  95
   Asp Met Val Asn Asn Ser Thr Ile His Arg Gly Gly Lys Val Ile Lys
               100                 105                 110
   Glu Lys Ala Lys Phe Asn Trp Asp Pro Glu Thr Val Gly Met Ile His
           115                 120                 125
   Gly Ser Phe Phe Trp Gly Tyr Ile Ile Thr Gln Ile Pro Gly Gly Tyr
       130                 135                 140
   Ile Ala Ser Arg Leu Ala Ala Asn Arg Val Phe Gly Ala Ala Ile Leu
   145                 150                 155                 160
   Leu Thr Ser Thr Leu Asn Met Leu Ile Pro Ser Ala Ala Arg Val His
                   165                 170                 175
   Tyr Gly Cys Val Ile Phe Val Arg Ile Leu Gln Gly Leu Val Glu Gly
   180 185 190
   Val Thr Tyr Pro Ala Cys His Gly Ile Trp Ser Lys Trp Ala Pro Pro
   195 200 205
   Leu Glu Arg Ser Arg Leu Ala Thr Thr Ser Phe Cys Gly Ser Tyr Ala
   210 215 220
   Gly Ala Val Ile Ala Met Pro Leu Ala Gly Ile Leu Val Gln Tyr Thr
   225 230 235 240
   Gly Trp Ser Ser Val Phe Tyr Val Tyr Gly Ser Phe Gly Met Val Trp
   245 250 255
   Tyr Met Phe Trp Leu Leu Val Ser Tyr Glu Ser Pro Ala Lys His Pro
   260 265 270
   Thr Ile Thr Asp Glu Glu Arg Arg Tyr Ile Glu Glu Ser Ile Gly Glu
   275 280 285
   Ser Ala Asn Leu Leu Gly Ala Met Glu Lys Phe Lys Thr Pro Trp Arg
   290 295 300
   Lys Phe Phe Thr Ser Met Pro Val Tyr Ala Ile Ile Val Ala Asn Phe
   305 310 315 320
   Cys Arg Ser Trp Thr Phe Tyr Leu Leu Leu Ile Ser Gln Pro Ala Tyr
   325 330 335
   Phe Glu Glu Val Phe Gly Phe Glu Ile Ser Lys Val Gly Met Leu Ser
   340 345 350
   Ala Val Pro His Leu Val Met Thr Ile Ile Val Pro Ile Gly Gly Gln
   355 360 365
   Ile Ala Asp Phe Leu Arg Ser Lys Gln Ile Leu Ser Thr Thr Thr Val
   370 375 380
   Arg Lys Ile Met Asn Cys Gly Gly Phe Gly Met Glu Ala Thr Leu Leu
   385 390 395 400
   Leu Val Val Gly Tyr Ser His Thr Arg Gly Val Ala Ile Ser Phe Leu
   405 410 415
   Val Leu Ala Val Gly Phe Ser Gly Phe Ala Ile Ser Gly Phe Asn Val
   420 425 430
   Asn His Leu Asp Ile Ala Pro Arg Tyr Ala Ser Ile Leu Met Gly Ile
           435                 440                 445
   Ser Asn Gly Val Gly Thr Leu Ser Gly Met Val Cys Pro Ile Ile Val
       450                 455                 460
   Gly Ala Met Thr Lys Asn Lys Ser Arg Glu Glu Trp Gln Tyr Val Phe
   465                 470                 475                 480
   Leu Ile Ala Ala Leu Val His Tyr Gly Gly Val Ile Phe Tyr Ala Ile
                   485                 490                 495
   Phe Ala Ser Gly Glu Lys Gln Pro Trp Ala Asp Pro Glu Glu Thr Ser
               500                 505                 510
   Glu Glu Lys Cys Gly Phe Ile His Glu Asp Glu Leu Asp Glu Glu Thr
           515                 520                 525
   Gly Asp Ile Thr Gln Asn Tyr Ile Asn Tyr Gly Thr Thr Lys Ser Tyr
       530                 535                 540
   Gly Ala Thr Thr Gln Ala Asn Gly Gly Trp Pro Ser Gly Trp Glu Lys
   545                 550                 555                 560
   Lys Glu Glu Phe Val Gln Gly Glu Val Gln Asp Ser His Ser Tyr Lys
                   565                 570                 575
   Asp Arg Val Asp Tyr Ser
               580
<210> 9
   <211> 3982
   <212> DNA
   <213> Rattus Norvegicus
<400> 9
   agacagtaag gttcttttgc ttttttccct tacacaagga ttcgatgacg tttttggtca 60
   atctgattaa aagacagcgg atttggttgc gttaagactt caaaaccggg aatttacgtt 120
   gtttttcggt gaggtgactt ccagaacggg gactcatcag cacccgccca aataccacgg 180
   cactgcgcgc gccctcggcc accggatcct ccccttccaa tgagactttg tgactgtgtg 240
   taccaattct cctattagga aacccgtggg ctgaatgcag ctattccgtt gtactctctt 300
   tctcgctctc cctcccctct ccaactcaca gccttgctga aaagctcatc tctgctgaga 360
   agaaaacgtt ctaccttaac ctattaagac tatgcgcaga actcgccttt catagccatc 420
   acaatttaaa tctggtaagg ctggacacga gtctttacaa gaatggagtc ggtaaaacaa 480
   aggattttgg ccccggggaa agaggggata aagaattttg ctggaaaatc cctcggacag 540
   atctacaggg tgctggagaa gaagcaggat aaccgagaga ccatcgagct gacagaggac 600
   ggcaagcccc tggaggtgcc tgagaagaag gctccgctat gcgactgtac gtgcttcggc 660
   ctgccgcgcc gctacatcat agccatcatg agcggcctcg gcttctgcat ctcctttggt 720
   atccgctgta acctgggtgt ggccattgtg gacatggtca acaacagcac catccaccgg 780
   ggtggcaaag ttatcaagga gaaagccaag tttaactggg accccgagac tgtggggatg 840
   attcacgggt cgttcttctg gggctatatc atcacgcaga ttccgggcgg atacatcgca 900
   tcgcgactgg ctgctaaccg ggtctttggg gctgccatac tgcttacctc taccctcaat 960
   atgctgatcc catctgcagc cagagtgcat tatggatgcg tcatctttgt tagaatattg 1020
   caaggacttg tggagggcgt cacctaccca gcctgtcacg ggatatggag caagtgggcc 1080
   cctcctttgg agaggagtag gttggctacc acctccttct gtggttccta tgctggagca 1140
   gtcattgcaa tgcccctagc tggtatcctg gtgcagtaca ctggatggtc ttcagtattt 1200
   tacgtatatg gaagctttgg tatggtctgg tatatgttct ggcttctggt gtcttacgag 1260
   agccccgcaa agcatccaac cataacagac gaagaacgta ggtacataga agagagcatc 1320
   ggggagagcg caaatctgtt aggagcaatg gagaaattca agaccccatg gaggaagttt 1380
   ttcacatcca tgcccgtcta tgcgataatt gttgcaaact tctgcaggag ttggactttt 1440
   tatttactgc tcatcagtca accagcttat ttcgaggagg tttttggatt tgaaatcagc 1500
   aaggttggca tgttgtctgc ggtcccacac ctggtcatga caatcattgt gcctatcggg 1560
   gggcaaattg cagactttct aaggagcaag caaattcttt caacaactac agtgcgaaag 1620
   atcatgaact gcgggggttt tggcatggaa gccacactgc ttctggttgt tggctactct 1680
   catactagag ggtggccatt ctccttcttg gtgcttgcag tgggattcag tggatttgct 1740
   atctctggtt tcaatgtgaa ccacttggat attgccccga gatatgccag tatcttaatg 1800
   ggcatttcaa atggtgttgg cacgctgtcg ggaatggtct gcccgatcat tgttggtgca 1860
   atgacgaaga acaagtcccg tgaagaatgg cagtatgtct tcctcatcgc tgcactggtc 1920
   cactatggtg gagtcatatt ttatgcacta tttgcctcag gagagaagca accttgggca 1980
   gaccctgagg aaacaagcga agaaaagtgt ggcttcattc atgaagatga actggatgaa 2040
   gaaacggggg acatcactca gaattacata aattacggta ccaccaaatc ctacggcgcc 2100
   acctcacagg agaacggagg ctggcctaac ggctgggaga aaaaggaaga atttgtgcaa 2160
   gaaagtgcgc aagacgcgta ctcctataag gaccgagatg attattcata acgaagctag 2220
   ttgctggatt cctttgtagt gtttgtgatt aaattaattg tgattgcaca aaatcatttt 2280
   aagaaatgtg gtgtaaacat gtaaacacat caaccaagca agtcttgctg ttcaaaaaat 2340
   aataataata tgaattcaaa acagaccgtg agagtcccat caagtgcaat ctgtggcggc 2400
   agtcacgtga cgccatttcc attcaggcca ttcgtccttt tcgtttgtga tttaaaggtt 2460
   tcctgtagaa ataagtaggt attcgttgga tccatcacca cgttagagag tacaactaca 2520
   acagttggca catgtcatcc tacggaagtt aggaagccaa agctactgga ttatgtgaac 2580
   tgcatttatt tatttattac actggactgc aaaatatccc agggaaatcc tgtctagaga 2640
   catagtagaa ctggaaagat ggctagattg ggtactgacg ataatcattg tgtgtatatc 2700
   atggagtggc tatatctttt aattggagaa ctatattgta tagctagcaa aattgtactg 2760
   aattattact aggagtgcac agtgtgtgat attttgtgat cttccaaaag cttatcttgc 2820
   agtgttttgt gaaacgcttg ggcacaaaca cttattttta tgaacaagag cttgtaaagg 2880
   gaggagtatg ctccatgctc tcccattcac tacctgacag tatcaaacct tcacatttca 2940
   atgaaatcca acgtccatgt aacatatcac atgacttttt ttgcaaaaaa gaatataaga 3000
   agaaatagac ttcaatgtat tttttattac aactttgtac tggttgtaac ttgcattagg 3060
   aaaaatgatt aatatatgta taatcgtaaa gaatctaata aaaatttact atgaagatat 3120
   agcccttaaa atgcaatatt aacaacaaaa atatatagaa aatttagata atcttccttg 3180
   ataactagag actatatgga actcacacca caaagctata tataatatga aaagataaac 3240
   aatagagatt gtatatgtag acgattttat gacctaatgt cccatttaag aggtatttgt 3300
   cttgagtata tagtacaaag tatattaaaa ttatatctac atccctgtat atcttataca 3360
   tatccactca cacaaacata acaaatactt ttcacacaga accaaaaaca agcatacacc 3420
   taatgttggg tttggggatt gcaatttcta ctttcataga gtcatagaat tttagatggg 3480
   aaaaaaaaag gcattttgct cgtcatttct taatataatt aattcaacag gaactgcaac 3540
   atttgtgtac caagcaataa gtgcgaagca taaacctgct gtgtgtaaac tatccccata 3600
   ctgcttgtgg tagcactgat ttctttcttt taaagaactt aacatcggag ctctttacaa 3660
   tgttttgcgc tgataagaat gcacatccca atttaacgca aagtgtcacc tggtgtgttt 3720
   acctgtctgt tttgggtatt tggtctgttt ggtgtcctgt gctcttgact ggaggccctg 3780
   ctactgcgaa tataaaacgt gaagtttgtt tctaaatgca aaccactcct gaccttaaga 3840
   aactaaagtc cctctctgct ttgtgtctcc aagtactatc atgtgaccat aacccttgct 3900
   gtgctgagta aaaagatgtg aactgtcatt ttgttgctgc gaagcaagtg ttaataaaat 3960
   gttctattta aaaaaaaaaa aa                                          3982
<210> 10
   <211> 582
   <212> PRT
   <213> Rattus Norvegicus
<400> 10
   Met Glu Ser Val Lys Gln Arg Ile Leu Ala Pro Gly Lys Glu Gly Ile
    1               5                  10                  15
   Lys Asn Phe Ala Gly Lys Ser Leu Gly Gln Ile Tyr Arg Val Leu Glu
               20                  25                  30
   Lys Lys Gln Asp Asn Arg Glu Thr Ile Glu Leu Thr Glu Asp Gly Lys
           35                  40                  45
   Pro Leu Glu Val Pro Glu Lys Lys Ala Pro Leu Cys Asp Cys Thr Cys
       50                  55                  60
   Phe Gly Leu Pro Arg Arg Tyr Ile Ile Ala Ile Met Ser Gly Leu Gly
   65                  70                  75                  80
   Phe Cys Ile Ser Phe Gly Ile Arg Cys Asn Leu Gly Val Ala Ile Val
                   85                  90                  95
   Asp Met Val Asn Asn Ser Thr Ile His Arg Gly Gly Lys Val Ile Lys
               100                 105                 110
   Glu Lys Ala Lys Phe Asn Trp Asp Pro Glu Thr Val Gly Met Ile His
           115                 120                 125
   Gly Ser Phe Phe Trp Gly Tyr Ile Ile Thr Gln Ile Pro Gly Gly Tyr
       130                 135                 140
   Ile Ala Ser Arg Leu Ala Ala Asn Arg Val Phe Gly Ala Ala Ile Leu
   145                 150                 155                 160
   Leu Thr Ser Thr Leu Asn Met Leu Ile Pro Ser Ala Ala Arg Val His
                   165                 170                 175
   Tyr Gly Cys Val Ile Phe Val Arg Ile Leu Gln Gly Leu Val Glu Gly
   180 185 190
   Val Thr Tyr Pro Ala Cys His Gly Ile Trp Ser Lys Trp Ala Pro Pro
   195 200 205
   Leu Glu Arg Ser Arg Leu Ala Thr Thr Ser Phe Cys Gly Ser Tyr Ala
   210 215 220
   Gly Ala Val Ile Ala Met Pro Leu Ala Gly Ile Leu Val Gln Tyr Thr
   225 230 235 240
   Gly Trp Ser Ser Val Phe Tyr Val Tyr Gly Ser Phe Gly Met Val Trp
   245 250 255
   Tyr Met Phe Trp Leu Leu Val Ser Tyr Glu Ser Pro Ala Lys His Pro
   260 265 270
   Thr Ile Thr Asp Glu Glu Arg Arg Tyr Ile Glu Glu Ser Ile Gly Glu
   275 280 285
   Ser Ala Asn Leu Leu Gly Ala Met Glu Lys Phe Lys Thr Pro Trp Arg
   290 295 300
   Lys Phe Phe Thr Ser Met Pro Val Tyr Ala Ile Ile Val Ala Asn Phe
   305 310 315 320
   Cys Arg Ser Trp Thr Phe Tyr Leu Leu Leu Ile Ser Gln Pro Ala Tyr
   325 330 335
   Phe Glu Glu Val Phe Gly Phe Glu Ile Ser Lys Val Gly Met Leu Ser
   340 345 350
   Ala Val Pro His Leu Val Met Thr Ile Ile Val Pro Ile Gly Gly Gln
   355 360 365
   Ile Ala Asp Phe Leu Arg Ser Lys Gln Ile Leu Ser Thr Thr Thr Val
   370 375 380
   Arg Lys Ile Met Asn Cys Gly Gly Phe Gly Met Glu Ala Thr Leu Leu
   385 390 395 400
   Leu Val Val Gly Tyr Ser His Thr Arg Gly Val Ala Ile Ser Phe Leu
   405 410 415
   Val Leu Ala Val Gly Phe Ser Gly Phe Ala Ile Ser Gly Phe Asn Val
   420 425 430
   Asn His Leu Asp Ile Ala Pro Arg Tyr Ala Ser Ile Leu Met Gly Ile
   435 440 445
   Ser Asn Gly Val Gly Thr Leu Ser Gly Met Val Cys Pro Ile Ile Val
   450 455 460
   Gly Ala Met Thr Lys Asn Lys Ser Arg Glu Glu Trp Gln Tyr Val Phe
   465 470 475 480
   Leu Ile Ala Ala Leu Val His Tyr Gly Gly Val Ile Phe Tyr Ala Leu
   485 490 495
   Phe Ala Ser Gly Glu Lys Gln Pro Trp Ala Asp Pro Glu Glu Thr Ser
   500 505 510
   Glu Glu Lys Cys Gly Phe Ile His Glu Asp Glu Leu Asp Glu Glu Thr
   515 520 525
   Gly Asp Ile Thr Gln Asn Tyr Ile Asn Tyr Gly Thr Thr Lys Ser Tyr
   530 535 540
   Gly Ala Thr Ser Gln Glu Asn Gly Gly Trp Pro Asn Gly Trp Glu Lys
   545 550 555 560
   Lys Glu Glu Phe Val Gln Glu Ser Ala Gln Asp Ala Tyr Ser Tyr Lys
   565 570 575
   Asp Arg Asp Asp Tyr Ser
   580
<210> 11
   <211> 2836
   <212> DNA
   <213> Mus musculus
<400> 11
   cggccgcccg ggcccgcggg cggggggatt ggcaggggac ccgcgtgggc acagccacca 60
   tggagttccg gcaggaggag tttcggaagc tggcggggcg cgccctgggg aggctgcacc 120
   ggttactgga gaagcggcag gaaggcgcgg agacactgga gctgagtgcc gacgggcggc 180
   cagtgaccac gcacactcgg gacccgcctg tggtggactg cacctgcttt ggcctccctc 240
   gtcgctacat catcgccatc atgagcggtc tgggtttctg tatcagcttt ggcatccgct 300
   gcaacctggg cgtggccatc gtgtccatgg tcaacaacag cacaacccac cgtgggggcc 360
   acgtggtggt gcagaaagcc cagttcaact gggatccaga gactgtcggc ctcatacatg 420
   gctccttttt ctggggctac attgtcactc agattcctgg aggatttatc tgccaaaaat 480
   tcgcagccaa cagggtcttt ggctttgcca ttgtggctac ctccacccta aacatgttga 540
   tcccttcagc agcccgcgtt cactatggct gtgtcatctt cgtgaggatc cttcagggat 600
   tggtggaggg ggtcacatac cctgcttgcc atggcatctg gagcaaatgg gcccctccct 660
   tagaacggag tcggctggca acgacagcct tttgcggttc ctatgctggg gcggtggttg 720
   ccatgccctt ggctggggtc cttgtgcagt attcaggatg gagttctgtc ttctatgtct 780
   atggcagctt cgggatcttt tggtacctgt tctggttgct tgtctcctat gagtcaccgg 840
   cactgcaccc cagcatctct gaggaggagc gcaaatacat tgaggatgcc atcggggaga 900
   gcgccaagct catgaaccct gttacgaagt ttaacacacc ctggaggcgc ttctttacgt 960
   ccatgcccgt ctatgccatc atcgttgcga acttttgccg cagctggacc ttctacctgc 1020
   tcctcatctc tcagcccgcc tactttgaag aagtgttcgg ctttgagatc agcaaggtgg 1080
   ggctggtgtc ggcgctgcct caccttgtca tgaccatcat cgtacccatt ggaggccaga 1140
   tcgctgactt tttgcgcagt cgtcacataa tgtccactac caacgtgcga aagctcatga 1200
   actgcggggg tttcgggatg gaagccacgc tgctgctggt ggtcggatac tcgcactcca 1260
   agggcgtggc catctccttc ctggtcctgg ctgtgggctt cagtggcttt gccatctctg 1320
   ggtttaacgt gaaccacttg gacatcgccc ctcgctatgc cagcatcttg atgggcattt 1380
   ccaatggcgt gggcacactg tctgggatgg tgtgccccat catcgtgggt gcaatgacca 1440
   agcacaagac gcgggaggag tggcagtacg tgttcctcat agcctccctg gtgcactacg 1500
   gcggtgtcat cttctatggg gtctttgctt cgggagagaa gcagccgtgg gcagagccgg 1560
   aggagatgag cgaggagaag tgtggctttg ttggccacga ccagctggct ggcagtgacg 1620
   aaagtgaaat ggaggacgag gctgagcccc caggggcgcc ccccgcgccg cctccgtcct 1680
   acggggccac acacagcaca gtgcagcctc cgaggccccc gccccctgtc cgggactact 1740
   gaccacgggc ctcccactgt ggggcagttt ccaggacttc cactccatac acctctagcc 1800
   tgagcggcag tgtcgaggaa ccccactcct cccctgcctc aggcttaaga tgcaagtcct 1860
   cccttgttcc cagtgctgtc cgaccagccc tctttccctc tcaactgcct cctgcggggg 1920
   gtgaagctgc acactagcag tttcaaggat acccagactc ccctgaaagt cgttctccgc 1980
   ttgtttctgc ctgtgtgggc tcaaatctcc cctttgaggg ctttatttgg agggacagtt 2040
   caacctcttc ctctcttgtg gttttgaggt ttcacccctt cccccaagac cccagggatt 2100
   ctcaggctac cccgagatta ttcaggtggt cccctactca gaagacttca tggtcgtcct 2160
   ctattagttt caaggctcgc ctaaccaatt ctacattttt ccaagctggt ttaacctaac 2220
   caccaatgcc gccgttccca ggactgattc tcaccagcgt ttctgaggga aaatggcggt 2280
   ttcaagtccc cccacccccc ttttcttccc tcgtcccctc accagcacac tttgccgggc 2340
   cttgacctta gcttagtaca atcattgtcc agggaaatgg ccaaaatggc tctgctcacc 2400
   ccgtgctctt tttctgactc agttttcagg tctcagtagt ggctgcccaa agctattaat 2460
   tcagcggctc gaggccacct cttcctcccc gtggtggttt caggatcccc ctcgcccccc 2520
   cccccccaaa tccttgcact ttattctcct gggtggttcc aggccgccct cggtttctca 2580
   gtggccattt gttgtgtgtc cctcaggggc taaatgattc caaatctggg gtgcttcccc 2640
   tcatagacac ccctctctca acgtagaaat ctgggtgggg gtgaggtgtg tgagagaagt 2700
   tacagaatcc caggaaaggg agcggggctg ggaggagagg gttgttcctg gggcagggtc 2760
   gtgtcttggt gtctgtctct gtgacgtaaa tcctgccctg ccccacccca ctcccaataa 2820
   acgctctggt gtacgg                                                  2836
<210> 12
   <211> 560
   <212> PRT
   <213> Mus musculus
<400> 12
   Met Glu Phe Arg Gln Glu Glu Phe Arg Lys Leu Ala Gly Arg Ala Leu
   1 5 10 15
   Gly Arg Leu His Arg Leu Leu Glu Lys Arg Gln Glu Gly Ala Glu Thr
   20 25 30
   Leu Glu Leu Ser Ala Asp Gly Arg Pro Val Thr Thr His Thr Arg Asp
   35 40 45
   Pro Pro Val Val Asp Cys Thr Cys Phe Gly Leu Pro Arg Arg Tyr Ile
   50 55 60
   Ile Ala Ile Met Ser Gly Leu Gly Phe Cys Ile Ser Phe Gly Ile Arg
   65 70 75 80
   Cys Asn Leu Gly Val Ala Ile Val Ser Met Val Asn Asn Ser Thr Thr
   85 90 95
   His Arg Gly Gly His Val Val Val Gln Lys Ala Gln Phe Asn Trp Asp
   100 105 110
   Pro Glu Thr Val Gly Leu Ile His Gly Ser Phe Phe Trp Gly Tyr Ile
   115 120 125
   Val Thr Gln Ile Pro Gly Gly Phe Ile Cys Gln Lys Phe Ala Ala Asn
   130 135 140
   Arg Val Phe Gly Phe Ala Ile Val Ala Thr Ser Thr Leu Asn Met Leu
   145 150 155 160
   Ile Pro Ser Ala Ala Arg Val His Tyr Gly Cys Val Ile Phe Val Arg
   165 170 175
   Ile Leu Gln Gly Leu Val Glu Gly Val Thr Tyr Pro Ala Cys His Gly
   180 185 190
   Ile Trp Ser Lys Trp Ala Pro Pro Leu Glu Arg Ser Arg Leu Ala Thr
   195 200 205
   Thr Ala Phe Cys Gly Ser Tyr Ala Gly Ala Val Val Ala Met Pro Leu
   210 215 220
   Ala Gly Val Leu Val Gln Tyr Ser Gly Trp Ser Ser Val Phe Tyr Val
   225 230 235 240
   Tyr Gly Ser Phe Gly Ile Phe Trp Tyr Leu Phe Trp Leu Leu Val Ser
   245 250 255
   Tyr Glu Ser Pro Ala Leu His Pro Ser Ile Ser Glu Glu Glu Arg Lys
   260 265 270
   Tyr Ile Glu Asp Ala Ile Gly Glu Ser Ala Lys Leu Met Asn Pro Val
   275 280 285
   Thr Lys Phe Asn Thr Pro Trp Arg Arg Phe Phe Thr Ser Met Pro Val
   290 295 300
   Tyr Ala Ile Ile Val Ala Asn Phe Cys Arg Ser Trp Thr Phe Tyr Leu
   305 310 315 320
   Leu Leu Ile Ser Gln Pro Ala Tyr Phe Glu Glu Val Phe Gly Phe Glu
   325 330 335
   Ile Ser Lys Val Gly Leu Val Ser Ala Leu Pro His Leu Val Met Thr
   340 345 350
   Ile Ile Val Pro Ile Gly Gly Gln Ile Ala Asp Phe Leu Arg Ser Arg
   355 360 365
   His Ile Met Ser Thr Thr Asn Val Arg Lys Leu Met Asn Cys Gly Gly
   370 375 380
   Phe Gly Met Glu Ala Thr Leu Leu Leu Val Val Gly Tyr Ser His Ser
   385 390 395 400
   Lys Gly Val Ala Ile Ser Phe Leu Val Leu Ala Val Gly Phe Ser Gly
   405 410 415
   Phe Ala Ile Ser Gly Phe Asn Val Asn His Leu Asp Ile Ala Pro Arg
   420 425 430
   Tyr Ala Ser Ile Leu Met Gly Ile Ser Asn Gly Val Gly Thr Leu Ser
   435 440 445
   Gly Met Val Cys Pro Ile Ile Val Gly Ala Met Thr Lys His Lys Thr
   450 455 460
   Arg Glu Glu Trp Gln Tyr Val Phe Leu Ile Ala Ser Leu Val His Tyr
   465 470 475 480
   Gly Gly Val Ile Phe Tyr Gly Val Phe Ala Ser Gly Glu Lys Gln Pro
   485 490 495
   Trp Ala Glu Pro Glu Glu Met Ser Glu Glu Lys Cys Gly Phe Val Gly
   500 505 510
   His Asp Gln Leu Ala Gly Ser Asp Glu Ser Glu Met Glu Asp Glu Ala
   515 520 525
   Glu Pro Pro Gly Ala Pro Pro Ala Pro Pro Pro Ser Tyr Gly Ala Thr
   530 535 540
   His Ser Thr Val Gln Pro Pro Arg Pro Pro Pro Pro Val Arg Asp Tyr
   545 550 555 560
<210> 13
   <211> 2528
   <212> DNA
   <213> Mus musculus
<400> 13
   ggcacgaggc tcagtcttaa ttccactctg ccactctgcc gcagagcaca attacgccgg 60
   cgcgatggga ggagaccatg ttaaggcagg aagctaacag cagccgctca cctgaggcct 120
   aggaagctcc caagggttct gagagctatg agctctgatc agcaaagtca ccattttaga 180
   cagtagggtt cttttgcttt tttccttaca caagggttcg atgacgtttc tggtcaatct 240
   gattaaaaga cagcggattt gattgcgata agacttcaaa accgggaatt tacgttgttt 300
   ttcggtgagg tgacttccag aacagggact catcagcacc cgcccaaata ccacggcact 360
   gcgcgcgccc tcggccaccg gatcctcccc ttccaatgag actttgtgac tgtgtgtacc 420
   aattctccta ttaggaaacc cgtgggctgc atgcagctat tctgttgtac tctctttctc 480
   gccctccctc ccctctccaa ctcacagcct tgctggaaag ctcacctctg ctgagaagaa 540
   aaagctctac cttaaccaac taagactatg cgcagaatcc gtctttcata gccacaacaa 600
   tttaaatctg gtaaggctgg acaccagtct ttacaagaat ggagtcggta aaacaaagga 660
   ttttggcccc ggggaaagag gggataaaga attttgctgg aaaatccctc ggacagatct 720
   acagggtgct ggagaagaag caggacaacc gagagaccat cgagctgaca gaggacggta 780
   agcccctgga ggtgcctgag aagaaggctc cgctatgcga ctgcacgtgc ttcggcctgc 840
   cgcgccgcta catcatagcc atcatgagcg gcctcggctt ctgcatatcc ttcggcatcc 900
   gctgtaacct gggcgtggcc atcgtggaca tggtcaacaa cagcactatc caccgcggag 960
   gcaaagttat caaggagaaa gccaaattta actgggaccc cgagaccgtg gggatgatcc 1020
   acggatcgtt cttctggggc tatatcatca cccagattcc aggaggatat atcgcatcgc 1080
   ggctggctgc taaccgggtc tttggggctg cgatactgct cacctctacc ctcaatatgc 1140
   tgatcccatc tgcagccaga gtgcattatg gatgtgtcat ctttgttagg atattgcaag 1200
   gacttgtgga gggtgtcacc tacccagcct gtcatgggat atggagcaag tgggcccctc 1260
   ccttggagag gagtaggttg gctacaacct ccttttgtgg ttcctatgct ggagcagtca 1320
   ttgcaatgcc cttagctggt atccttgtgc agtacactgg atggtcgtca gtattttatg 1380
   tgtatggaag ctttggcatg gtctggtaca tgttctggct tctggtgtct tatgagagcc 1440
   ctgcaaagca tcctaccatt acagatgaag aacgtaggta catagaggag agcattggag 1500
   agagcgcaaa tctgctaggt gcaatggaaa aatttaagac cccatggagg aagtttttca 1560
   catccatgcc cgtctacgcg ataattgttg ccaacttctg caggagctgg actttttatt 1620
   tactgctcat cagtcagcca gcttattttg aggaggtttt tggatttgaa atcagcaagg 1680
   ttggcatgtt gtctgcagtc cctcaccttg tcatgacaat cattgtgcct atcggggggc 1740
   aaattgcaga tttcctaagg agcaagcaaa ttctctcaac aactacagtg agaaagatca 1800
   tgaattgtgg gggttttggc atggaagcca cgctgcttct ggttgttggc tactctcata 1860
   ctagaggggt ggccatctcc ttcttggtgc ttgcagtagg attcagtgga tttgctatct 1920
   ctggtttcaa tgttaatcac ttggatattg ctccaagata tgccagtatc ttaatgggca 1980
   tttcaaatgg cgttggcacg ctgtcgggga tggtttgccc tatcattgtt ggtgcaatga 2040
   caaagaataa gtcccgtgaa gaatggcagt atgtcttcct cattgctgca ctcgtccact 2100
   atggtggagt catattttat gcactatttg cctcaggaga gaaacaacct tgggcagacc 2160
   ctgaggaaac aagcgaagaa aaatgtggct tcattcacga agatgaactg gatgaagaaa 2220
   cgggggacat cactcagaat tacataaatt acggtaccac caaatcttac ggtgctacct 2280
   cacaggagaa tggaggctgg cctaacggct gggagaaaaa ggaagaattt gtgcaagaag 2340
   gtgcgcaaga cgcgtacacc tataaggacc gagatgatta ttcataacga tgctagttgc 2400
   tggattcatt tgtagtgttt gtgaatcaat taattgtgat tgcacaaaaa taattttaaa 2460
   aatgtggtgt gaacatgtaa acatatcaac caagcaagtc ttgctgttca aaaaaaaaaa 2520
   aaaaaaaa                                                          2528
<210> 14
   <211> 582
   <212> PRT
   <213> Mus musculus
<400> 14
   Met Glu Ser Val Lys Gln Arg Ile Leu Ala Pro Gly Lys Glu Gly Ile
   1               5                   10                  15
   Lys Asn Phe Ala Gly Lys Ser Leu Gly Gln Ile Tyr Arg Val Leu Glu
               20                  25                  30
   Lys Lys Gln Asp Asn Arg Glu Thr Ile Glu Leu Thr Glu Asp Gly Lys
           35                  40                  45
   Pro Leu Glu Val Pro Glu Lys Lys Ala Pro Leu Cys Asp Cys Thr Cys
       50                  55                  60
   Phe Gly Leu Pro Arg Arg Tyr Ile Ile Ala Ile Met Ser Gly Leu Gly
   65                  70                  75                  80
   Phe Cys Ile Ser Phe Gly Ile Arg Cys Asn Leu Gly Val Ala Ile Val
                   85                  90                  95
   Asp Met Val Asn Asn Ser Thr Ile His Arg Gly Gly Lys Val Ile Lys
               100                 105                 110
   Glu Lys Ala Lys Phe Asn Trp Asp Pro Glu Thr Val Gly Met Ile His
           115                 120                 125
   Gly Ser Phe Phe Trp Gly Tyr Ile Ile Thr Gln Ile Pro Gly Gly Tyr
       130                 135                 140
   Ile Ala Ser Arg Leu Ala Ala Asn Arg Val Phe Gly Ala Ala Ile Leu
   145 150 155 160
   Leu Thr Ser Thr Leu Asn Met Leu Ile Pro Ser Ala Ala Arg Val His
   165 170 175
   Tyr Gly Cys Val Ile Phe Val Arg Ile Leu Gln Gly Leu Val Glu Gly
   180 185 190
   Val Thr Tyr Pro Ala Cys His Gly Ile Trp Ser Lys Trp Ala Pro Pro
   195 200 205
   Leu Glu Arg Ser Arg Leu Ala Thr Thr Ser Phe Cys Gly Ser Tyr Ala
   210 215 220
   Gly Ala Val Ile Ala Met Pro Leu Ala Gly Ile Leu Val Gln Tyr Thr
   225 230 235 240
   Gly Trp Ser Ser Val Phe Tyr Val Tyr Gly Ser Phe Gly Met Val Trp
   245 250 255
   Tyr Met Phe Trp Leu Leu Val Ser Tyr Glu Ser Pro Ala Lys His Pro
   260 265 270
   Thr Ile Thr Asp Glu Glu Arg Arg Tyr Ile Glu Glu Ser Ile Gly Glu
   275 280 285
   Ser Ala Asn Leu Leu Gly Ala Met Glu Lys Phe Lys Thr Pro Trp Arg
   290 295 300
   Lys Phe Phe Thr Ser Met Pro Val Tyr Ala Ile Ile Val Ala Asn Phe
   305 310 315 320
   Cys Arg Ser Trp Thr Phe Tyr Leu Leu Leu Ile Ser Gln Pro Ala Tyr
   325 330 335
   Phe Glu Glu Val Phe Gly Phe Glu Ile Ser Lys Val Gly Met Leu Ser
   340 345 350
   Ala Val Pro His Leu Val Met Thr Ile Ile Val Pro Ile Gly Gly Gln
   355 360 365
   Ile Ala Asp Phe Leu Arg Ser Lys Gln Ile Leu Ser Thr Thr Thr Val
   370 375 380
   Arg Lys Ile Met Asn Cys Gly Gly Phe Gly Met Glu Ala Thr Leu Leu
   385 390 395 400
   Leu Val Val Gly Tyr Ser His Thr Arg Gly Val Ala Ile Ser Phe Leu
                   405                 410                 415
   Val Leu Ala Val Gly Phe Ser Gly Phe Ala Ile Ser Gly Phe Asn Val
               420                 425                 430
   Asn His Leu Asp Ile Ala Pro Arg Tyr Ala Ser Ile Leu Met Gly Ile
           435                 440                 445
   Ser Asn Gly Val Gly Thr Leu Ser Gly Met Val Cys Pro Ile Ile Val
       450                 455                 460
   Gly Ala Met Thr Lys Asn Lys Ser Arg Glu Glu Trp Gln Tyr Val Phe
   465                 470                 475                 480
   Leu Ile Ala Ala Leu Val His Tyr Gly Gly Val Ile Phe Tyr Ala Leu
                   485                 490                 495
   Phe Ala Ser Gly Glu Lys Gln Pro Trp Ala Asp Pro Glu Glu Thr Ser
               500                 505                 510
   Glu Glu Lys Cys Gly Phe Ile His Glu Asp Glu Leu Asp Glu Glu Thr
           515                 520                 525
   Gly Asp Ile Thr Gln Asn Tyr Ile Asn Tyr Gly Thr Thr Lys Ser Tyr
       530                 535                 540
   Gly Ala Thr Ser Gln Glu Asn Gly Gly Trp Pro Asn Gly Trp Glu Lys
   545                 550                 555                 560
   Lys Glu Glu Phe Val Gln Glu Gly Ala Gln Asp Ala Tyr Thr Tyr Lys
                   565                 570                 575
   Asp Arg Asp Asp Tyr Ser
               580

## Beschreibung

Die Erfindung betrifft ein Verfahren zur Auffindung schmerzregulierender Substanzen unter Verwendung von BNPI und/oder DNPI.

Zur Therapie von Schmerzen stehen unterschiedliche Arzneimittel zur Verfügung wie z.B. Acetylsalicylsäure, Paracetamol, Dipyrone, Tramadol, Morphin und Fentanyl; aber auch Substanzen wie Amitryptilin und Ketamin kommen zur Behandlung von Schmerzpatienten zum Einsatz. Trotz zunehmend verfeinerter Therapleschemata kann jedoch insbesondere bei chronischen Schmerzzuständen oft keine dauerhafte Verbesserung für die Patienten erzielt werden. Hierfür ist unter anderem auch die Tatsache verantwortlich, daß es beim chronischen Schmerz zu dauerhaften Veränderungen beteiligter Nervenzellen kommt.

Die Schmerzforschung der letzten Jahre erbrachte die grundlegende Erkenntnis, daß der Entwicklung gerade chronischer Schmerzzustände plastische Veränderungen des Nervensystems, insbesondere in den nozizeptiven Neuronen der Hinterwurzelganglien und der Neurone im Bereich der Dorsalhömer des Rückenmarks, zugrunde liegen (als Überblick siehe: Coderre et al. 1993; Zimmermann & Herdegen, 1996). Die neuronale Plastizität geht einher mit Veränderungen in der Expression bestimmter Gene und führt zur langanhaltenden Veränderung des Phänotyps der betroffenen Neuronen. Das Konzept der neuronalen Plastizität wurde bisher vor allem auf Entwicklungs-, Lern- und Regenerationsprozesse angewandt, doch die neueren Befunde aus der Schmerzforschung zeigen, daß dieses Konzept auch bei pathophysiologischen Vorgängen greift (Tölle, 1997).

Die Chronifizierung des Schmerzes ist tierexperimentell auf phänomenologischer Ebene bereits relativ gut charakterisiert. Die Induktion chronischer Schmerzzustände führt zu folgenden Veränderungen:
- Erhöhte Empfindlichkeit und verringerte Reizschwelle peripherer Nozizeptoren
- Aktivierung sogenannter stiller Nozizeptoren
- Reorganisation rezeptiver Felder
- Erregbarkeitszunahme im Rückenmark.

Diese plastischen Veränderungen sind sowohl für die in den Ganglien vorkommenden primären Afferenzen, als auch für die im Rückenmark lokalisierten nachgeschalteten Neurone beschrieben worden und werden auch supraspinal z. B. im Thalamus vermutet. In Analogie zu den für Lern- und Gedächtnisprozesse beschriebenen Mechanismen ist anzunehmen, daß in den beteiligten Zellen ein spezifisches Genprogramm abläuft, das die koordinierte Regulation relevanter Gene beinhaltet, deren Expression dann maßgeblich zur pathophysiologischen Ausprägung chronischer Schmerzen beiträgt.

Ausgangspunkt der Erfindung war daher die Identifizierung derartiger schmerzregulierter Gene, die in ihrer Expression unter Schmerzbedingungen verändert und deshalb wahrscheinlich an der Entstehung und Verarbeitung von insbesondere chronischen Schmerzen beteiligt sind, über ihre Regulationszusammenhänge.

So beschreibt bspw. WO 98/26054 ein Verfahren zur Behandlung eines Organismus mit einer Erkrankung oder Störung des Signaltransduktionsweges, an welchem ein PKY2-Protein beteiligt ist. WO 98/26054 stellt weiterhin Verfahren zur Diagnose sowie zum Screening von Wirkstoffen zur Behandlung von solchen Störungen oder Erkrankungen bereit. Die über ein solches Screening-Verfahren identifizierten PKY2-Inhibitoren sind u.a. verwendbar zur Therapie von Epilepsie, Schizophrenie, extremer Hyperaktivität bei Kindern, chronischem und akutem Schmerz, Morbus Alzheimer, Morbus Parkinson, neurodegenerativen Erkrankungen und Migräne.

WO 96/34288 offenbart einen aus neuronalem Gewebe isolierten Na⁺-abhängigen Kotransporter für anorganisches Phosphat (hBNPI). WO 96/34288 offenbart ebenfalls Verfahren unter Verwendung von hBNPI zum Screening von Testverbindungen auf deren Wirksamkeit zur Stimulation von hBNPI. WO 96/34288 beschreibt kein Verfahren zum Screening von schmerzrelevanten Verbindungen.

Für eine Reihe bekannter Gene wurde bereits eine Regulation in verschiedenen Schmerzmodellen nachgewiesen (s. Tabelle 1), so zum Beispiel für Neurotransmitter (Substanz P, CGRP), Rezeptoren (Substanz P-Rezeptor, µ, κ, δ-Opiatrezeptoren, NMDA-Rezeptor) und Transkriptionsfaktoren (cJun, JunB, cFos oder Krox24). Die Tatsache, daß die genannten Rezeptoren bereits als molekulare Targets für die Entwicklung neuer Analgetika verwendet werden (Dickenson, 1995), gibt einen deutlichen Hinweis darauf, daß auch die Identifizierung neuer schmerzregulierter Gene für die Entwicklung von Analgetika, insbesondere für entsprechende Screeningverfahren, von großem Interesse ist. Die zentrale Idee ist hierbei, die Entstehung oder Persistenz von Schmerzen, insbesondere chronischer Art, zu unterbrechen, indem solche Proteine in ihrer Funktion beeinflußt werden, die in Schmerz-Zuständen verstärkt oder vermindert gebildet werden.

**Tabelle 1: Regulation bekannter Gene/Genprodukte in Schmerz-Tiermodellen**

| **Gen(produkt)** | **Reg** | **Gewebe/Zelle** | **Modell** | **Literatur** |
|---|---|---|---|---|
| **(a) Neurotransmitter** | | | | |
| CGRP | ⇑ | RM-Dorsalhorn | UV-Bestrahlung | Gillardon F et al. (1992) |
| | | | der Haut | Ann NY Acad Sci657: 493- |
| | | | | 96 |
| Preprotachykinin & | ⇑ | DRG | Monoarthritis | Donaldson LF et al. (1992) |
| CGRP-mRNA | | | | Mol Brain Res 16:143-49 |
| Preprotachykinin | ⇑ | RM-Dorsalhorn | Formalin | Noguchi &Ruda (1992) |
| -mRNA | | | | J Neurosci 12:2563-72 |
| Prodynorphln mRNA | ⇑ | Rückenmark | Exp Arthritis | Höllt et al (1987) |
| | | | | Neurosci Lett 96:247-52 |
| Dynorphin Prot. | ⇑ | Rückenmark | Formalin | Ruda et al. (1988) |
| | | | | PNAS 85: 622-26 |
| Substanz P | ⇑ | Nozizeptoren | Exp. Arthritis | Levine JD et al. (1984) |
| | | | | Science 226:547-49 |
| **(b) Neurotrophine** | | | | |
| BDNF mRNA & | ⇑ | DRG: trkA+ | i. th. NGF Inj. | Michael GC et al. (1997) |
| Immunreaktivität | | Zellen | | J Neurosci 17: 8476-90 |
| **(c) Rezeptoren** | | | | |
| µ, κ, δ-Bindung | ⇓ ⇑ | RM-Dorsalhom | Monoarthritis | Besse D et al. (1992) |
| | | | | Eur J Pharmacol 223:123- |
| | | | | 31 |
| µ-Opiatrezeptor- | ⇑ | DRG | Carageenan ind. | Ji R-R et al. (1995) |
| Immunrealtivität | | | Entzündung | J Neurosci 15: 8156-66 |
| κ & δ-Opiatrez.-mRNA | ⇓ | DRG | Carageenan ind. | Ji R-R et al. (1995) |
| | | | Entzündung | J Neurosci 15: 8156-66 |
| κ & µ-Opiatrezeptor-mRNA | ⇑ | RM-Dorsalhom | Monoarthritis | Maekawa K et al. (1995) |
| | | | | Pain 64:365-71 |
| CCK_{B}-Rez. mRNA | ⇑ | DRG | Axotomie | Zhang X et al. (1993) |
| | | | | Neuroscience 57: 227-233 |
| NMDA-R1-mRNA | ⇑ | RM-Dorsalhorn | CFA-induzierte | Kus L et al. (1995) |
| | | Laminae I & II | Entzündung | Neuroscience 68: 159-65 |
| **(d) Enzyme** | | | | |
| NADPH-Diaphorase | ⇑ | RM-Dorsalhorn | Ischlaticus- | Fiallos-Estrada et al. ('93) |
| Aktivität | | | Transektion | Neurosci Lett 150:169-73 |
| NADPH-Diaphorase | ⇑ | Rückenmark | Formalin | Solodkin et al. 1992 |
| | | | | Neurosci 51: 495-99 |
| NO-Synthetase mRNA | ⇑ | DRG | Axotomie | Verge VMK et al. (1992) |
| | | | | PNAS 89: 11617-62 |
| NO-Synthetase Protein | ⇑ | RM-Dorsalhorn | Formalin | Herdegen et al. (1994) |
| | | | | Mol Brain Res 22:245-58 |
| NO-Synthetase- | ⇑ | DRG | Ischiaticus- | Fiallos-Estrada et al. ('93) |
| Immunreaktivität | | | Transektion | Neurosci Lett 150:169-73 |
| **(e) Signalkaskade** | | | | |
| rap1A, rap1B, H-r-as | ⇑ | Rückenmark | Formalin | Urayama O et al. (1997) |
| mRNA | | | | Mol Brain Res 45:331-34 |
| PKC-Bindung | ⇑ | RM-Dorsalhorn | CFA-induzierte | Tölle TR et al (82) |
| | | | Monoarthritis | J Neurol 242(S2):135 |
| **(f)Transkriptionsf.** | | | | |
| cFOS | ⇑ | Rückenmark | Noxische | Hunt SP et al. (1987) |
| | | | Stimulierung | Nature 328: 632-34 |
| cJun,JunB, cFOS | ⇑ | RM-Dorsalhorn | Formalin | Herdegen T et al. (1994) |
| Krox24 | | | | Mol Brain Res 22: 245-48 |

| | | | | |
|---|---|---|---|---|
| RM, Rückenmark; DRG, Dorsal Root Ganglia; CFA, Complete Freund Adjunvans; NGF, Nerve Growth Factor. | | | | |

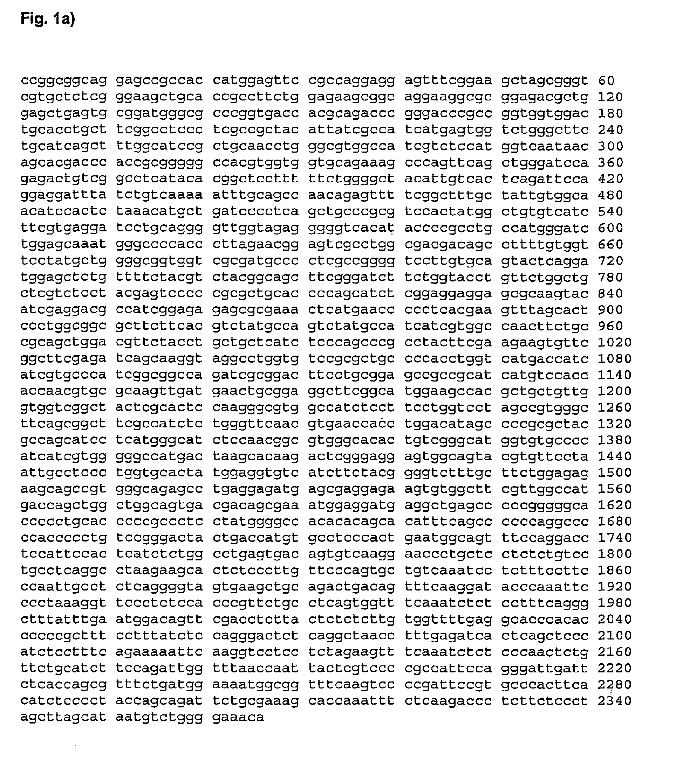

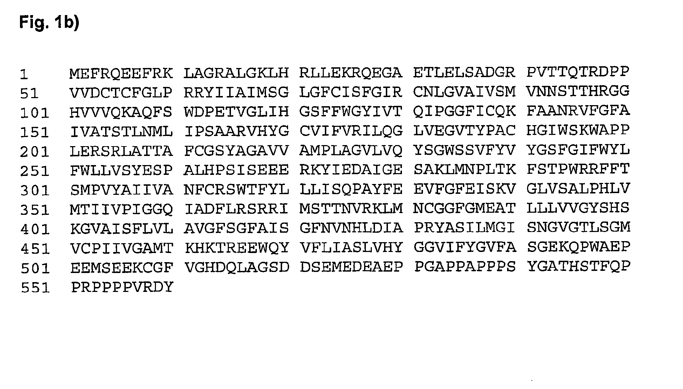

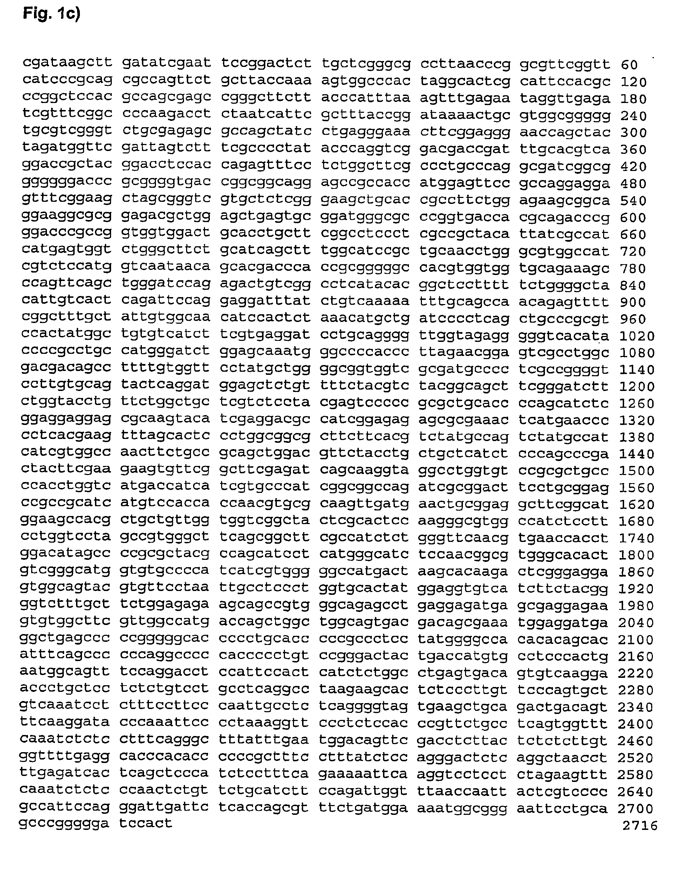

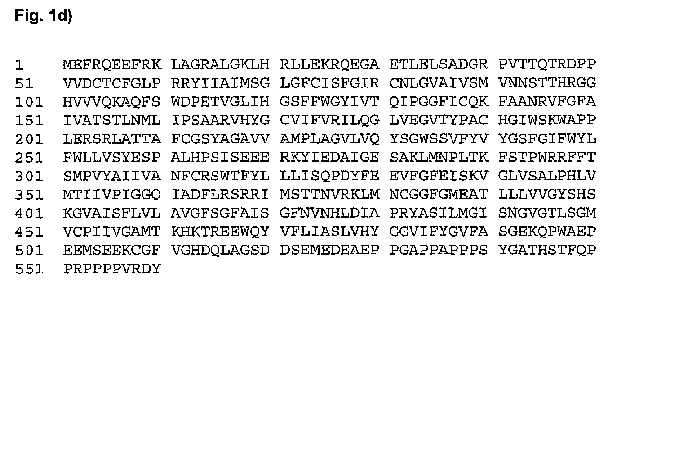

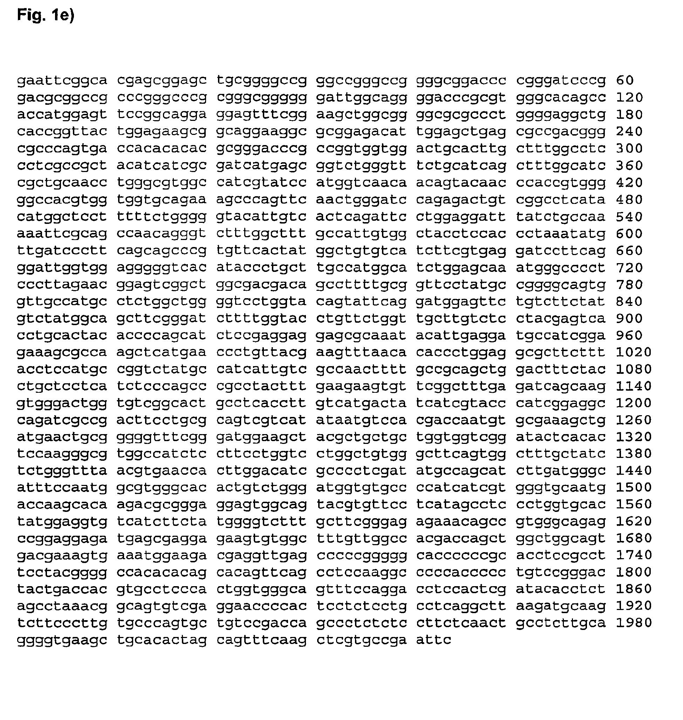

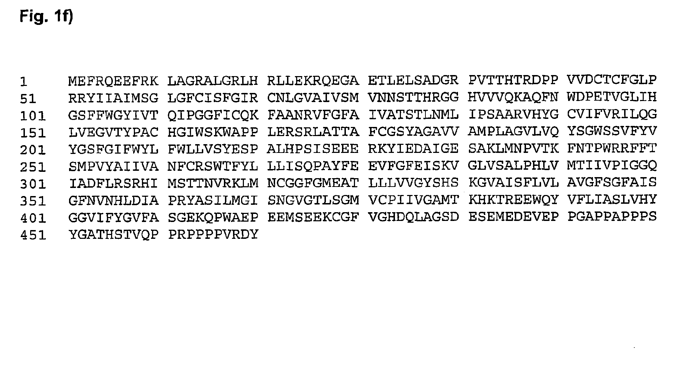

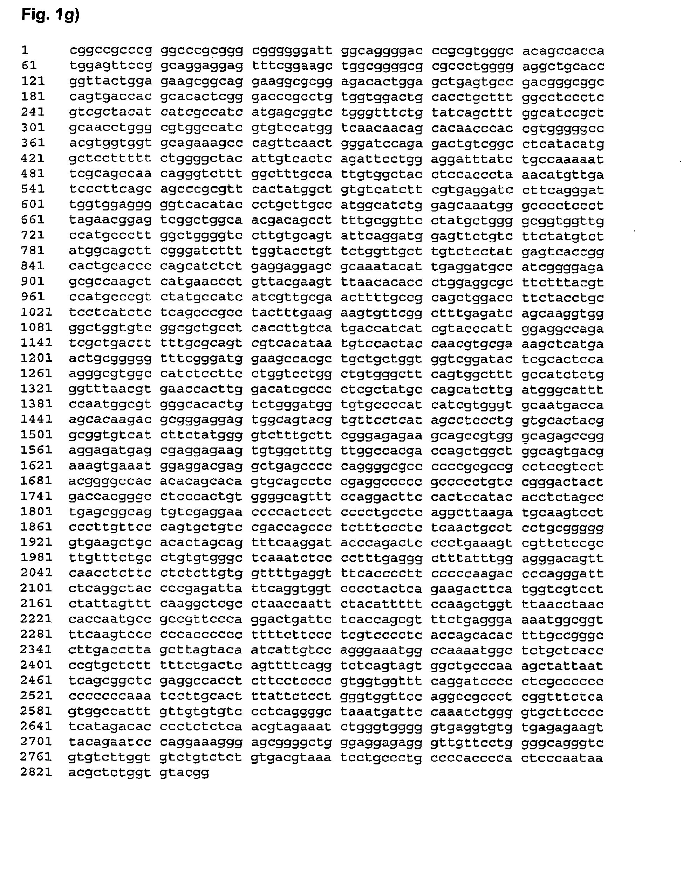

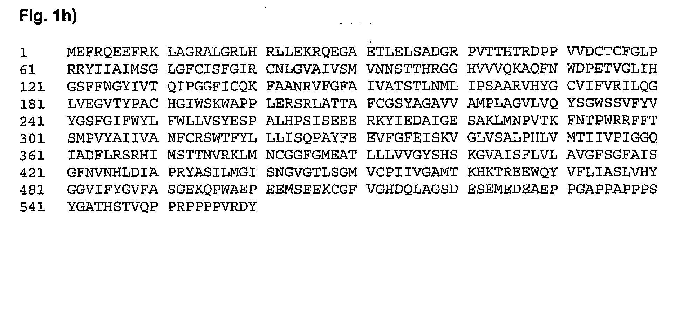

Daraus folgend war primäre Aufgabe der Erfindung, ein Screeningverfahren zur Identifizierung im Schmerz relevanter, insbesondere schmerzregulierender Substanzen zu entwickeln. Die Erfindung betrifft daher ein Verfahren zur Auffindung schmerzregulierender Substanzen mit folgenden Verfahrensschritten:
(a) Inkubation einer zu testenden Substanz unter geeigneten Bedingungen mit einem Biomolekül aus Gruppe I: dem Protein BNPI oder DNPI und/oder einem Protein gemäß einer der Abbildungen 1 b), 1 d), 1f), 1h), 2b), 2d) oder 2f) und/oder einem zu einem dieser vorgenannten Proteine zu mindestens 90 % ähnliches Protein und/oder einem Protein, für das ein Polynukleotid gemäß einer der Abbildungen 1a), 1c), 1e), 1g), 2a), 2c) oder 2e) oder ein dazu zu mindestens 90 % ähnliches Polynukleotid kodiert, und/oder einem Protein, das durch eine Nukleinsäure kodiert wird, die unter stringenten Bedingungen an ein Polynukleotid gemäß einer der Abbildungen 1a), 1c), 1e), 1g), 2a), 2c) oder 2e) oder deren Antisense Polynukleotide binden, und/oder einer Zelle und/oder einer Präparation aus einer solchen Zelle, die mindestens eines der vorgenannten Proteine synthetisiert hat,
(b) Messung der Bindung der Testsubstanz an dem von der Zelle synthetisierten Protein oder Teilprotein oder Messung mindestens eines der durch die Bindung der Testsubstanz an das Protein veränderten funktionellen Parameter über Messung der Regulation, Hemmung und/oder Aktivierung von Rezeptoren, Ionenkanälen und/oder Enzymen, insbesondere über Messung der Veränderung der Genexpression, des lonenmileus, des pH oder des Membranpotentials, über Veränderung der Enzymaktivität oder der Konzentration der 2^{nd} messenger, oder
   über Messung der Bindung über die Verdrängung eines bekannten markierten Liganden des Proteins und/oder über die daran gebundene Aktivität einer markierten Testsubstanz.

Dieses neuartige Screeningverfahren basiert darauf, daß hier eine potentielle Schmerz-Wirksamkeit einer Substanz über ihre Wechselwirkung mit einer schmerzregulierten Proteinstruktur, BNPI oder DNPI oder verwandte Strukturen, aufgefunden werden kann.

Dabei bezieht sich der Begriff schmerzregulierend auf einen potentiellen regulierenden Einfluß auf das physiologische Schmerzgeschehen, insbesondere auf eine analgetische Wirkung. Der Begriff Substanz umfaßt jede als Arzneimittel-Wirkstoff geeignete Verbindung, insbesondere also niedermolekulare Wirkstoffe, aber auch andere wie Nukleinsäuren, Fette, Zucker, Peptide oder Proteine wie Antikörper.

Die Inkubation unter geeigneten Bedingungen ist hier so zu verstehen, daß die zu untersuchende Substanz mit der Zelle oder der entsprechenden Präparation in einem wässrigen Medium eine definierte Zeit vor der Messung reagieren kann. Dabei kann das wässrige Medium temperiert werden, beispielsweise zwischen 4°C und 40°C, vorzugsweise bei Raumtemperatur oder bei 37°C. Die Inkubationszeit kann zwischen wenigen Sekunden und mehreren Stunden variiert werden, je nach der Wechselwirkung der Substanz mit dem Protein. Bevorzugt sind aber Zeiten zwischen 1 min und 60 min. Das wäßrige Medium kann geeignete Salze und/oder Puffersysteme enthalten, so daß bei der Inkubation beispielsweise ein pH zwischen 6 und 8, vorzugsweise pH 7,0 - 7,5 im Medium herrscht. Dem Medium können weiter geeignete Substanzen, wie Coenzyme, Nährstoffe etc. beigefügt werden. Die geeigneten Bedingungen können vom Fachmann in Abhängigkeit von der zu untersuchenden Wechselwirkung der Substanz mit dem Protein aufgrund seiner Erfahrung, der Literatur oder weniger, einfacher Vorversuche leicht festgelegt werden, um im Verfahren einen möglichst deutlichen Meßwert zu erhalten.

Eine Zelle, die ein bestimmtes Protein synthetisiert hat, ist eine Zelle, die dieses Protein bereits endogen exprimiert hat oder eine solche, die gentechnisch verändert wurden, so daß sie dieses Protein exprimiert und entsprechend vor Beginn des erfindungsgemäßen Verfahrens das Protein enthält. Die Zellen können Zellen aus evt. immortalisierten Zellinien sein oder native aus Geweben stammende und aus diesen isolierte Zellen sein, wobei der Zellverband meist aufgelöst ist. Die Präparation aus diesen Zellen umfaßt insbesondere Homogenate aus den Zellen, das Cytosol, eine Membranfraktion der Zellen mit Membranfragmenten, eine Suspension isolierter Zellorganellen etc.

Die hier aufgezählten Proteine wurden im Rahmen dieser Erfindung als durch Schmerz reguliert oder schmerzrelevant verteilt identifiziert, in dem in einem Tier Schmerz ausgelöst wurde und nach angemessener Zeit durch Schnitte im Rückenmark das Expressionsmuster des Tieres mit denen eines Kontroll-Tieres ohne schmerzauslösende Maßnahmen verglichen. Die dabei gefundenen verändert exprimierten sind BNPI sowie insbesondere bezüglich der schmerzrelevanten Verteilung die DNPI.

Aus welcher Spezies diese Proteine stammen, ist für die Funktion des Verfahrens unerheblich, es ist aber bevorzugt, die humane, Maus- oder Ratten-Variante zu verwenden. BNPI und DNPI sind in Hinblick auf die kodierende DNA- und die Aminosäure-Sequenz bekannt und auch in Ihrer generellen Funktion beschrieben. BNPI, der "brain Na+ dependent inorganic phosphate cotransporter" ist in der WO 96/34288 beschrieben und der DNPI, der "differentiation-associated Na+ dependent inorganic phosphate Cotransporter", wurde von Aihara et al. (2000) im J. Neurochem. 74, 2622-2625 beschrieben.
Keiner dieser Transporter wurde aber bisher im Stand der Technik in einen Zusammenhang mit Schmerz und insbesondere der Schmerzregulation gebracht. Da hier die Identifizierung der Proteine über eine Veränderung der Expression oder die Expressionsverteilung in einem In-vivo-Schmerzmodell erfolgte, hat das daraus abgeleitete erfindungsgemäße Screening-Verfahren für zukünftige Arzneimittel unter Verwendung dieser Proteine den erheblichen Vorteil, nicht nur auf theoretischen Oberlegungen aufzubauen, sondern vermutlich eine starke In-vivo-Relevanz zu besitzen. Da mit diesem Verfahren die Wechselwirkung von Substanzen mit im Schmerzbereich bisher nicht verwendeten Proteinen und Peptiden als Maßstab für das Auffinden schmerzregulierender Substanzen ermöglicht wird, sind mit diesem Verfahren jetzt möglicherweise schmerzrelevante Substanzen aufzufinden, die bei den im Stand der Technik bisher bekannten Verfahren mit anderen Peptiden oder Proteinen nicht aufgefallen wären. Auch dies ist ein erheblicher Vorteil des neuen erfindungsgemäßen Verfahrens.

Der Maßstab Ober den das Verfahren die Auffindung interessanter Substanzen erlaubt, ist entweder die Bindung an das Protein, die z.B. durch Verdrängung eines bekannten Liganden oder das Ausmaß gebundener Substanz nachgewiesen werden kann, oder die Veränderung eines funktionellen Parameters durch die Wechselwirkung der Substanz mit dem Protein. Diese Wechselwirkung kann Insbesondere in einer Regulation, Hemmung und/oder Aktivierung von Rezeptoren, Ionenkanälen und/oder Enzymen liegen und veränderte funktionelle Parameter können beispielsweise die Genexpression, das Ionenmilieu der pH oder das Membranpotentials, bzw. die Veränderung der Enzymaktivität oder der Konzentration der 2^{nd} messenger sein.

Zur Erläuterung der Erfindung werden im folgenden neben den im allgemeinen Text zu Begriffen gegebenen Erklärungen weitere Definitionen angegeben, um klarzustellen, wie bestimmte, insbesondere in den Ansprüchen verwendete Begriffe im Sinne dieser Erfindung zu verstehen und auszulegen sind.
- Substanz: Damit ist eine chemische Verbindung gemeint. Hier handelt es sich im engeren Sinne um Verbindungen, die potentiell eine Wirkung im Körper entfalten können, niedermolekulare Wirkstoffe, Nukleinsäuren, Fette, Zucker, Peptide oder Proteine, insbesondere hier niedermolekulare Wirkstoffe.
- schmerzregulierend: Im Sinne der Erfindung heißt schmerzregulierend, daß die Substanz die Wahrnehmung von Schmerz direkt oder indirekt beeinflußt, insbesondere natürlich analgetisch wirkt.
- Schmerz: Im Sinne der Erfindung bedeutet Schmerz insbesondere ein Schmerzempfinden, präziser akkuter, chronischer, neuropathischer und entzündlicher Schmerz inclusive Migräne, insbesondere ist der Schmerz zugehörig zu folgenden Arten:
   chronischem Schmerz, insbesondere muskuloskelettalem Schmerz; neuropathischem Schmerz, insbesondere allodynischem Schmerz, mechanischer Hyperalgesie oder diabetischer Neuropathie; viszeralem Schmerz, cerebralem Schmerz, peripherem Schmerz oder entzündungsbedingtem Schmerz, insbesondere peripherem Entzündungsschmerz; sowie Migräne, Cluster-Kopfschmerz oder den Schmerz bei Trigeminus Neuralgie.
- Inkubation: Unter Inkubation ist das Einbringen und Belassen eines biologischen Untersuchungsobjektes, beispielsweise einer Zelle oder eines Proteins, in einem temperierten Medium wie in einem Brutschrank oder auf einem Wasserbad zu verstehen. Dabei heißt hier unter geeigneten Bedingungen eine Inkubation unter physiologischen Bedingungen (z.B. 37°C pH7,2) oder bei den Bedingungen, bei denen eine optimale Messung im Verfahren möglich wird.
- Zelle: Die Zelle ist ein sich selbst regulierendes, offenes, mit seiner Umgebung durch permanenten Stoffaustausch in einem Fließgleichgewicht stehendes System mit eigenem Stoffwechsel, und Vermehrungsfähigkeit. Die Zelle kann separat kultiviert oder Teil eines Gewebes, insbesondere aus einem Organ, sein, und dort vereinzelt oder noch im Zellverband vorliegen.
- Präparation aus einer Zelle: Darunter versteht man Präparate, die mittels chemischer, biologischer, mechanischer oder physikalischer Methoden unter Änderung der Zellstruktur hergestellt werden, beispielsweise Membranfragmente, isolierte Zellkompartimente, isoliertes Cytosol, oder aus Gewebe gewonnenes Homogenat.
- Peptid: Verbindung aus über peptidische Bindungen zu Ketten verknüpften Aminosäuren. Ein Oligopeptid besteht aus zwischen 2 und 9 Aminosäuren, ein Polypeptid aus zwischen 10 und 100 Aminosäuren.
- Protein: Verbindung aus über peptidische Bindungen zu Ketten verknüpften mehr als 100 Aminosäuren u.U. mit einer definierten Raumstruktur.
- PIM1-Kinase: PIM3-Kinase: Ein Proto-Oncogen und eine Serin-Threonin-Kinase.
- Polynukleotid: Das zugrundeliegende Nukleotid ist ein grundsätzlich aus Nucleinbase, Pentose und Phosphorsäure bestehender Grundbaustein der Nucleinsäuren. Diese entspricht einem hochmolekularen Polynucleotid aus mehreren Nukleotiden, die über Phosphorsäure-Pentose-Veresterung miteinander verknüpft sind. Unter diese Erfindung fallen aber auch modifizierte Polynukleotide, die zwar die Basenabfolge beibehalten, aber über ein modifiziertes Rückrat statt der Phosphorsäure-Pentose verfügen.
- zu mindestens 90 (95, 97)% ähnlich: Darunter ist zu verstehen, daß die mit erfaßten Polynucleotide in ihrem kodierenden Bereich bezüglich der Basenabfolge zu mindestens 90% (95%, 97%) identisch mit der Referenz (Abbildung etc.) sind und die mit erfaßten Peptide und Proteine in ihrer Primärstruktur, der Abfolge der Aminosäuren zu mindestens 90% (95%, 97%) mit der Referenz identisch sind.
- Gen: Mit dem Begriff Gen wird ein Genomabschnitt mit einer definierten Nukleotidsequenz bezeichnet, der die Information zur Synthese einer m- oder prä-mRNA oder einer sonstigen RNA (z.B. tRNA, rRNA, snRNA etc.) enthält. Es besteht aus kodierenden und nicht kodierenden Abschnitten.
- Genfragment: Nukleinsäureabschnitt, der in seiner Basenabfolge einen Teilbereich eines Gens beinhaltet
- Bindung an das Peptid oder Protein: Wechselwirkung zwischen Substanz und Peptid oder Protein, die zu Fixierung führt.
- funktionelle Parameter: darunter versteht man Meßgrößen eines Experimentes, die mit der Funktion eines Proteins (lonenkanal, Rezeptor, Enzym) korrelieren
- gentechnisch manipuliert: Manipulation von Zellen, Geweben oder Organismen derart, daß hier genetisches Material eingebracht wird
- endogen exprimiert: Expression eines Proteins, die eine Zelllinie unter geeigneten Kulturbedingungen aufweist, ohne das dieses entsprechende Protein durch gentechnische Manipulation zur Expression veranlasst wurde
- G-Protein: International übliche Abkürzung für ein Gunaosintriphosphat (GTP)-bindendes Protein, das als Signalprotein durch G-Protein gekoppelte Rezeptoren aktiviert wird
- Reportergen: Generelle Bezeichnung für Gene, deren Genprodukte sich mit Hilfe einfacher biochemischer Methoden oder histochemischer Methoden einfach nachweisen lassen, wie z.B. Luziferase, alkalische Phosphatase oder Green Fluorescent Protein (GFP).
- (rekombinantes) DNA-Konstrukt: Generelle Bezeichnung für jede Art von DNA-Molekülen, die durch die in vitro-Verknüpfung von DNA-Molekülen entstanden sind.
- Klonierungsvektor: Generelle Bezeichnung für Nukleinsäure-Moleküle, die beim Klonieren als Träger von Fremdgenen oder Teilen dieser Gene dienen.
- Expressionsvektor: Bezeichnung für speziell konstruierte Klonierungsvektoren, die nach Einbringen in eine geeignete Wirtszelle die Transcription und Translation des in den Vektor einklonierten Fremdgens erlauben.
- LTR-Sequenz: Abkürzung für Long terminal repeat. Generelle Bezeichnung für längere Sequenzbereiche, die an beiden Enden eines linearen Genoms zu finden sind. Derartige Sequenzbereiche kommen z.B. in den Genomen von Retroviren und an den Enden eukaryotischer Transposons vor.
- Poly-.A-Schwanz: die am 3'-Ende von messenger-RNAs durch Polyadenylierung angeheftenen Adenyl-Reste (ca. 20-250).
- Promotor-Sequenz: Bezeichnung für einen DNA-Sequenzbereich, von dem aus die Transkription eines Gens, d.h. die Synthese der mRNA, gesteuert wird.
- ORI-Sequenz: Abkürzung für Origin of replication. Die ORI-Sequenz erlaubt einem DNA-Molekül, sich als autonome Einheit in der Zelle zu vermehren.
- Enhancer-Sequenz: Bezeichung für relativ kurze, zum Teil als Repetitionen auftretende, genetische Elemente, die in der Regel die Expression mancher Gene in unterschiedlichem Maße verstärken.
- Transkriptionsfaktor: Bezeichnung für ein Protein, das über eine Bindung an spezifische DNA-Sequenzen, die Transkription eines Gens beeinflußt.
- kultivieren: Zellen oder Gewebe unter geeigneten Kulturbedingungen halten
- Bedingungen, die eine Expression erlauben, darunter versteht man die Auswahl und Anwendung von Kulturbedingungen die eine Expression des interessierenden Proteins erlauben, darunter gehören Temperaturänderung, Mediumwechsel, Zusatz von induzierenden Substanzen, Weglassen hemmender Substanzen.
- Inkubationszeit: Zeitdauer für die Zellen oder Gewebe inkubiert, d.h. einer definierten Temperatur ausgesetzt werden.
- Selektionsdruck: Anwendungen von Kulturbedingungen die Zellen mit einem bestimmtem Genprodukt, dem sog. Selektionsmarker, einen Wachstumsvorteil verschaffen.
- Amphibienzelle, Zelle aus einem Tier der Klasse der Amphibia
- Bakterienzelle, Zelle die dem Überreich der Eubacteria oder Archaebacteria zuzuordnen ist, oder von ihr abstammt.
- Hefezelle, Zelle die der Ordnung der Endomycetalse zuzuordnen ist, oder von ihr abstammt.
- Insektenzelle, Zelle die der Ordnung der Hexapoda zuzuordnen ist, oder von ihr abstammt.
- native Säugetierzelle, aus einem Säugetier stammende Zelle, die in ihren relevanten Merkmalen der im Organismus befindlichen Zelle entspricht.
- immortalisierte Säugetierzelle: Zelle die durch die angewendeten Kulturbedingungen oder gentechnische Manipulation die Eigenschaft erlangt hat, sich über die normalerweise übliche Teilungshäufigkeit hinaus (ca.100) in der Kultur zu teilen.
- markiert: durch entsprechende Modifizierung oder Derivatisierung für eine Nachweisreaktion zugänglich gemacht. Beispielsweise radioaktiv, fluoreszierend oder lumineszierend.
- Ligand: Substanz, die an ein im Körper oder einer Zelle befindliches Molekül, im speziellen einen Rezeptor, bindet
- Verdrängung: vollständiges oder partielles Entfernen eines Liganden von seiner Bindungsstelle
- gebundene Aktivität: Biochemisch oder physikalisch erfaßter Meßwert, der mit der an einem Rezptor gebundenen Ligandenmenge korreliert
- Regulation: die als Teil eines Regelprozese erfolgte Hemmung oder Aktivierung eines Vorgangs
- Hemmung: als Sonderfall der Regulation die Verhinderung/Minderung eines Vorgangs
- Aktivierung: als Sonderfall der Regulation die Verstärkung eines Vorgangs
- Rezeptoren, Im weitesten Sinne alle im pro- oder eukaryoten Organismus vorhandenen Moleküle, an die ein Wirkstoff binden kann. Im engeren Sinne membrangebundene Proteine oder Komplexen mehrerer Proteine, die durch Bindung eines Wirkstoffes eine Änderung in der Zelle hervorrufen.
- lonenkanäle: Membrangebundene Proteine oder Komplexe mehrerer Proteine, durch die Kationen oder Anionen durch die Membran hindurchgelangen können.
- Enzyme: Bezeichnung für Proteine oder Komplexe aus einer aktivierenden Nichteiweißkomponente mit einem Protein, die katalytische Eigenschaften besitzen.
- Genexpression (exprimieren/exprimierbar): das Übersetzen der genetischen Information eines Genes in RNA (RNA-Expression) oder in Protein (Proteinexpression).
- lonenmilieu: lonenkonzentration eines oder mehrerer Ionen in einem bestimmten Kompartiment.
- Membranpotential: Spannungsdifferenz über eine Membran aufgrund eines Überschusses an Kationen auf der einen Seite und Anionen auf der anderen Seite der Membran.
- Veränderung der Enzymaktivität: Hemmung oder Induktion der katalytischen Aktivität eines Enzyms.
- 2^{nd} messenger kleines Molekül, das als Antwort auf ein extrazelluläres Signal entweder im Cytosol gebildet wird oder in das Cytosol hineinwandert und dabei hilft die Information an das Zellinlere weiterzugeben, wie zum Beispiel cAMP, IP₃.
- (Gen-)Sonde: Bezeichnung für jede Art von Nukleinsäuren, mit deren Hilfe man ein gesuchtes Gen oder eine bestimmte DNA-Sequenz nachweisen kann. Durch Derivatisierung der Gensonde (z.B. Blotin, magnetische Beads, Digoxinin) können zudem DNA-Moleküle aus einem Gemisch herausgezogen werden. Als Sonden werden klonierte Gene, Genfragmente, chemisch synthetisierte Oligonukleotide und auch RNA verwendet, die meist radioaktiv markiert ist.
- DNA: Internationale Bezeichnung für Desoxyribonukleinsäure
- genomische DNA: Generelle Bezeichnung für die bei eukaryotischen Organismen aus dem Zellkern einer Zelle stammenden DNA.
- cDNA: Abkürzung für complementary DNA. Bezeichnung für die einzel- bzw. doppelsträngige DNA-Kopie eines RNA-Moleküls.
- cDNA-Bank/Bibliothek: Bezeichung für eine Sammlung von willkürlich klonierten cDNA-Fragmenten, die zusammengenommen die Gesamtheit aller von einer Zelle oder einem Gewebe synthetisierten RNA repäsentieren.
- cDNA-Klon: Bezeichnung für eine Population genetisch einheitlicher Zellen, die sich von einer einzigen Zelle ableiten, derart, daß diese Zelle eine künstlich eingebrachtes cDNA-Fragment enthält.
- Hybridisierung: Durch Basenpaarung bewirkte Ausbildung eines doppelsträngigen Nukleinsäuremoleküls aus zwei getrennten Einzelsträngen.
- stringente Bedingungen: Bedingungen, unter denen nur perfekt basengepaarte Nukleinsäure-Stränge gebildet werden und stabil bleiben.
- isolieren: ein gesuchtes Molekül aus einem Gemisch herausfinden und abtrennen.
- DNA-Sequenzierung: Bestimmung der Abfolge der von Basen in einem DNA-Molekül.
- Nukleinsäuresequenz: Bezeichnung für die Primärstruktur eines DNA-Moleküls, d.h. die Abfolge der einzelnen Basen, aus denen sich eine DNA zusammensetzt.
- Genspezifische Oligonukleotid Primer: Oligonukleinsäuren, also 10-40 Basen lange Nukleinsäurefragmente, die in ihrer Basenzusammensetzung eine stringente Hybridisierung an das gesuchte Gen oder die gesuchte cDNA erlauben.
- Ermitteln von Oligonukleotid Primern: Die manuelle oder Computerunterstützte Suche von Oligonukleotiden zu einer vorgegebenen DNA-Sequenz, die für eine Hybridisierung und/oder eine Polymerase-Ketten Reaktion optimal geeignet sind.
- PCR: Abkürzung für Polymerase-Kettenreaktion. In vitro-Verfahren zur selektiven Anreicherung von Nucleinsäure-Bereichen definierter Länge und definierter Sequenz aus einem Gemisch von NukleinsäureMolekülen.
- DNA-Template: Nukleinsäuremolekül oder ein Gemisch von Nukleinsäuremolekülen, aus denen ein DNA-Abschnitt mit Hilfe der PCR (s.o.) vervielfältigt wird.
- RNA: International gebräuchliche Abkürzung für Ribonukleinsäuren
- mRNA: International gebräuchliche Abkürzung für messenger-Ribonukleinsäuren, die am Transfer der genetischen Information aus dem Kern in die Zelle beteiligt sind und die Information für die Synthese eines Polypetids oder eines Proteins beinhalten.
- Antisense Polynukleotid: Eine aus mehreren natürlichen oder modifizierten Nukleinsäuren bestehendes Molekül, deren Basenabfolge komplementär zur Basenabfolge eines Teilbereiches einer in der Natur vorkommenden RNA ist.
- PNA: International gebräuchliche Abkürzung für peptidic Nucleic Acids. Hierbei bilden peptidisch verknüpfte Aminosäuren eine Kette, wobei die Aminosäuren als Seitenkette eine für die Hybridisierung mit DNA oder RNA fähigen Base trägt.
- Sequenz: Abfolge von Nukleotiden oder Aminosäuren. Im spezifischen Sinne dieser Erfindung ist damit die Nukleinsäuresequenz gemeint.
- Ribozym: Bezeichnung für eine katalytisch aktive Ribonukleinsäure (z.B. Ligase, Endonuklease, Polymerase, Exonuklease)
- DNA-Enzym: Bezeichnung für ein DNA-Molekül, das katalytische Aktivität beinhaltet (z.B. Ligase, Endonuklease, Polymerase, Exonuklease)
- katalytische RNA/DNA: generelle Bezeichnung für Ribozyme bzw. DNA-Enzyme (s.o.).
- Adenovirus: bei Vertebraten vorkommender cytopathogener Virus
- Adenoassoziiertes Virus (AAV): Gehört zur Familie der Parvoviren. Für eine effektive Vermehrung des AAV ist eine Coinfektion der Wirtszellen mit Helferviren (z.B. Herpes-, Vaccina- oder Adenoviren) erforderlich. Die Eigenschaft von AAV, stabil in das Wirtsgenom zu integrieren, macht es als Transduktionsvektor für Säugetierzellen besonders interessant.
- Herpesvirus: Viraler Erreger der Herpes-Infektion
- posttranslationale Modifikation: Veränderung an Proteinen oder Polypetiden, die nach der Translation durchgeführt wird, hierzu zählen z.B. Phosphorylierung, Glykosylierung, Amidierung, Acetylierung oder Proteolyse.
- glykosylieren: Bezeichnung für das Anhängen von einzelnen Zuckermolekülen oder ganzen Zuckerketten an Proteine.
- phosphorylieren: Bezeichnung für das Anhängen von einem oder mehreren Phosphatresten an ein Protein, bevorzugt an die OH-Gruppen der Aminosäuren Serin, Threonin oder Tyrosin.
- amidieren. Die Bezeichnung für das Umwandeln einer Carboxylfunktion in eine Amidfunktion, z.B. an den carboxyterminalen Aminosäurerest eines Peptides oder Proteins.
- mit Membrananker versehen: Posttranslationelle Modifikation eines Proteins, oder eines anderen organischen Moleküls, derart daß es durch Anhängen eines hydrophoben Moleküls, geeigneterweise eine Fettsäure oder ein Derivat derselben, an die Lipiddoppelschicht-Membran von Zellen verankert wird.
- spalten: in diesem spezifischen Fall die Spaltung eines Peptids oder Proteins in mehrere Untersequenzen.
- verkürzen: Ein aus mehreren Einzelteilen bestehendes Molekül um eine oder mehrere Teile zu verkürzen.
- Antikörper: Lösliche, oder an Zellmembranen gebundene, als immunglobuline bezeichnete Proteine mit einer spezifischen Bindungsstelle für Antigene.
- monoklonaler Antikörper: sind gegen eine einzige antigene Determinante eines Antigens gerichtete Antikörper mit extrem hoher Selektivität
- Polyklonaler Antikörper: Gemisch aus Antikörpern, die gegen mehrere Determinanten eines Antigens gerichtet sind.
- transgen: genetisch verändert
- nichthumanes Säugetier: Die Gesamtheit der Säugetiere (Klasse der Mammalia) mit Ausnahme der Spezies Mensch.
- Keim-Zelle: Zelle mit haploidem Genom, die durch Verschmelzung mit einer zweiten Keimzelle die Bildung eines neuen Organismus ermöglicht.
- somatische Zelle: diploide Zelle als Bestandteil eines Organismus
- chromosomale Einbringung: Eingriff in die Nukleotidsequenz auf chromosomaler Ebene
- Genom: Allgemeine Beschreibung für die Gesamtheit aller Gene in einem Organismus
- Vorfahr des Tieres: Ein Tier (der Vorfahr), das auf natürliche oder künstliche Weise durch Weitergabe an seinem genetischen Material in direkter Linie mit einem anderen Tier (dem Nachfahren) verwandt ist.
- exprimierbar: Ein Nukleinsäuremolekül ist dann exprimierbar, wenn es die Information zur Synthese eine Proteins oder Polypetids beinhaltet und mit ensprechenden regulatorischen Sequenzen versehen ist, die eine Synthese dieses Proteins oder Polypeptids in vitro oder in vivo erlauben. Wenn diese Voraussetzungen nicht mehr gegeben sind, beispielsweise durch Eingriff in der kodierenden Sequenz, ist das Nukleinsäuremolekül nicht mehr exprimierbar.
- Nagetier: Tier aus der Ordnung der Rodentia, z.B. Ratte oder Maus
- als schmerzregulierende Substanz identifizierbar: Substanz die bei Einbringung in einen lebenden Organismus eine Verhaltensänderung bewirkt, die der Fachmann als schmerzhemmend bezeichnet (antinozizeptiv, antihyperalgetisch oder antiallodynisch). Im Falle des Screeningverfahrens bezieht sich das darauf, daß die Substanz beim Screening durch stärkere Bindung oder Auslösung einer Änderung eines funktionellen Parameters deutlich, beispielsweise 100 %, die Bindung oder Wechselwirkung des Durchschnitts der getesteten Substanzen übertrifft.
- Verbindung: ein anderer Name für Molekül, als aus mehreren Atomen bestehend, hier ein durch das erfindungsgemäße Verfahren identifiziertes Molekül.
- Wirkstoff: Eine Verbindung, die bei Anwendung an einem Organismus eine Veränderung in diesem Organismus hervorruft. Im Besonderen werden darunter organisch-chemisch synthetisierte Moleküle verstanden, die auf den Organismus eine heilende Wirkung ausüben. Hier insbesondere Moleküle, die an die erfindungsgemäßen Proteine und Peptide binden.
- niedermolekular: Molekül mit einem Molekulargewicht < 2kDa
- Arzneimittel: ein Stoff entsprechend der Definition im Artikel 1 §2 des Gesetzes über den Verkehr mit Arzneimitteln
- Diagnostikum: Verbindung oder Verfahren, das verwendet werden kann, um eine Krankheit zu diagnostizieren
- Behandlung von Schmerz: Verfahren, mit dem Ziel Schmerzen zu lindern oder aufzuheben, oder das zu erwartende Auftreten von Schmerzen zu hemmen (preemptive Analgesie).
- chronischer Schmerz: eine Schmerzempfindung von länger anhaltender Dauer, oft dadurch gekennzeichnet, daß sie über Zeitpunkt und Ort des initialen Stimulus hinausreicht, die Schmerzempfindlichkeit des Körpers steigert.
- Gentherapie: Unter Gentherapie versteht man alle Verfahren, die das Ziel haben, genetische Erkrankungen durch geeignete Veränderungen des Genoms kausal zu behandeln.
- In-vivo-Gentherapie: Einbringen von genetischem Material in den lebenden Organismus mit dem Ziel der Gentherapie. Man kann zwischen somatischem und Keimbahn-Eingriff unterscheiden, der einmal an diploiden Zellen und einmal an haploiden Zellen stattfindet.
- In-vitro-Gentherapie: Einbringen von genetischem Material in Zellen außerhalb des menschlichen Körpers mit dem Ziel diese nachher wieder durch Einbringen in den menschlichen Körper zur Gentherapie zur verwenden.
- Diagnostik: Verfahren, um eine Krankheit zu identifizieren.
- Wirksamkeitsuntersuchung: Untersuchung mit dem Ziel die Wirksamkeit einer Verbindung nach Einwirkung auf einen lebenden Organismus zu untersuchen.

In einer bevorzugten Ausführungsform des Verfahrens wird die Zelle vor dem Schritt (a) gentechnisch manipuliert. Dabei wird genetisches Material in die Zelle eingebracht, insbesondere eine oder mehrere Polynukleotidsequenzen. In einer weiter bevorzugten Variante dieser Ausführungsform erlaubt die gentechnische Manipulation die Messung mindestens eines der durch die Testsubstanz veränderten funktionellen Parameter. In dieser Ausführungsform werden durch gentechnische Manipulation Voraussetzungen geschaffen unter denen die Veränderung eines funktionellen Parameters überhaupt oder verbessert gemessen werden kann. Dabei ist es insbesondere bevorzugt, daß durch die gentechnische Manipulion eine in der Zelle nicht endogen exprimierte Form eines G-Proteins exprimiert oder ein Reportergen eingeführt wird. Darunter ist insbesondere die gentechnische Einführung eines endogen nicht vorhandenen oder physiologisch nicht exprimierten G-Proteins (GTPbindenden Proteins) in die Zelle zu verstehen, beispielsweise die Einführung eines chimären G-Proteins, das eine Veränderung des Signalweges erlaubt oder eines promiskuitiven G-Proteins, das sehr bindungsfreudig ist Die Einführung eines Reportergens wiederum erlaubt die Messung einer (extrazellulär ausgelösten) induzierten Expression des Genproduktes.

In einer weiteren bevorzugten Ausführungsform wird die Zelle gentechnisch so manipuliert, daß die Zelle mindestens ein Polynukleotid gemäß einer der Abbildungen 1a), 1c), 1e), 1g), 2a), 2c) oder 2e) oder ein dazu zu mindestens 90 % ähnliches Polynukleotid enthält. Damit kann beispielsweise erreicht werden, daß ein Protein, das in der im Verfahren verwendeten Zelle oder Präparation nicht endogen exprimiert wird, von der Zelle synthetisiert wird. Dabei ist es insbesondere bevorzugt, wenn das Polynukleotid in einem rekombinanten DNA-Konstrukt enthalten ist. Unter einem (rekombinanten) DNA-Konstrukt versteht man ein in-vitro hergestelltes DNA-Molekül.

Wenn beim Verfahren vor dem Schritt a) die Zelle gentechnisch manipuliert wird, ist es bevorzugt, daß die Zelle nach der gentechnischen Manipulation und vor dem Schritt (a) unter Bedingungen, die eine Expression erlauben, kultiviert wird, gegebenenfalls unter Selektionsdruck. Unter kultivieren versteht man, Zellen oder Gewebe bei Bedingungen, die ein Überleben der Zellen, bzw. deren Nachfolgegeneration sichern, zu halten. Dabei sollten die Bedingungen hier so gewählt werden, daß eine Expression des durch die gentechnische Manipulation eingefügten Materials ermöglicht wird. Dazu sollten pH, Sauerstoffgehalt, und Temperatur physiologisch gehalten sein und ausreichend Nährstoffe und notwendige Cofaktoren beigefügt sein. Der Selektionsdruck erlaubt, nur die Zellen weiter zu kultivieren, bei denen die gentechnische Manipulation zumindest teilweise erfolgreich war. Dazu gehört beispielsweise die Einführung einer Antibiotikaresistenz über das DNA-Konstrukt.

Es ist bei dem erfindungsgemäßen Verfahren besonders bevorzugt, wenn die verwendete Zelle eine Amphibienzelle, Bakterienzelle, Hefezelle, Insektenzelle oder eine immortalisierte oder native Säugetierzelle ist. Beispiele für Amphibienzellen sind Xenopus Oocyten, für Bakterienzellen E-coli-Zellen, für Hefezellen solche auch Saccharomyces cerevislae, für Insektenzellen Sf9-Zellen, für immortalisierte Säugetierzelle HeLa-Zellen und für native Säugetierzellen die CHO (Chinese Hamster Ovary)-Zelle.

Bei einer Alternative der Meßmethode zur Feststellung der Bindung der Substanz an ein Protein im erfindungsgemäßen Verfahren erfolgt die Messung der Bindung über die Verdrängung eines bekannten markierten Liganden vom Protein und/oder über die daran gebundene Aktivität einer markierten Testsubstanz. Dabei ist ein Ligand ein mit hoher Spezifität an das Protein bindendes Molekül, das durch eine ebenfalls bindende, zu testende Substanz aus der Bindungsstelle verdrängt wird. Unter Markierung ist eine den Nachweis erleichternde künstliche Modifikation am Molekül zu verstehen. Beispiele sind radioaktive, fluoreszierende oder lumineszierende Markierung.

Bei einer anderen Alternative der Meßmethode zur Feststellung der durch die Bindung der Substanz an ein Protein im erfindungsgemäßen Verfahren ausgelösten Veränderung der funktionellen Parameter, erfolgt die Messung mindestens eines der durch die Testsubstanz veränderten funktionellen Parameter über Messung der Regulation, Hemmung und/oder Aktivierung von Rezeptoren, lonenkanälen und/oder Enzymen, insbesondere über Messung der Veränderung der Genexpression, des lonenmilieus, des pH oder des Membranpotentials, über Veränderung der Enzymaktivität oder der Konzentration der 2^{nd} messenger. Damit ist auf der einen Seite direkt die Messung der Wirkung der Substanz über die Beeinflußung von Rezeptoren, Ionenkanälen und/oder Enzymen erfaßt, auf der anderen Seite als zu messende Beispiele sich ändernder Parameter wie Genexpression, Ionenmilieu, pH, Membranpotential, Enzymaktivität oder Konzentration der 2^{nd} messenger. Dabei versteht man unter Ionenmilieu insbesondere die Konzentration eines oder mehrer Ionen in einem Zelkompartiment, insbesondere dem Cytosol, unter Membranpotential die Ladungsdiffferenz zwischen zwei Seiten einer Biomembran und unter 2^{nd} messenger Botenstoffe des intrazellulären Signalwegs wie z.B. zyklisches AMP (cAMP), Inosotoltriphosphat (IP3) oder Diacylglycerol (DAG).

In diesem bzw. diesen Verfahren erfaßt ist die Verwendung von Proteinen mit bekannter Sequenz und Funktion, ohne daß für diese im Stand der Technik eine Funktion im Schmerz bekannt war.

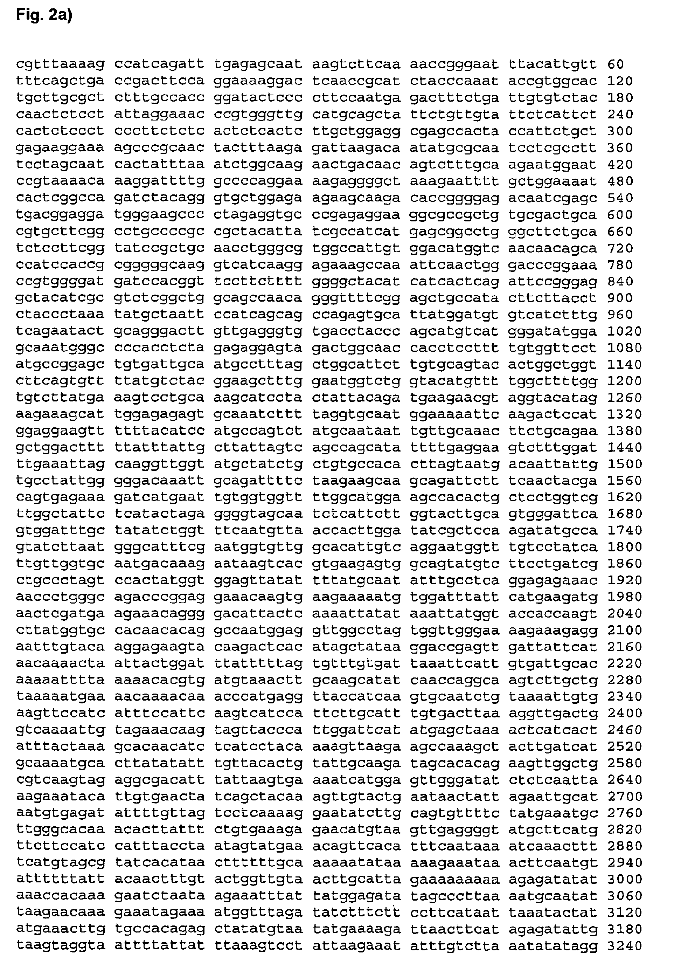

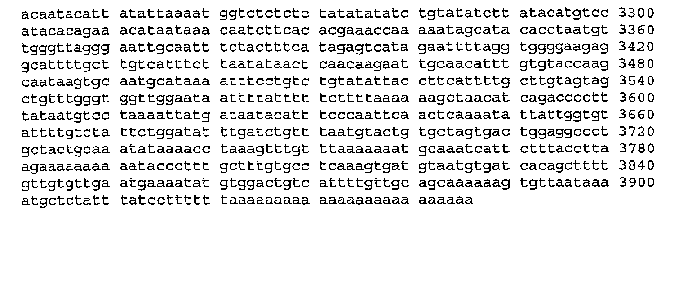

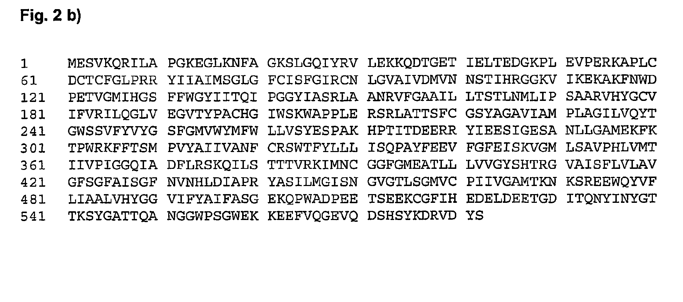

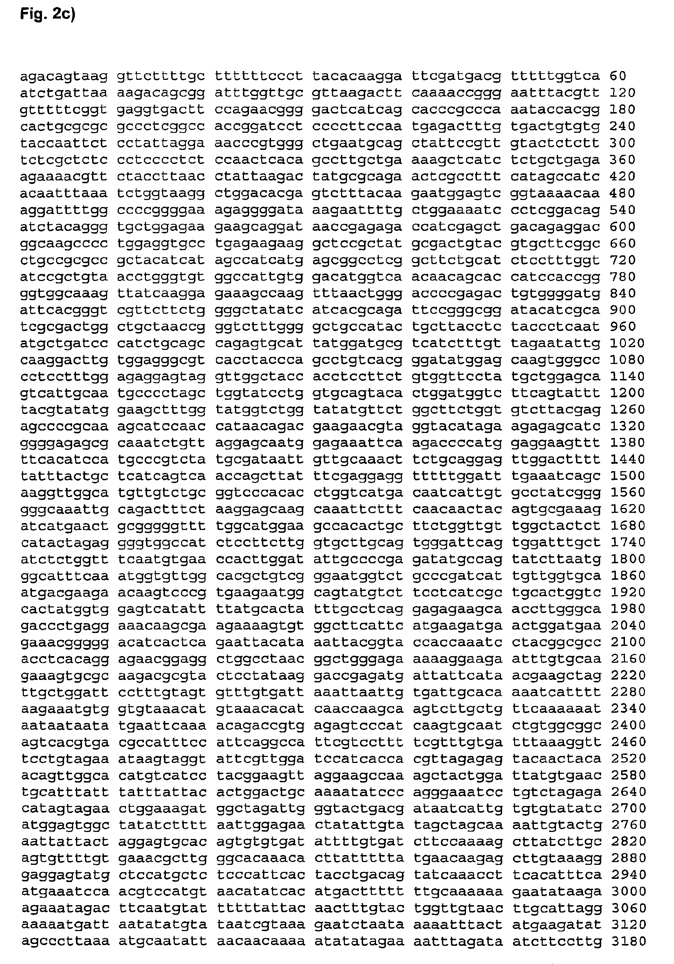

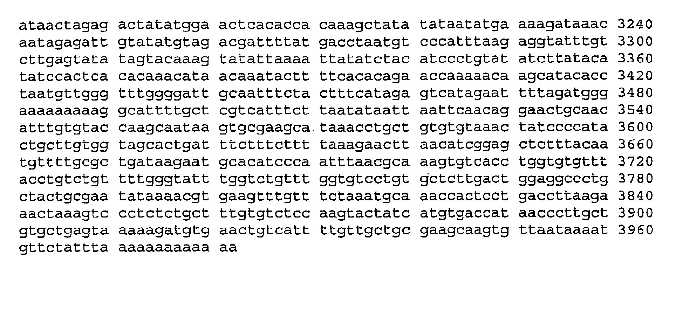

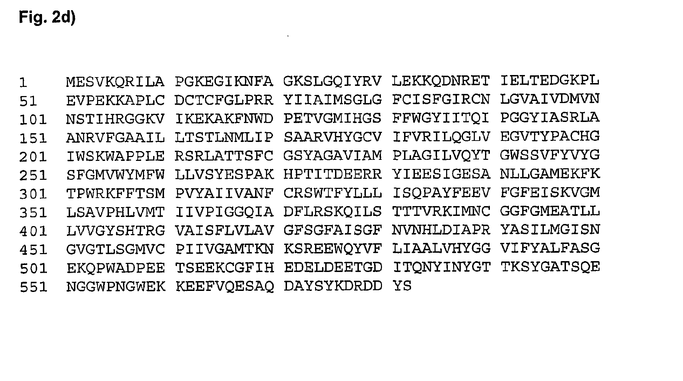

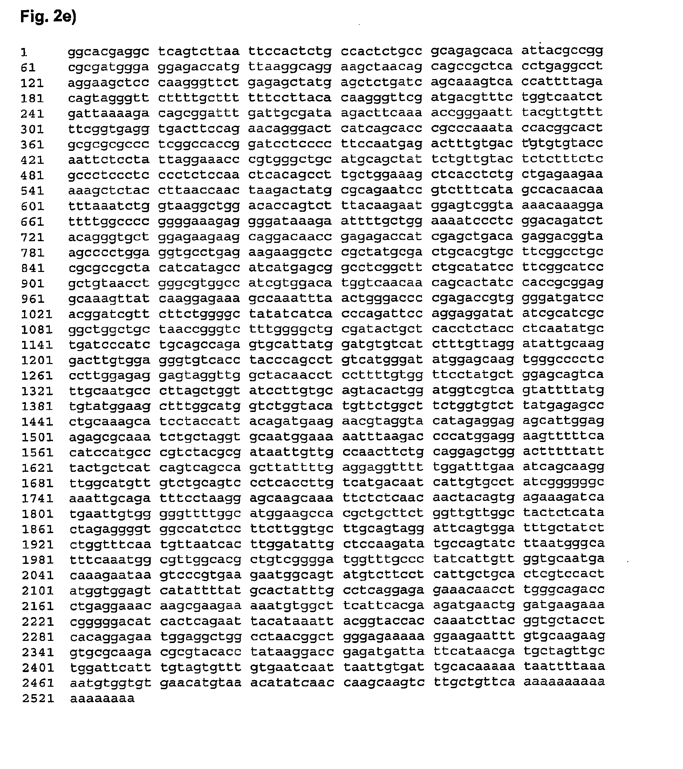

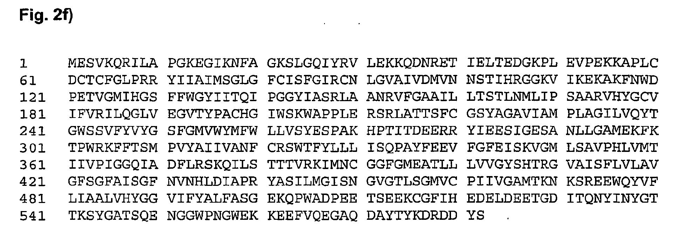

Eine weitere besonders bevorzugte Ausführungsform ist ein erfindungsgemäßes Verfahren, bei dem ein erstes der bisher beschriebenen erfindungsgemäßen Verfahren mit einem zweiten der bisher beschriebenen erfindungsgemäßen Verfahren derart gekoppelt wird, daß die Meßwerte und Ergebnisse des ersten Verfahrens hinsichtlich der zu messenden Substanz mit den Meßwerten und Ergebnissen des zweiten Verfahrens hinsichtlich der zu messenden Substanz verglichen werden, dadurch gekennzeichnet, daß in einem der zwei Verfahren, im folgenden Hauptverfahren genannt, im Schritt (a) die zu testende Substanz
entweder
mit einem Biomolekül aus Gruppe II: dem Protein BNPI und/oder einem Protein gemäß einer der Abbildungen 1b), 1d), 1f) oder 1h) und/oder einem zu einem dieser vorgenannten Proteine zu mindestens 90 % ähnlichen Protein und/oder einem Protein, für das ein Polynukleotid gemäß einer der Abbildungen 1a), 1c), 1e) oder 1g) oder ein dazu zu mindestens 90 % ähnlichen Polynukleotid kodiert, und/oder einem Protein, das durch eine Nukleinsäure kodiert wird, die unter stringenten Bedingungen an ein Polynukleotid gemäß einer der Abbildungen 1a), 1c), 1e) oder 1g) oder deren Antisense Polynukleotide bindet und/oder einer Zelle und/oder einer Präparation aus einer solchen Zelle, die mindestens eines der vorgenannten Proteine synthetisiert hat,
oder
mit einem Biomolekül aus Gruppe III: dem Protein DNPI und/oder einem Protein gemäß einer der Abbildungen 2b), 2d) oder 2f) und/oder einem zu einem dieser vorgenannten Proteine zu mindestens 90 % ähnlichen Protein und/oder einem Protein, für das ein Polynukleotid gemäß einer der Abbildungen 2a), 2c) oder 2e) oder ein dazu zu mindestens 90 % ähnliches Polynukleotid kodiert, und/oder einem Protein, das durch eine Nukleinsäure kodiert wird, die unter stringenten Bedingungen an ein Polynukleotid gemäß einer der Abbildungen 2a), 2c) oder 2e) oder deren Antisense Polynukleotide bindet und/oder einer Zelle und/oder einer Präparation aus einer solchen Zelle, die mindestens eines der vorgenannten Proteine synthetisiert hat, inkubiert wird,
und
daß im anderen der zwei Verfahren, im folgenden Nebenverfahren genannt, im Schritt (a) die zu testende Substanz mit einem Biomolekül aus der Gruppe I oder mit einem Biomolekül aus derjenigen Gruppe ausgewählt aus Gruppe II und Gruppe III inkubiert wird, aus der das Biomolekül, mit der die Substanz im Hauptverfahren inkubiert wird, nicht ausgewählt ist.

Unter dieser besonders bevorzugten Ausführungsform ist insbesondere die Kombination der Messung der Bindung an BNPI oder davon abgeleiteten Biomolekülen oder der Messung der daraus entstehenden Modifikation zellulärer Parameter auf der einen und der Bindung an DNPI und jeweils davon abgeleiteten Biomolekülen oder der Messung der daraus entstehenden Modifikation zellulärer Parameter auf der anderen Seite zu verstehen, da gerade ein Vergleich angesichts der vollkommen getrennten aber eng aneinanderliegenden Verteilung der beiden Kanäle im Gewebe einen wichtigen Aufschluß über physiologische Funktionen geben kann. Damit erlaubt aber der differentielle Abgleich der Daten die Identifizierung optimiert pharmazeutisch bzw. medizinisch wirksamer Substanzen.

Weiter bevorzugt ist ein erfindungsgemäßes Verfahren worin der durch die aufzufindende Substanz regulierte Schmerz ausgewählt ist aus:
chronischem Schmerz, insbesondere muskuloskelettalem Schmerz; neuropathischem Schmerz, insbesondere allodynischem Schmerz, mechanischer Hyperalgesie oder diabetischer Neuropathie; viszeralem Schmerz, cerebralem Schmerz, peripherem Schmerz oder entzündungsbedingtem Schmerz, insbesondere peripherem Entzündungsschmerz; sowie Migräne, Cluster-Kopfschmerz oder den Schmerz bei Trigeminus Neuralgie.

Eine durch ein erfindungsgemäßes Verfahren identifizierbare Verbindung ist eine schmerzregulierende Substanz. Hierbei bezieht sich Verbindung insbesondere auf niedermolekulare Wirkstoffe, aber auch auf Peptide. Proteine und Nukleinsäuren. Dabei bedeutet identifizierbar, daß die Verbindung das Merkmal aufweist, daß es beim erfindungsgemäßen Screeningverfahren bezüglich der Bindung deutlich stärker, vorzugsweise doppelt so stark bindet wie der Durchschnitt der zu testenden Substanzen oder bezüglich der Änderung der funktionellen Parameter deutlich vom Durchschnitt der zu testenden Substanzen abweicht. Besonders bevorzugt ist es, wenn die Verbindung eine niedermolekulare Verbindung ist.

Mit den in den erfindungsgemäßen Verfahren eingesetzten Polynukleotiden sind auch die dargestellten Genfragmente selbst gemeint, wie auch ein Polynukleotid, das entweder vollständig oder zumindest Teilen der kodierenden Sequenz des dem Fragment entsprechenden Gens entspricht. Damit sind auch Polynukleotide gemeint, die mindestens 90%-ige, vorzugsweise 95%-ige, insbesondere wenigstens 97%-ige Übereinstimmung in der Basenabfolge mit der kodierenden Sequenz der abgebildeten Polynukleotide oder der kodierenden Sequenz der Gens aufweisen. Es ist weiter bevorzugt, daß es sich bei dem Polynukleotid um RNA bzw. ein- oder doppelstängige DNA, insbesondere mRNA oder cDNA handelt. Ebenso ist es bevorzugt, daß es sich bei dem Polynukleotid um ein Antisense-Polynukleotid oder PNA handelt, das eine Sequenz aufweist, die in der Lage ist, spezifisch an ein erfindungsgemäßes Polynukleotid zu binden. Dabei versteht man unter PNA "peptidic nucleic acid" (peptidische Nukleinsäure), die zwar die Basenpaare trägt, aber deren Rückrat peptidisch gebunden ist. Ein Antisense-Polynukleotid zeigt die komplementäre Basenabfolge zu mindestens einem Teil einer Basis-Nukleinsäure. Ebenfalls bevorzugt ist es, daß das Polynukleotid Teil eines Ribozym oder sonstigen DNA-Enzyms oder einer katalytischen RNA bzw. DNA ist. Unter Ribozym ist eine katalytisch aktive Ribonukleinsäure zu verstehen, unter DNA-Enzym ein entsprechende Desoxyribonukleinsäure, also katalytische RNA beziehungsweise DNA.

Bevorzugt ist es für ein verwendetes Protein, wenn diese posttranslational modifiziert wurde, es insbesondere glykosyliert, phosphoryliert, amidiert, methyliert, acetyliert, ADP-ribosyliert, hydroxyliert, mit einem Membrananker versehen, gespalten oder verkürzt wurde. Posttranslationale Modifikationen sind beispielsweise dem Voet/Voet, Biochemistry, 1^{st} Edition, 1990, S. 935-938 zu entnehmen.

Die Verabreichung der durch ein erfindungsgemäßes Verfahren identifizierten Verbindungen kann beispielsweise in Form eines Arzneimittels erfolgen.

Insgesamt ist eine wichtige Grundlage der Erfindung die Identifizierung schmerzregulierter Gene und Genfragmente. Darauf basiert das Screeningverfahren. Aber auch die Verwendung zur Diagnose oder Therapie bietet sich wie bereits ausgeführt an. Im folgenden werden entsprechende Anwendungsmöglichkeiten und weitere Ausführungsbeispiele erläutert.

### 1. Therapie chronischer Schmerzen

Die mRNA-Expression der Kinasen wurde durch in-situ-Hybridisierung im Rückenmarksgewebe untersucht. Im Rückenmark projizieren die primären sensorischen Neurone auf nachgeschaltete zentralnervöse Neurone, es handelt sich hierbei neben supraspinalen Vorgängen um die zentrale Umschaltstelle für nozizeptive Information. Zahlreiche Experimente konnten zeigen, daß der Entwicklung chronischer Schmerzzustände plastische Veränderungen des Nervensystems zugrundeliegen (als Überblick siehe Corderre et al., 1993; Zimmermann und Herdegen, 1996). Insbesondere In den Neuronen der dorsalen Wurzelganglien und des Rückenmarks sind plastische Veränderungen beschrieben worden, die mit der Regulation schmerzrelevanter Gene einhergeht. So ist für eine Reihe von Neurotransmitter-Rezeptoren, die für die Schmerztherapie von Bedeutung sind, eine Gen-Regulation im Rückenmark beschrieben worden (siehe Tabelle 1). Auf dieser Grundlage könnten die gefundenenen, unter Schmerz regulierten cDNA-Sequenzen zur Therapie (Gentherapie, Antisense, Ribozyme) und Diagnose chronischer Schmerzzustände verwendet werden.

### 1.1 Antisense-Strategien

Hierbei werden, abgeleitet von der Nukleinsäuresequenz der vollständigen cDNA oder von Teilbereichen Konstrukte erstellt, die die mRNA oder Proteinkonzentration herabsetzen können. Dies können z.B. antisense-Oligonukleotide (DNA oder RNA) sein, die eventuell unter Verwendung modifizierter Nukleotidbausteine ( z.B. O-Allyl-Ribose) eine erhöhte Stabilität gegenüber Nukleasen aufweisen. Zudem ist die Verwendung von Ribozymen denkbar, die als enzymatisch aktive RNA-Moleküle eine spezifische Spaltung der RNA katalysieren. Daneben könnten auch Vektoren eingesetzt werden, die die hier verwendeten Sequenzen oder Teilbereiche dieser Nukleotidsequenzen unter Kontrolle eines geeigneten Promotors exprimieren und somit für eine in-vivo oder ex-vivo Therapie geeignet sind. Zusätzlich sind auch Antisense-Konstrukte möglich, die unter Austausch des Phosphatrückgrats von Nukleotidsequenzen (z.B. PNAs, d.h. Peptide Nucleic Acids) oder Verwendung nichttraditioneller Basen wie Inosine, Queosine oder Wybutosine sowohl wie Acetyl-, Methyl-, Thio- und ähnlich modifizierte Formen von Adenin, Cytidin, Guanosin u, Thymidin und Uridin nicht oder in geringerem Masse durch endogene Nukleasen abgebaut werden können.

### 1.2. Antagonisten/ Agonisten bzw. Inhibitoren/Aktivatoren der im Screeningverfahren verwendeten Genprodukte.

Dies umfaßt Substanzen, die durch eine Bindung an das Genprodukt dessen Funktion verändern. Dies können sein:
1.2.1. Organisch-chemische Moleküle, die im Rahmen eines Wirkstoffscreenings unter Verwendung der Genprodukte der erfindungsgemäß eingesetzten cDNA als Bindungspartner gefunden werden.
1.2.2. Antikörper, seien es polyklonale, chimäre, single-chain, F_{ab}-Fragmente oder Fragmente aus Phagen-Banken, die bevorzugt als neutralisierende Antikörper über eine Bindung an die Genprodukte spezifisch die Funktion beeinflußen.
1.2.3. Aptamere, d.h. Nukleinsäuren oder Nukleinsäurederivate mit Proteinbindenden Eigenschaften. Dazu gehören auch sog. Spiegelmere, die durch Spiegelevolution gewonnene spiegelbildliche und damit stabile Oligonukleotide darstellen, die hochaffin und hochspezifisch ein Zielmolekül binden können (Klußmann et al., 1996).

### 1.3. Gentherapie

Die beschriebenen Sequenzen können zur Therapie neurologischer Erkrankungen insbesondere chronischer Schmerzustände eingesetzt werden, indem sie nach Klonierung in geeignete Vektoren (z. B. Adenovirus-Vektoren oder adeno-assozierter-Virus-Vektoren) zur in vivo oder ex-vivo Therapie verwendet werden, um dort z.B. einer Überexpression oder Unterexpression des endogenen Genproduktes entgegenzusteuem, die Sequenz des defekten Genproduktes zu korrigieren (z. B. durch Transsplicing mit dem exogenen Konstrukt) oder ein funktionelles Genprodukt zur Verfügung zu stellen.

### 2. Diagnose

Polynukleotidsequenzen (Oligonukleotide, antisense -DNA & RNA-Moleküle, PNAs), die von den im Screeningverfahren etc. verwendeten Nukleotidsequenzen abgeleitet sind, könnten zur Diagnose von Zuständen oder Erkrankungen eingesetzt werden, die mit einer Expression dieser Gensequenzen assoziiert sind. Beispiele dieser Zustände oder Erkrankungen beinhalten neurologische Erkrankungen inklusive chronischer Schmerzen oder neuropathischer Schmerzen (hervorgerufen z.B. durch Diabetes, Krebs oder AIDS) oder neurodegenerativer Erkrankungen wie Alzheimer, Parkinson, Chorea Huntington, Jacob-Creutzfeld, amyotrophe Lateralsklerose und Demenzen. Die Nukleotidsequenzen können auf vielfältige Weise (Northemblot, Southernblot, FISH-Analyse, PRINS-Analyse, PCR) entweder zur Identifizierung der Genproduktes oder abweichender diagnostisch relevanter Genprodukte oder zur Quantifizierung des Genproduktes dienen. Neben der Nukleinsäurediagnostik können auch Antikörper oder Aptamere gegen das von den erfindungsgemäß verwendeten Nukleinsäuren kodierte Protein zur Diagnostik eingesetzt werden (z. B. mittels ELISA, RIA, immuncytochemische oder immunhistochemische Verfahren), um das Protein oder abweichende Formen zu identifizieren und das Protein zu quantifizieren.

Im Hinblick auf eine Gendiagnostik könnten Nukleinsäure-Sonden abgeleitet von den erfindungsgemäß verwendeten Nukleotidsequenzen zur Bestimmung des Gen-Lokus eingesetzt werden (z.B. durch FISH, FACS, artifizielle Chromosomen wie YACs, BACs oder P1-Konstrukte).

Die folgenden Beispiele und Abbildungen sollen die Erfindung erläutern, ohne sie darauf zu beschränken.

### Abbildungen und Beispiele

### Abbildungen:

- Fig. 1a): cDNA-Sequenz von BNPI, human; AN: NM_020309
- Fig. 1 b): Aminosäure-Sequenz von BNPI human; AN: NM_020309
- Fig. 1c): cDNA-Sequenz von BNPI, human; Nr.: AAT42064 aus WO96/34288
- Fig. 1d): Aminosäure-Sequenz von BNPI, human; Nr.: AAT42064 aus WO96/34288
- Fig. 1e): cDNA-Sequenz von BNPI, Ratte; AN: U07609
- Fig. 1f): Aminosäure-Sequenz von BNPI, Ratte; AN: U07609
- Fig. 1g): cDNA-Sequenz von BNPI, Maus; AN: XM_133432
- Fig. 1h): Aminosäure-Sequenz von BNPI, Maus; AN: XM_133432
- Fig. 2a): cDNA-Sequenz von DNPI, human; AN: AB032435
- Fig. 2b): Aminosäure-Sequenz von DNPI, human; AN: AB032435
- Fig. 2c): cDNA-Sequenz von DNPI, Ratte; AN: AF271235
- Fig. 2d): Aminosäure-Sequenz von DNPI, Ratte; AN: AF271235
- Fig. 2e): cDNA-Sequenz von DNPI, Maus; AN: NM_080853
- Fig. 2f): Aminosäure-Sequenz von DNPI, Maus;AN: NM_080853
- Fig. 3): Auftrennung von radioaktiv-markierten RFDD-PCR-Fragmenten in einem 6%-igen denaturierenden PAA-Gel (s. Beispiel 1)
- Fig. 4): Hochregulation der DNPI- und BNPI-Proteinexpression in primären sensorischen Rattten-DRG-Neuronen und Fasern nach Collageninduzierter Arthritis. (s. Beispiel 2)
- Fig. 5): Differentielle Expression von DNPI und BNPI in Synapsen der Schmerzleitung und motorischen Arealen des lumbaren Rückenmarks der Ratte (s. Beispiel 3a)
- Fig. 6): Differentielle Expression von DNPI und BNPI in Synapsen der Dorsalhorn-Schmerzleitung-Arealen des lumbaren Rückenmarks der Ratte (s. Beispiel 3b)
- Fig. 7): Differentielle Expression von DNPI und BNPI in Synapsen der Schmerzleitung des sakralen Rückenmarks der Ratte (s. Beispiel 3c)
- Fig. 8): Differentielle Expression von DNPI und BNPI In Synapsen der medullo-cervicospinalen Schmerzleitung des Trigeminalnervs der Ratte (s. Beispiel 3d)
- Fig. 9): Differentielle Expression von DNPI und BNPI in schmerzrelevanten Hinregionen der Ratte (s. Beispiel 3e)
- Fig.10): Differentielle Expression von DNPI und BNPI in schmerzrelevanten Hinregionen der Ratte (s. Beispiel 3f)
- Fig. 11): Differentielle Expression von DNPI und BNPI in schmerzrelevanten Hinregionen der Ratte (s. Beispiel 3g)
- Fig.12): Differentielle Expression von DNPI und BNPI in schmerzrelevanten Hinregionen der Ratte (s. Beispiel 3h)

### Beispiele:

### Beispiel 1: Identifizierung schmerzregulierter Gene mittels RFDD-PCR

### A) Vorgehen

Es wurde folgendes Vorgehen gewählt:
Als Ausgangspunkt für die Isolierung schmerzregulierter Gene wurde die CFA-induzierte Arthritis an der Ratte gewählt, bei der das Complete Freund Adjuvans in die Schwanzwurzel injiziert wird. Das Zielgewebe, in dem die schmerzregulierte Expression der erfindungsgemäß verwendeten Gene nachgewiesen wurde, waren die dorsalen Wurzelganglien des fünften Lumbalsegmentes. Zur Isolierung differentiell regulierter Gene stehen vier Methoden zur Verfügung:
- cDNA-RDA (cDNA-representational difference analysis; Hubank & Schatz, 1994)
- DDRT-PCR (Differential Display RT-PCR; Liang & Pardee 1992, Bauer et al., 1994), Hiervon gibt es inzwischen verbesserte Modifikationen, wie die sogenannte"Restriction-fragment-differential-display-PCR" (RFDD-PCR), die druch zusätzliche Restriktionsfragmentierung der cDNA in Verbindung mit einer optimierten PCR-Amplifikation eine reproduzierbarere Reaktion erlaubt und zudem vermehrt Fragmente in der kodierenden Regoin detektiert (Ivanova et al., 1995).
- Subtraktive Hybridisierung (Watson & Margulies, 1993)
- SAGE (Serial Analysis of Gene expression, Velculescu et al., 1995).

Eine vergleichende Bewertung der genannten Methoden führte zur Auswahl der RFDD-PCR, da diese Methode im Gegensatz zur subtraktiven Hybridisierung und der SAGE in der Lage ist, sowohl hoch- und herunterregulierte Gene als auch seltene Transkripte zu erfassen und darüberhinaus innerhalb kurzer Zeitspannen eine Fülle von Ergebnissen liefert.

### B) MATERIAL UND METHODEN

### Isolierung und Charakterisierung schmerzregulierter cDNA-Sequenzen

### Tiermodell: CFA-induzierten Polyarthritis

Die Adjuvans Arthritis (AA) ist eine induzierte Form der (sub)chronischen Arthritis. Sie wird durch Immunisierung von Ratten mit einer Suspension von Mycobakterien in Öl induziert. Bei der dadurch ausgelösten Erkrankung handelt es sich um eine T-Zell-vermittelte Autoimmun-Arthritis, die - da bei der Induktion jedoch kein definiertes Autoantigen eingesetzt wird - einer spontan auftretenden Arthritis im Menschen entspricht. Die AA wird häufig für Untersuchungen immunologischer Aspekte der Rheumatoiden Arthritis genutzt. Darüber hinaus wird das Modell zur Testung antiinflammatorischer und analgetischer Substanzen herangezogen. Die AA ist eine ziemlich aggressive Form der Arthritis. Der Entzündungsprozess der AA ist zwar selbstheilend, dennoch bleiben schwere Gelenkveränderungen bestehen. Die Schwere der Erkrankung kann über das Aufstellen eines Arthritis Indexes quantifiziert werden. Dabei werden alle vier Pfoten auf Rötung, Schwellung und Deformation der Gelenke untersucht. Ferner kann der Krankheitsverlauf über die Bestimmung des Körpergewichts, der Pfotenschwellung mittels Plethysmographie sowie durch histologische Untersuchungen der Gelenke näher charakterisiert werden.
Die Arthritis wird durch intracutane Injektion von CFA (100 µl der 5 mg/ml Stammlösung) in die Schwanzwurzel (dorsal) induziert. Durch tägliche Beobachtung der Tiere auf Beweglichkeit, Hautrötungen, Schwellungen von Tarsal- und Carpalgelenk wird der Schweregrad der Arthritis anhand eines Scoring-Indexes bestimmt. Das Einsetzen sichtbarer Entzündungen von Tarsal- oder Carpalgelenk beginnt etwa an Tag 10 nach Immunisierung. Die Schwere der Erkrankung nimmt über einen Zeitraum von 10-14 Tagen zu, erreicht ein Optimum, das für etwa 6-7 Tage gehalten wird, um dann wieder abzuklingen. Wurden Ratten nur mit IFA immunisiert, wurde keine Arthritis ausgelöst.

**Gewebeentnahme**. Die Tier werden dekapitiert, und die dorsalen Wurzelganglien nach Lubalektomie herauspräpariert und sofort in flüssigem Stickstoff eingefroren.

**RNA-Isolierung**. Aus den Gewebeproben wurde die Gesamt-RNA mit dem Trizol-Kit (Life Technologies) nach Angaben des Herstellers isoliert. Die RNA wurde UV-spektrometrisch quantifiziert (Extinktion bei 260nm) und durch denaturierende Gelelektropherese in einem Formaldehyd-Agarosegel (Sambrook et al., 1989) auf Integrität überprüft.

**DNase-Verdau.** Vor Einsatz in die DDRT-PCR werden eventuelle Spuren an genomischer DNA durch DNase-Verdau entfernt. Hierbei wurden je 6µg RNA in einem Gesamtvolumen von 100µl in 1X First-Strandbuffer (Life Techn.) und 10 Units RNase-freie DNasel (Boehringer Mannheim) für 15 Minuten bei 37°c inkubiert. Nach Phenol-Chloroform-Extraktion wurde die RNA durch Zugabe an 1/10 Vol. Natriumacetat pH5.2 und 2.5 Vol. Ethanol präzipitiert, in DEPC-Wasser gelöst, UV-spektrometrisch quantifiziert und durch erneute Formaldehyd-Agarose-Gelelektrophorese charakterisiert.

**Reverse Transkription.** Je 1 µg DNasel-verdaute RNA wurden mit Hilfe des displayProfile-Kits (Firma Display Systems Biotech, Vista, USA) nach Angaben des Herstellers revers transkribiert und Doppel-Strang cDNA erzeugt. Nach Aufreinigung der cDNA durch Phenol-Chloroform - Extraktion und Ethanol-Präzipitation wurde die Effizienz der cDNA-Synthese durch Gelektrophorese in einem 1.5%igen Agarosegel nachgewiesen.

**Taq1-Restriktionsverdau.** Je 10 µl der doppelsträngigen cDNA wurden mit dem Restriktionsenzym Taq1 verdaut. Dies wurde ebenfalls mit Hilfe des displayProfile-Kits (Firma Display Systems Biotech, Vista, USA) nach Angaben des Herstellers durchgeführt. Ausgehend von diesem Ansatz werden Adaptoren an die verdaute cDNA ligiert.

**³³P-Endmarkierungsreaktion.** Zur nachfolgenden Detektion der Fragmente wurde einer der beiden Primer (sog. O-Extension Primer) durch eine Endmarkierungsreaktion mit der T4 Polynukleotidkinase und [γ³³P]ATP radioaktiv markiert. Dies wurde ebenfalls mit Hilfe des displayProfile-Kits (Firma Display Systems Biotech, Vista, USA) nach Angaben des Herstellers durchgeführt.

**PCR-Amplifikation der cDNAs.** Nach Ligation werden in parallelen Reaktionsansätzen je 0.2µl der cDNA mit dem markierten o-Extension-Primer und einem der 64 Eu-Primer amplifziert und die Reaktionsansätze in einem 6% igen Tris-Taurin-EDTA-Polyacrylamidgel elektrophoretisch aufgetrennt. Das Gel wurde anschließend für eine Stunde bei 80°C getrocknet und über Nacht auf einem BASIII-Detektionsscreen (Fa. Fuji) exponiert. Zur Auswertung wurde der STORM-Phosphor-Imager (Fa. Molecular Dynamics) unter Verwendung der lmageQuant-Software verwendet. Die Autoradiographie-Daten wurden im gleichen Größenmaßstab auf Folie gedruckt, die dann zum Auschneiden der Fragmente verwendet wurde.

**Reamplifikation der DDRT-PCR-Fragmente.** Differentiell regulierte PCR-Banden wurden mit einem Skalpell aus dem Gel ausgeschnitten und durch 15minütiges Kochen In 50 µl Tris-EDTA-Puffer aus dem Gelstück eluiert mit Hilfe des displayProfile-Kits (Firma Display Systems Biotech, Vista, USA) nach Angaben des Herstellers durch PCR reamplifiziert. Das Temperaturprofil entsprach der originalen PCR-Reaktion (s.o.). Die PCR-Ansätze anschließend mit 10µl Probenpuffer (0.25% Bromphenolblau, 0.25% Xylenecyanol FF, 30 % Glycerin) versetzt und in einem 3%Igen TAE-Agarosegel mit 10µg/ml Ethidiumbromid gelelektrophoretisch aufgetrennt und PCR-Produkte der erwarteten Größe aus dem Gel ausgeschnitten.

**Klonierung In TA-Klonierungsvektoren.** Die ausgeschnittenen Fragmente wurden mit dem Qiaquick-Gel-Extraktionskit (Fa. Qiagen) nach Angaben des Herstellers aufgereinigt, bis zur Trockne eingeengt und in 5µl bidest. Wasser aufgenommen. Anschließend wurden sie in den pCRII-TOPO-Vektor mittels des TOPO TA Cloning Kits (Fa. Invitrogen) nach Angaben des Herstellers ligiert und in TOP10F'-E.coli-Zellen transformiert. Der Transformationsansatz wurde auf LB-Agar-Platten mit 100µg/ml Ampicillin ausgestrichen, die vorher mit 50µl 2% X-Gal (Fa. Sigma) und 50µl Isopropylthiogalactosid (Fa. Sigma) behandelt wurden. Die nach 15 stündiger Inkubation bei 37°C erhaltenen weißen Bakterien-Klone wurden in 5 ml LB-Flüssigmedium mit 100µg/ml Ampicillin (100µg/ml) überführt und über Nacht bei 37°C unter Schütteln inkubiert. Aus diesen Kulturen wurde Plasmid-DNA unter Verwendung des Qiagen-Spin-Miniprep-Kits (Fa. Qiagen) nach Angaben des Herstellers isoliert und je 5µl der Plasmid-DNA durch EcoRI-Restriktionsverdau und anschließende TAE-Agarose-Gelelektrophorese charakterisiert.

**Sequenzanalyse**. Hierbei wurden je 500 ng der Plasmid-DNA mit dem T7-PCR-Primer unter Verwendung des Dye Terminator Cycle Sequencing Kits (Fa. Perkin-Elmer) nach Angaben des Herstellers sequenziert und die Reaktionen mittels des automatischen Sequencers ABI 370 (Fa. Applied Biosystems Inc.) analysiert. Die DNA-Sequenzen wurden unter Verwendung der bioSCOUT-Software (Fa. LION, Heidelberg) mit den Gendatenbanken abgeglichen.

### C) Ergebnis

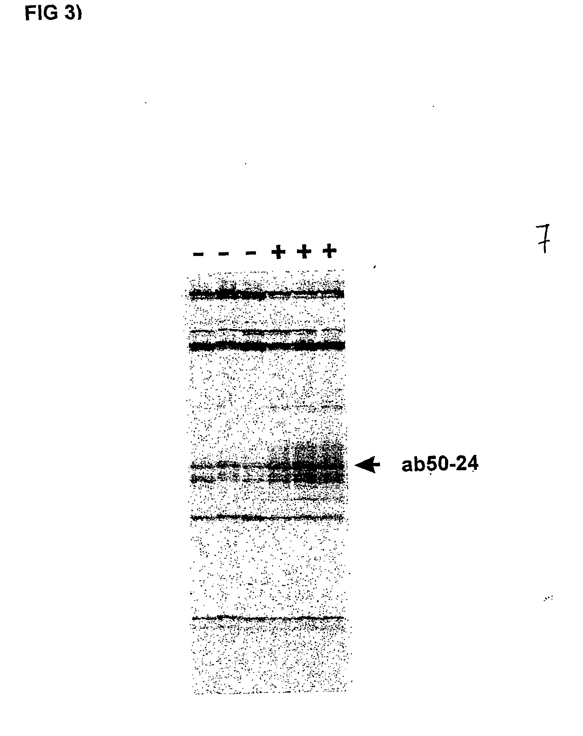

In Abbildung 3 ist ein entsprechendes Autoradiogramm gezeigt. Das Autoradiogramm zeigt die Auftrennung von PCR-Fragmenten die durch Amplifikation verschiedener cDNAs entstanden sind. Die cDNAs wurden durch reverse Transkription aus Gesamt-RNA aus L5-Spinalganglien synthetisiert. Die Gesamt-RNA wurde aus Kontrolltieren (-) und CFAbehandelten Tieren (+) isoliert. Mit einem Pfeil ist das Fragment ab50-24 gekennzeichnet, das, wie mittels der RFDD-Methode gezeigt, eine Hochregulation aufweist. Das Fragment ab50-24 zeigt eine hochsignifikante Homologie zur cDNA-Sequenz AC-Nr. AAT42064 von hBNPI (s. Abb 1c). Damit ist nachgewiesen, daß BNPI unter den Bedingungen einer CFA-Behandlung stärker exprimiert wird.

### Beispiel 2

### Identifizierung schmerzregulierter Gene über immunzytochemische Färbung

Es wurde folgendes Vorgehen gewählt:

Als Ausgangspunkt für die Isolierung schmerzregulierter Gene wurde das sogenannte CIA-Model (Collagen-Induzierte Arthritis) an der Ratte gewählt, bei dem Collagen injiziert wird, um in der Ratte Arthritis auszulösen.

Das Vorgehen entsprach dem bei Persson S., Schäfer MK-H., Nohr D., Ekström G., Post C., Nyberg F. und Weihe E. (1994), Neuroscience 63; 313-326 bzw. Nohr D., Schäfer MK-H., Romeo H., Persson S., Nyberg F. Post C. und Weihe E. (1999), Neuroscience 93; 759-773 beschriebenen Verfahren.

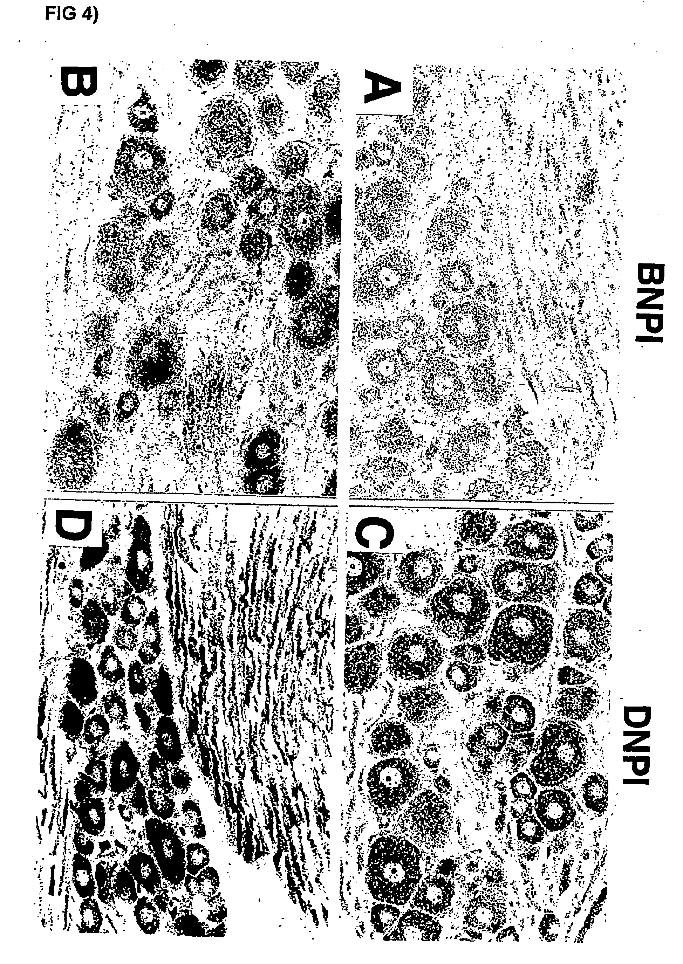

Zur immunhistochemischen Anfärbung wurden polyklonale Kanichen-Antiseren gegen das rekombinante DNPI- bzw. BNPI-Fusionsprotein verwendet. Es zeigte sich in Abbildung 4, daß die Intensität der DNPI- und BNPI-Immunfärbung im lumbaren Dorsal-Root-Ganglion der arthritischen Ratte (B und D/CIA) im Vergleich zu den Kontrolltieren (A und C/CTLR) zunahm. Zu beachten ist die Zunahme sowohl in den Zellkörpern und der Faseranfärbung bei B im Vergleich zu A und bei C im Vergleich zu D.

### Beispiel 3

### Differentielle Betrachtung der Expression zwischen DNPI und BNPI über immuncytochemische Färbung

Zur immunhistochemischen Anfärbung wurden polyklonale Kaninchen-Antiseren gegen das rekombinante DNPI- bzw. BNPI-Fusionsprotein verwendet Generell wurden Schnitte verschiedener Regionen des ZNS angelegt und die Expression von DNPI mit der von BNPI verglichen.

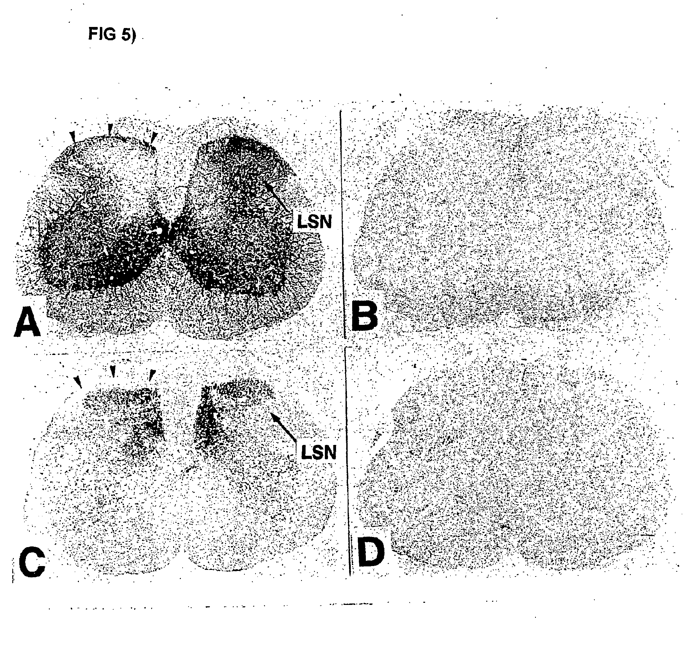

### Beispiel 3a zu Abbildung 5)

Es ist die differentielle Verteilung der Immunreaktivität von BNPI und DNPI im lumbaren Rückenmark der Ratte zu sehen. Die angrenzenden deparafinierten Abschnitte A- bis D sind wie folgt gefärbt:
A = anti-DNPI;
B= anti-DNPI präadsorbiert mit DNPI-Fusionsprotein;
C = anti-BNPI;
D = anti-BNPI präadsorbiert mit BNPI-Fusionsprotein;

Die DNPI- (A) und BNPI- (C) Immunfarbstoffe waren voll mit homologem rekombinanten BNPI- (D) und BNPI- (B) Fusionsprotein präadsorbierbar, was die Spezifität der Immunreaktion beweist.

Bemerkenswert ist das gegenseitig ausschließende Verteilungsmuster von DNPI und BNPI-Immunfärbung im äußeren und tiefen Dorsalhorn. (A;C).Punktierte Immunfärbung von DNPI ist in den synaptischen Endungen des äußeren Dorsalhorns (Lamina 1 und Substantia gelatinosaa) (Pfeil in A), während BNPI Immunreaktivität vollständig fehlt (Pfeile In B). Akkumulation von starker positiver ppunktierter BNPI-Immunfärbung liegt im tieferen Dorsalhom vor, während DNPI-Färbung relativ niedrig ist. DNPI ist präsent in der lateralen spinalen Nukleus (LSN in A), während BNPI völlig fehlt (LSN in C). DNPI ist in der Lamina X .um den zentralen Kanal abundant, während BNPI selten ist. BNPI Immunfärbung ist im lateralen Ventralhorn schwach und gering oder fehlend im medialen Ventralhorn. Durch das ganze Ventralhorn ist punktförmige DNPI-Färbung abundant, etwas weniger im lateralen Horn im Vergleich zum medialen Ventralhorn. Es gibt eine schwache BNPI und DNPI Färbung in einigen Zellkörpern des Ventralhorn Motoneurons, was aber nicht durch die homologen transporter Fusionsproteine präadsorbiert wurde und daher als nichtspezifisch eingestuft wurde.

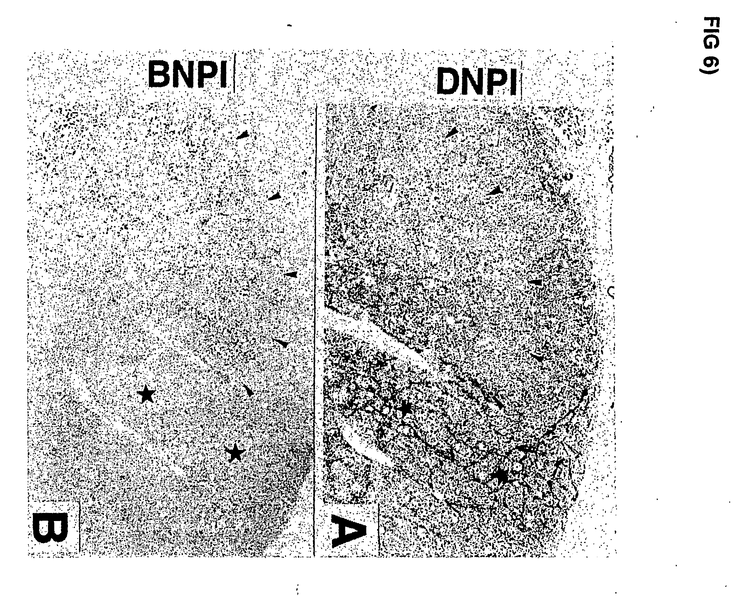

### Beispiel 3b zu Abbildung 6)

Es ist die differentielle Verteilung der Immunreaktivität von BNPI und DNPI im linken lateralen oberflächlichen dorsalen lumbaren Rückenmark (left lateral superficial dorsal lumbar spinal cord) der Ratte zu sehen. A und B jeweils für BNPI (A) und DNPI (B) gefärbt zeigen viele punktförmige Anfärbungen für DNPI, die in der Lamina I und substantia gelatinosa konzentriert sind wo BNPI fast vollständig fehlt. Weiter sind dichte Komplexe von DNPI positiven Punkten im lateralen spinalen Nukleus zu sehen, wo BNPI fast vollständug fehlt. Feine DNPI positive Punkte sind auch in den tieferen dorsalen Horn zu finden, wenn auch mit geringerer Dichte.

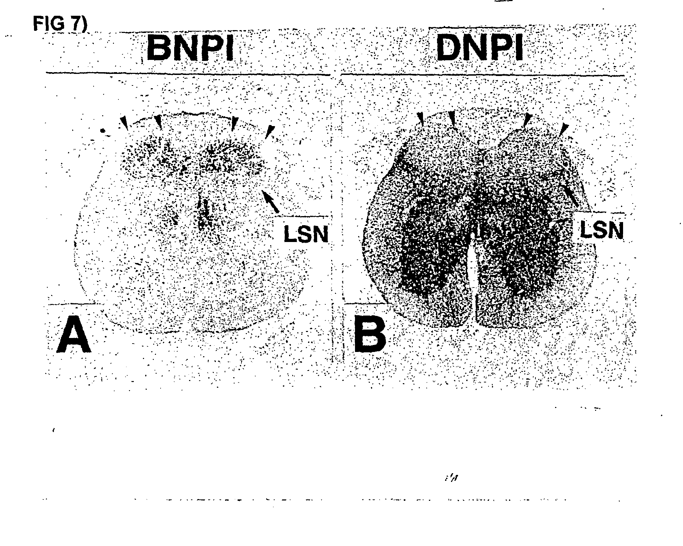

### Beispiel 3c zu Abbildung 7)

Es ist die differentielle Verteilung der Immunreaktivität von BNPI und DNPI im sakralen Rückenmark der Ratte zu sehen. Die angrenzenden Abschnitte A und B jeweils für BNPI (A) und DNPI (B) gefärbt zeigen gegenseitige Exclusions-Zonen punktierter DNPI- und BNPI-Immunfärbung im Dorsalhorn. DNPI ist in der gesamten Grey Matter präsent und ist in den sehr äußeren Schichten des Dorsalhorns konzentriert, wo es es eine schmale Bande an der Grenze zu White Matter bildet. DNPI ist abundant im lateralen spinalen Nukleus und in der Lamina X wie auch in der Lamina VNI und im ganzen ventralen Horn. BNPI ist abundant im tiefen Dorsalhom und selten in Ventralhorn.

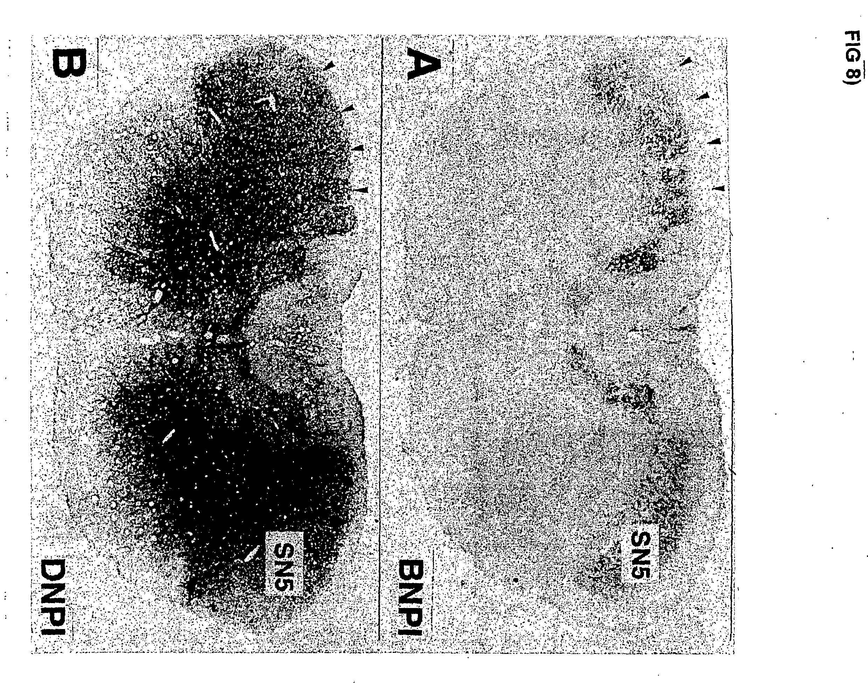

### Beispiel 3d zu Abbildung 8)

Es ist die differentielle Verteilung der Immunreaktivität von BNPI und DNPI in der unteren Medulla oblongate am Übergang zum cervicalen Rückenmark zu sehen. Die angrenzenden Abschnitte A und B jeweils für BNPI (A) und DNPI (B) gefärbt zeigen eine bevorzugte Akkumulation der BNPI-Färbung im medialen Teil des spinalen trigeminalen Nukleus und in dem mittleren und unteren Teil der dorsalen Medulla. es ist nur eine sehr schwache Färbung mit BNPI in den ventralen Medulla zu sehen. DNPI ist abundant in Grey Matter der Medulla. DNPI-Färbung überlappt mit der BNPI-Färbung im inneren spinalen Nucleus V. Es ist zu beachten, daß BNPI auch im oberen spinalen trigeminalen Nukleus, der gleich der spinalen substantia gelatinosa ist, zu finden ist. DNPI-Färbung ist in Gebieten schwächer, in denen BNPI präsent ist, schwächer als in Gebieten, wo BNPI niedrig ist oder fehlt. Einige wenige BNPI Punkte sind im ventralen Grey Motor Gebiet zu sehen.

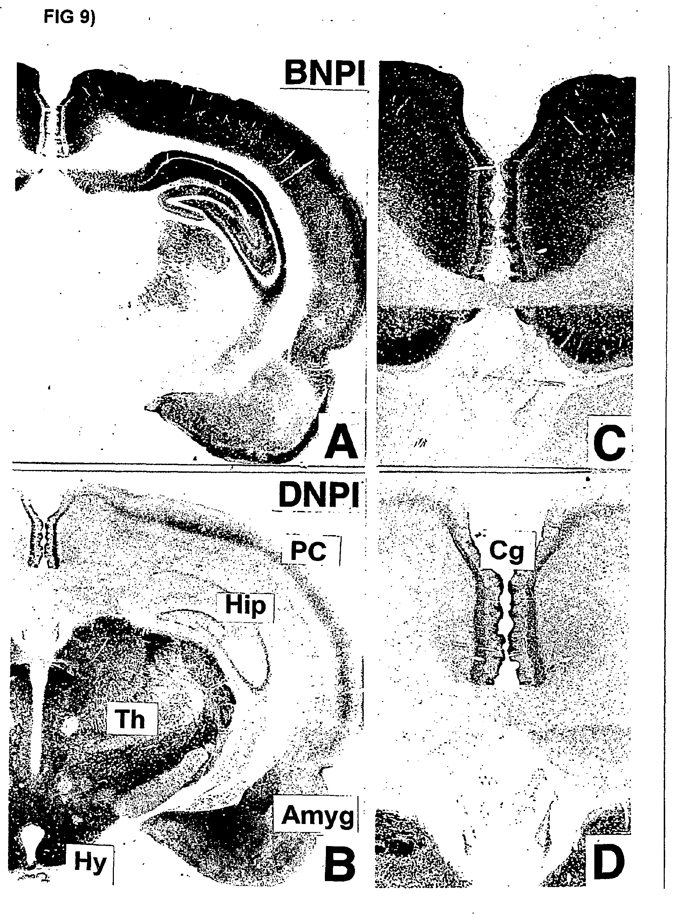

### Beispiel 3e zu Abbildung 9)

Komplementär differentielle Verteilung von DNPI und BNPI Imunreaktivität in 2 Folgeschnitten des Rattenhirns in schmerzrelevanten Hirnregionen wie sensorischer parietaler Cortex; cingulärer Cortex, Thalamus, Corpus amygdaloideum sowie auch Hypothalamus. DNPI ist im Cortex in den granulären sensorischen Schichten insbesondere in Lamina IV konzentriert; BNPI ist Im Cortex abundant aber schwächer in der Lamina IV als in anderen Laminae. Im cingulären Cortex (C vs D als Hochvergrößerung) ist die Verteilung von DNPI und BNPI komplementär wechselseitig excludierend bzw. reziprok in der Dichte der jeweiligen Synapsen. DNPI überwiegt im Thalamus eindeutig über BNPI, BNPI ist im Hypothalamus spärlich, DNPI abundant. Abundantes BNPI überwiegt im Hypocampus über spärliches DNPI bei wechselseitig komplementärer Verteilung.
Thalamus = Th,
Amygdala = Amyg.
Hippocampus = Hip,
Cinguläerer Cortex = Cg,
Hypothalamus = Hy,
parietaler Cortex = PC.

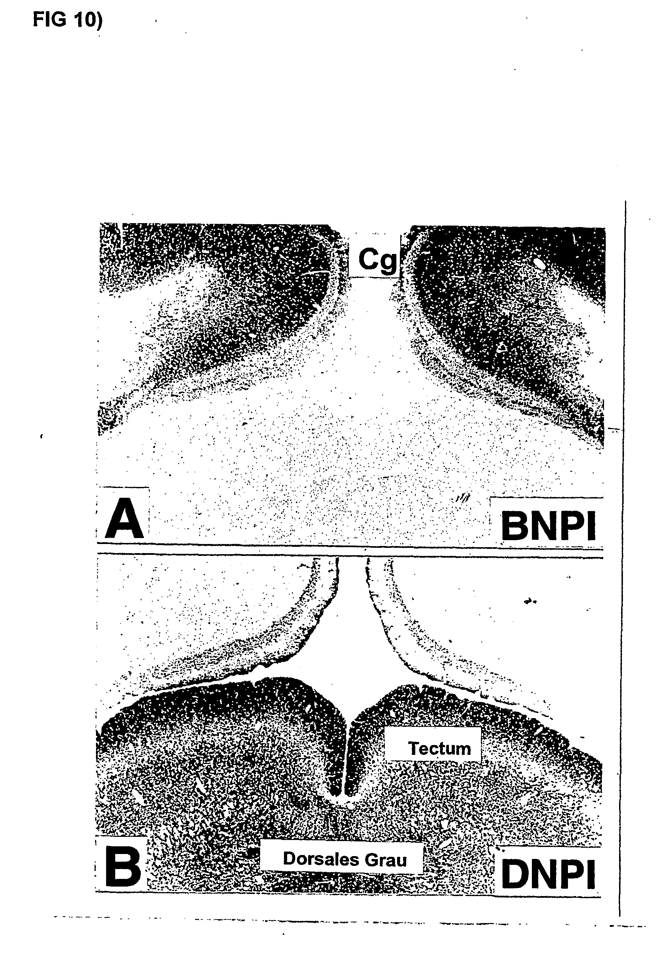

### Beispiel 3f zu Abbildung 10)

Komplementär differentielle Verteilung von DNPI und BNPI Immunreaktivität in schmerzrelevanten Hirnregionen wie cingulärer Cortex (Cg) und Tectum sowie dorsalem periaquäductalen Grau. DNPI Dominanz Im Tectum und dorsalem Grau. Folgeschnitte eines Rattenhirns durch das obere Mesencephalon.

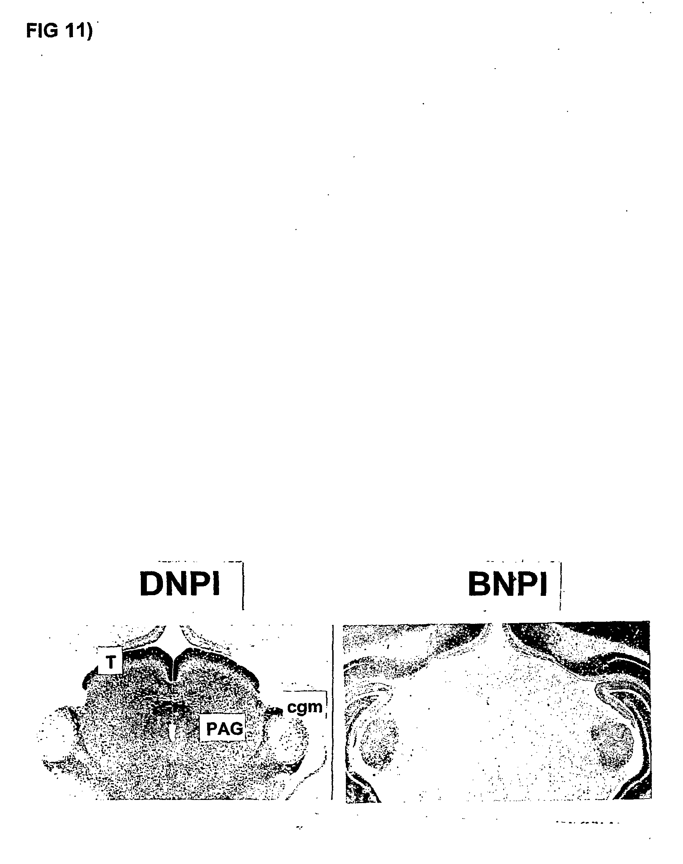

### Beispiel 3g zu Abbildung 11)

Komplementär differentielle Verteilung von DNPI und BNPI Immunreaktivität In schmerzrelevanten Hirnregionen wie Tectum (T) sowie periaquäductalen Grau (PAG). DNPI Dominanz im Tectum und dorsalem Grau. Man notiere differentielle Verteilung von DNPI und BNPI im corpus geniculatum mediale (cgm) der Hörbahn. Folgeschnitte eines Rattenhirns durch das obere Mesencephalon; Ebene colliculus superior.

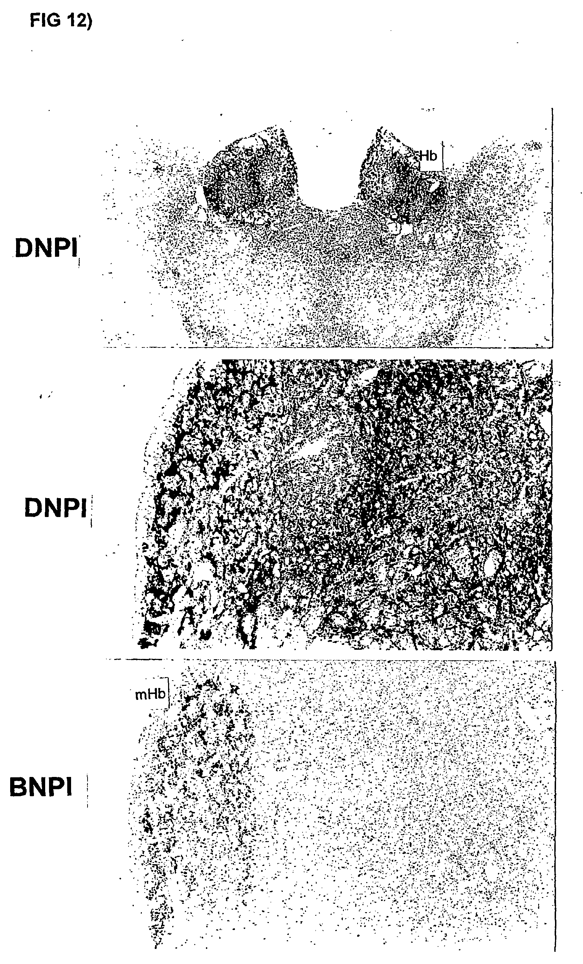

### Beispiel 3h zu Abbildung 12)

Abundanz von DNPI über BNPI in den Habenulae (Hb). DNPI ist präsent im gesamten Habenularkomplex (niedrige Vergrößerung, obere Abbildung; hohe Vergrößerung, mittlere Abb.). BNPI ist nur im medialen Habenularkem (mHb untere Abb., Folgeschnitt zu mittlerer Abbildung).

### Analyse zu Beispiel 3 allgemein:

Die differentielle Verteilung von BNPI und DNPI in Synapsen des primärafferenten, spinalen trigeminalen und supraspinalen nociceptiven Systems ist eine starke Evidenz für eine selektive Beeinftußbarkeit nociceptiver Funktionen durch selektive Modulation des DNPI bzw. BNPIvermittelten Glutamat-Transports. Die Verteilung von BNPI im tiefen Hinterhorn ist eine Indikation für eine präferentielle Rolle des BNPI bei glutamaterg getriebenen neuropathischen Schmerzen.

Die präferentielle Verteilung von DNPI in der lamina 1 und der substantia gelatinosa des spinalen und trigeminalen nociceptiven Systems spricht für eine proimäre und präferentielle Rolle von DNPI bei Entzündungsschmerz. Da DNPI-Synapsen auch im tieferen Hinterhorn liegen, ist DNPI auch ein Kandidat bei neuropathischem Schmerz.

BNPI ist ein präferentieller Kandidat für Allodynie und mechanische Hyperalgesie bei Entzündungsschmerz. Glutamat-vermittelter Aß-Input auf spinale nociceptive Projektions-Neurone konvergierend könnte ein wesentlicher Mechanismus für chronischen tiefen muskuloskelettalen Schmerz sein, ein Hauptproblem des chronischen Schmerzes.

Die Präsenz in visceralen sakralen Afferenzen deutet auf Indikation bei viszeralem Schmerz.

Trigeminale Afferenz: Migräne, Cluster-Kopfschmerz, Trigeminus-Neuralgie.

### Beispiel 4:

### Durchführung des Screeningverfahrens mit Messung der Bindung über die Verdrängung eines radioaktiv markierten Liganden

Ein Nukleinsäureabschnitt, der für BNPI kodiert, wird in einem Expressionsvektor kloniert, der eine konstitutive Expression (z.B. CMV-Promoter) oder eine induzierbare Expression in eukaryoten Zellen erlaubt. Die DNA wird mit geeignetem Transfektionsverfahren, z.B. mit Lipofectamin (Fa. Roche Diagnostics) in eukaryotischen Zellen (z.B. CHO-Zellen, HEK293-Zellen oder NIH-3T3-Zellen) hineingebracht. Die Zellen werden in Anwesenheit eines Selektionsreagenzen (z.B. Zeocin, Hygromycin oder Neomycin) kultiviert so daß nur die Zellen überleben, die das DNA-Konstrukt aufgenommen haben, und bei länger andauernder Selektion, auch in das Genom inkorporiert haben.
Ausgehend von diesen Zellen werden Membranfraktionen gewonnen, die BNPI in großer Menge enthalten und für einen Bindungsassay verwendet werden können. Dieser besteht aus 1.) dem BNPI enthaltenden Membranen 2.) einem radioaktiv markierten Liganden 3.) einem Bindungspuffer (z.B. 50 mM HEPES pH 7.4, 1 mM EDTA) und dem auf Bindung zu untersuchenden Liganden. Nach Inkubation der oben genannten Reaktionsgemische (für z.B. 30-60 min) bei einer geeigneten Temperatur (meistens Raumtemperatur) werden die nichtgebundenen radioaktiven Ligandenmoleküle abfiltriert. Die Restmenge an gebundenem Liganden wird nach Zugabe eines Szintillationscocktails in einem β-Counter (z.B. Trilux, Fa. Wallac) vermessen. Zeigt die Testsubstanz eine Bindung an BMPI, so wird dies als verringerter radioaktiver Einbau detektiert. Dieses Verfahren wird geeignetermaßen so miniaturisiert, daß es in (96-, 384- oder 1536-well) Mikrotiterplatten durchgeführt werden kann, um dieses Verfahren mittels eines Roboters im sogenannten Hightroughput-Screening (HTS)-Verfahren durchzuführen.

### Beispiel 5:

### Durchführung des erfindungsgemäßen Screeningverfahrens mit BNPI und Messung der durch die Bindung der Substanz veränderten funktionellen Parameter

Ein Nukleinsäureabschnitt, der für BNPI kodiert, wird in einem Expressionsvektor kloniert, der eine induzierbare Expression in Prokaryonten, wie z.B. E.coli erlaubt. Hierbei wird der Nukleinsäureabschnitt so modifiziert, daß er als Fusionsprotein mit einer zusätzlichen N- oder C-terminalen Aminosäuresequenz exprimiert wird. Diese Sequenz sollte bei unveränderter Funktion des BNPI eine Aufreinigung über ein spezifisches Verfahren erlauben, z.B. Glutathion S-Transferasefragment, das über Bindung an Glutathion eine Isolierung aus dem Proteingemisch erlaubt. Nach Transfektion der Bakterien, Induktion des Genes (z.B. mit IPTG beim Iac-Promoter) und Aufschließen der Bakterien werden die Fusionsproteine aufgereinigt und in einem in vitro-Kinase Experiment eingesetzt. Hierbei werden 5 µg Protein bei 30°C für 30 Minuten in 50 µl Kinasepuffer (20 mM PIPES, pH 7.0, 5 mM MnCl₂, 7 mM β-Mercaptoethanol, 0.4 mM Spermin, 10 mM rATP) ergänzt mit 10 µCi [γ³²P] ATP. Als Substrate werden gereinigtes Histon H1-Protein (Fa. Sigma) oder bakteriell exprimiertes GST-NFATc1-fusionsprotein hinzugegeben. Nach der Inkubationszeit wird das nicht-inkorpierte [γ-³²P] ATP abfiltriert und die Menge an eingebautem ³²Phosphat durch β-Szintillation (Trilux, Fa. Wallac) bestimmt. In einem Experiment zum Aufspüren neuer BNPI-Inhibitoren werden in diesem Ansatz die Testsubstanzen mitinkubiert und eine Abnahme der ³²P-Inkorporation als Indikator für einen Inhibitor benutzt. Dieses Verfahren wird geeignetermaßen so miniaturisiert, daß es in (96-, 384- oder 1536-well) Mikrotiterplatten durchgeführt werden kann, um dieses Verfahren mittels eines Roboters im sogenannten Hightroughput-Screening (HTS)-Verfahren durchzuführen.

### Beispiel 6:

### Durchführung des erfindungsgemäßen Screeningverfahrens mit DNPI und Messung der durch die Bindung der Substanz veränderten funktionellen Parameter

Das Verfahren wird wie in Beispiel 5 beschrieben durchgeführt mit der Ausnahme, daß statt eines Nukleinsäureabschnittes, der für BNPI kodiert, ein Nukleinsäureabschnitt eingesetzt wurde, der für DNPI kodiert

### Beispiel 7:

### Beispiel für ein Arzneimittel zur Schmerzbehandlung enthaltend eine durch ein erfindungsgemäßes Verfahren identifizierte Verbindung-Tablettenformulierung

Tabletten können durch direktes Verpressen von Mischungen der durch ein erfindungsgemäßes Verfahren identifizierten Verbindung mit entprechenden Hilfsstoffen oder durch Verpressen von verbindungshaltigen Granulaten (mit gegebenenfalls weiteren Hilfsstoffen) hergestellt werden. Die Granulate können dabei entweder durch Feuchtgranulation mit z.B. wäßrigen Granullerflüssigkeiten und anschließender Trocknung dieser Granulate oder durch Trockengranulation z.B. über Kompaktierung hergestellt werden.
■ Direktverpressung

| | | |
|---|---|---|
| z.B. pro Tablette: | 25 mg | einer durch ein erfindungsgemäßes Verfahren identifizierten Verbindung |
| | 271 mg | LudipressTM (Granulat zur Direkttabtettierung aus Lactose monohydrat, Povidon K30 und Crospovidon) |
| | 4 mg | Magnesiumstearat |
| | 300 mg | Gesamt |

Homogene Mischung des Wirkstoffes mit den Hilfsstoffen herstellen und diese auf einer Tablettenpresse zu Tabletten mit einem Ø von 10 mm verpressen.
■ Trockengranulation

| | | |
|---|---|---|
| z.B. pro Tablette: | 25 mg | einer durch ein erfindungsgemäßes Verfahren identifizierten Verbindung |
| | 166 mg | Microcristalline Cellulose |
| | 80 mg | Niedrig substituierte Hydroxypropylcellulose (I-HPC LH 11^{™}) |
| | 5 mg | Hochdisperses Siliziumdioxid |
| | 4 mg | Magnesiumstearat |
| | 280 mg | Gesamt |

Homogene Mischung der Verbindung mit der Mikrokristallinen Cellulose und der I-HPC herstellen und diese Kopaktieren. Nach dem Sieben der Komprimate wird das entstandene Granulat mit Magnesiumstearat und Siliziumdioxid gemischt und auf einer Tablettenpresse zu Tabletten mit einem Ø von 9 mm verpreßt.
■ Feuchtgranulation

| | | |
|---|---|---|
| z.B. pro Tablette: | 25 mg | einer durch ein erfindungsgemäßes Verfahren identifizierten Verbindung |
| | 205 mg | Mikrokristalline Cellulose |
| | 6 mg | Povidon K30 |
| | 10 mg | Crospovidon |
| | 4 mg | Magnesiumstearat |
| | 250 mg | Gesamt |

Homogene Mischung der Verbindung mit der Mikrokristallinen Cellulose und dem Crospovidon herstellen und diese in einem Granulator mit einer wäßrigen Lösung des Povidons granulieren. Das feuchte Granulat wird anschließend nachgranuliert und nach der Trocknung im Trockenschrank (50°C) 10 h getrocknet. Das trockene Granulat wird mit dem Magnesiumstearat zusammen gesiebt, endgemischt und auf einer Tablettenpresse zu Tabletten mit einem Ø von 8 mm verpreßt.

### Beispiel 8:

### Beispiel für ein Arzneimittel zur Schmerzbehandlung enthaltend eine durch ein erfindungsgemäßes Verfahren identifizierten Verbindung - parenterale Lösung

1 g einer durch ein erfindungsgemäßes Verfahren identifizierten Verbindung wird in 1 l Wasser für Injektionszwecke bei Raumtemperatur gelöst und anschließend durch Zugabe von NaCl (Natriumchlorid) auf isotone Bedingungen eingestellt.

### Literatur:

Aihara Y, Mashima H. Onda H. Hisano Setsuji, Kasuya H., Hori T. Yamada S., Tomura H. Yamada Y., Inoue I., Kojima I. und Takeda J. (2000), J. Neurochem. 74: 2622 - 2625
Akopian AN, Sivilotti L & Wood JN (1995) Nature 379: 257-262
Ausubel FM, Brent R, Kingdton RE, Moore DD, Seidman JG, Smith JA & Struhl K eds.(1190) Current protocols in molecular biology. John Wiley &Sons, Inc. New York, NY.
Baba H, Doubell TP, Woolf CJ 1999: Peripheral inflammation facilitates Aβ fiber-mediated synaptic input to the substantia gelatinosa of the adult rat spinal cord. J Neurosci 19: 859-867
Bauer D, Müller H, Reich J, Riedel H, Ahrenkiel V, Warthoe P & Strauss M (1993): Identification of differentially expressed mRNA species by an improved display technique (DDRT-PCR) Nucl Acids Res 21: 4272-4280.
Bonini A, Anderson SM, Steiner DF (1997) Molecular cloning and tissue expression of a novel orphan G Protein-coupled receptor from rat lung. Biochem Biophys Res Comm 234: 190-193.
Chih-Cheng et al., (1995): A P2X prinoceptor expressed by a subset of sensory neurons. Nature 377:428-432
Corderre TJ, Katz J, Vaccarino AL, Melzack R (1993): Contribution of central plasticity to pathological pain: review of clinical and experimental evidence. Pain 52: 259-285.
Dickenson (1995) Novel pharmacological targets in the treatment of pain. Pain Rev., **2**,1-12.
Dubuisson et al., 1997 Pain, 4:161-174.
Feng Y & Gregor P (1997) Cloning of a novel member of the G Protein-coupled receptor family related to peptide receptors. Biochem Biophys Res Comm 231: 651-654.
Furukawa T, Yang Y, Nakamoto B, Stamatoyannopoulos G, Papayannopoulou T (1996): Identification of new genes expressed in a human erythroleukemia cell line. Bloods Cell Mol & Dis 22:11-22.
Gunasekar PG, Kanthasamy, AG, Borowitz JL, Isom GE 1995: NMDA receptor activation produces concurrent generation of nitric oxide and reactive oxygen species: implication for cell death. J Neurochem 65: 2016-2021.
Hawes BE, Fried S, Yao X, Weig B, Graziano MP 1998: Nociceptin (ORL1) and µ-Opioid receptors mediate mitogen-activated protein kinase activation in CHO cells through a Gi-coupled signaling pathway: evidence for distinct mechanisms of agonist-mediated desensitization. J Neurochem 71: 1024-1033.
Hubank M & Schatz DG (1994): Identifying differences in mRNA expression by representational difference analysis of cDNA. Nucl Acids Res 22: 5640-5648.
Klußmann S et al., 1996: Nature Biotechnology 14: 1112-1115.
Li L-Y & Chang K-J 1996: The stimulatory effect of opioids on mitogen-activated protein kinase in chinese hamster ovary cells transfected to express µ-opioid receptors. Mol Pharm 50:599-602.
Liang P & Pardee AB 1992: Differential Display of eukaryotic messenger RNA by means of the polymerase chain reaction. Science 257:967-971.
Methner A, Hermey G, Schinke B, Hermanns-Borgmeyer I (1997): A novel G Protein-coupled receptor with homology to neuropeptide and chemoattractant receptors expressed during bone development. Biochem Biophys Res Comm 233: 336-342.
Mohit AA, Martin JH & Miller CA 1995: p493F12 Kinase: a novel MAP kinase expressed in a subset of neurons in the human nervous system. Neuron 14: 67-78.
Poirier GM-C, Pyati J, Wan JS, Erlander MG 1997: Screening differentially expressed cDNA clones obtained by differential display using amplified RNA. Nucleic Acids Research 25: 913-914.
Sambrook J, Fritsch EF & Maniatis T 1989: Molecular Cloning: A Laboratory Manual. Second Edition. Cold Spring Harbor Laboratory Press, Cold Spring Harbor.
Sompayrac L, Jane S, Bum TC, Tenen DG & Danna KJ 1995: Overcoming limitations of the mRNA differential display technique. Nucleic Acids Research 23: 4738-4739.
Tal M 1996: A novel antioxidant alleviates heat hyperalgesia in rats with an experimental painful neuropathy. Neurreport 7: 1382-1384.
Tölle TR (1997): Chronischer Schmerz. In: Klinische Neurobiologie, Herdergen T, Tölle TR, Bähr M (Hrsg.): S. 307-336; Spektrum Verlag, Heidelberg.
U.S.Patent 5.262.311
Velculescu VE, Zhang L, Vogelstein B, Kinzler KW (1995): Serial analysis of gene expression. Science 270: 484-487.
Wan JS, Sharp JS et al. (1996): Cloning differentially expressed mRNAs Nature Biotech 14:1685-1691.
Watson JB & Margulies JE (1993) Differential cDNA screening strategies to identify novel stage-specific proteins in the developing mammalian brain. Dev Neurosci 15: 77-86.
Wilks AF (1989) Two putative protein-tyrosine kinases identified by application of the polymerase chain reaction. Poc Natl Acad Sci USA 86: 1603-1607.
WO96/34288
Woolf CJ, Shortland P, Coggeshall RE 1992: Peripheral nerve injury triggers central sprouting of myelinated afferents. Nature 355:75-78.
Zimmermann, M & Herdegen, T (1996): Plasticity of the nervous system at the systemic, cellular and molecular levels: a mechanism of chronic pain and hyperalgesia. Progr Brain Res 110: 233-259

## Patentansprüche

1. Verfahren zur Auffindung schmerzregulierender Substanzen umfassend folgende Verfahrensschritte:
(a) Inkubation einer zu testenden Substanz unter geeigneten Bedingungen mit einem Biomolekül aus Gruppe I: dem Protein BNPI oder DNPI und/oder einem Protein gemäss einer der Abbildungen 1b), 1d), 1f), 1h), 2b), 2d) oder 2f) und/oder einem zu einem dieser vorgenannten Proteine zu mindestens 90 % ähnliches Protein und/oder einem Protein, für das ein Polynukleotid gemäss einer der Abbildungen 1a), 1c), 1e), 1g), 2a), 2c) oder 2e) oder ein dazu zu mindestens 90 % ähnliches Polynukleotid kodiert, und/oder einem Protein, das durch eine Nukleinsäure kodiert wird, die unter stringenten Bedingungen an ein Polynukleotid gemäss einer der Abbildungen 1 a), 1 c), 1 e), 1 g), 2a), 2c) oder 2e) oder deren Antisense Polynukleotide binden, und/oder einer Zelle und/oder einer Präparation aus einer solchen Zelle, die mindestens eines der vorgenannten Proteine synthetisiert hat,
(b) Messung der Bindung der Testsubstanz an dem von der Zelle synthetisierten Protein oder Messung mindestens eines der durch die Bindung der Testsubstanz an das Protein veränderten funktionellen Parameter über Messung der Regulation, Hemmung und/oder Aktivierung von Rezeptoren, lonenkanälen und/oder Enzymen, insbesondere über Messung der Veränderung der Genexpression, des Ionenmilieus, des pH oder des Membranpotentials, über Veränderung der Enzymaktivität oder der Konzentration der 2^{nd} messenger, oder über Messung der Bindung über die Verdrängung eines bekannten markierten Liganden des Proteins und/oder über die daran gebundene Aktivität einer markierten Testsubstanz.

2. Verfahren gemäss Anspruch 1, **dadurch gekennzeichnet, dass** die Zelle vor dem Schritt (a) gentechnisch manipuliert wird.

3. Verfahren gemäss Anspruch 2, **dadurch gekennzeichnet, dass** die gentechnische Manipulation die Messung mindestens eines der durch die Testsubstanz veränderten funktionellen Parameter erlaubt.

4. Verfahren gemäss Anspruch 3, **dadurch gekennzeichnet, dass** durch die gentechnische Manipulation eine in der Zelle nicht endogen exprimierte Form eines G-Proteins exprimiert oder ein Reportergen eingeführt wird.

5. Verfahren gemäss einem der Ansprüche 2-4, **dadurch gekennzeichnet, dass** die Zelle gentechnisch so manipuliert wird, dass die Zelle mindestens ein Polynukleotid gemäss einer der Abbildungen 1 a), 1 c), 1 e), 1 g), 2a), 2c) oder 2e) oder ein dazu zu mindestens 90 % ähnliches Polynukleotid enthält.

6. Verfahren gemäss Anspruch 5, **dadurch gekennzeichnet, dass** das Polynukleotid in einem rekombinanten DNA-Konstrukt enthalten ist.

7. Verfahren gemäss einem der Ansprüche 2 bis 6, **dadurch gekennzeichnet, dass** die Zelle nach der gentechnischen Manipulation gemäss Anspruch 2 und vor dem Schritt (a) gemäss Anspruch 1 unter Bedingungen, die eine Expression erlauben, kultiviert wird, gegebenenfalls unter Selektionsdruck.

8. Verfahren gemäss einem der Ansprüche 1 bis 7, **dadurch gekennzeichnet, dass** die Zelle eine Amphibienzelle, Bakterienzelle, Hefezelle, Insektenzelle oder eine immortalisierte oder native Säugetierzelle ist.

9. Verfahren gemäss einem der Ansprüche 1 bis 8, bei dem ein erstes Verfahren gemäss einem der Ansprüche 1 bis 8 mit einem zweiten Verfahren gemäss einem der Ansprüche 1 bis 8 derart gekoppelt wird, dass die Messwerte und Ergebnisse des ersten Verfahrens hinsichtlich der zu messenden Substanz mit den Messwerten und Ergebnissen des zweiten Verfahrens hinsichtlich der zu messenden Substanz verglichen werden, **dadurch gekennzeichnet, dass** in einem der zwei Verfahren, im folgenden Hauptverfahren genannt, im Schritt (a) die zu testende Substanz
entweder
mit einem Biomolekül aus Gruppe II: dem Protein BNPI und/oder einem Protein gemäss einer der Abbildungen 1b), 1d), 1f) oder 1h) und/oder einem zu einem dieser vorgenannten Proteine zu mindestens 90 % ähnlichen Protein und/oder einem Protein, für das ein Polynukleotid gemäss einer der Abbildungen 1a), 1c), 1e) oder 1g) oder ein dazu zu mindestens 90 % ähnlichen Polynukleotid kodiert, und/oder einem Protein, das durch eine Nukleinsäure kodiert wird, die unter stringenten Bedingungen an ein Polynukleotid gemäss einer der Abbildungen 1a), 1c), 1e) oder 1g) oder deren Antisense Polynukleotide bindet und/oder einer Zelle und/oder einer Präparation aus einer solchen Zelle, die mindestens eines der vorgenannten Proteine synthetisiert hat,
oder
mit einem Biomolekül aus Gruppe III : dem Protein DNPI und/oder einem Protein gemäss einer der Abbildungen 2b), 2d) oder 2f) und/oder einem zu einem dieser vorgenannten Proteine zu mindestens 90 % ähnlichen Protein und/oder einem Protein, für das ein Polynukleotid gemäss einer der Abbildungen 2a), 2c) oder 2e) oder ein dazu zu mindestens 90 % ähnliches Polynukleotid kodiert, und/oder einem Protein, das durch eine Nukleinsäure kodiert wird, die unter stringenten Bedingungen an ein Polynukleotid gemäss einer der Abbildungen 2a), 2c) oder 2e) oder deren Antisense Polynukleotide bindet und/oder einer Zelle und/oder einer Präparation aus einer solchen Zelle, die mindestens eines der vorgenannten Proteine synthetisiert hat, inkubiert wird,
und
dass im anderen der zwei Verfahren, im folgenden Nebenverfahren genannt, im Schritt (a) die zu testende Substanz mit einem Biomolekül aus der Gruppe I oder mit einem Biomolekül aus derjenigen Gruppe ausgewählt aus Gruppe II und Gruppe III inkubiert wird, aus der das Biomolekül, mit der die Substanz im Hauptverfahren inkubiert wird, nicht ausgewählt ist.

10. Verfahren gemäss einem der Ansprüche 1 bis 9, **dadurch gekennzeichnet, dass** der durch die aufzufindende Substanz regulierte Schmerz ausgewählt ist aus:
chronischem Schmerz, insbesondere muskuloskelettalem Schmerz; neuropathischem Schmerz, insbesondere allodynischem Schmerz, mechanischer Hyperalgesie oder diabetischer Neuropathie; viszeralem Schmerz, cerebralem Schmerz, peripherem Schmerz oder entzündungsbedingtem Schmerz, insbesondere peripherem Entzündungsschmerz; sowie Migräne, Cluster-Kopfschmerz oder den Schmerz bei Trigeminus Neuralgie.

## Claims

1. A method for screening for pain-regulating substances, comprising the following steps:
(a) incubating under suitable conditions a substance to be tested with a biomolecule of Group I: the protein BNPI or DNPI and/or a protein according to any one of Figures 1b), 1d), 1f), 1h), 2b), 2d) or 2f) and/or a protein having a similarity of at least 90% to any one of the aforementioned proteins, and/or a protein encoded by a polynucleotide according to any one of Figures 1a), 1c), 1e), 1g), 2a), 2c) or 2e) or polynucleotide having a similarity of at least 90% thereto, and/or a protein encoded by a nucleic acid which binds to a polynucleotide according to any one of Figures 1a), 1c), 1e), 1g), 2a), 2c) or 2e) or their antisense polynucleotides under stringent conditions, and/or a cell and/or a preparation of such a cell having synthesized at least one of the aforementioned proteins,
(b) measuring the binding of the test substance to the protein synthesized by the cell or measuring at least one of the functional parameters altered by the binding of the test substance to the protein via measuring the regulation, inhibition and/or activation of receptors, ion channels and/or enzymes, in particular via measuring the alteration of the gene expression, of the ion milieu, of the pH or of the membrane potential, via measuring the enzyme activity or the concentration of the second messengers, or via measuring the binding via the displacement of a known labelled ligand of the protein and/or via the activity of a labelled test substance bound thereto.

2. The method according to claim 1, **characterized in that** the cell is genetically manipulated prior to step (a).

3. The method according to claim 2, **characterized in that** the genetic manipulation allows for the measurement of at least one of the functional parameters altered by the test substance.

4. The method according to claim 3, **characterized in that** by the genetic manipulation a form of a G-protein, which is not endogenously expressed in the cell, is expressed or a reporter gene is introduced.

5. The method according to any one of claims 2 to 4, **characterized in that** the cell is genetically manipulated such that the cell contains at least one polynucleotide according to any one of Figures 1a), 1c), 1e), 1g), 2a), 2c) or 2e) or polynucleotide having a similarity of at least 90% thereto.

6. The method according to claim 5, **characterized in that** the polynucleotide is contained in a recombinant DNA construct.

7. The method according to any one of claims 2 to 6, **characterized in that** the cell is cultivated, after the genetic manipulation according to claim 2 and prior to step (a) according to claim 1, under conditions permissive to expression, optionally under selection pressure.

8. The method according to any one of claims 1 to 7, **characterized in that** the cell is an amphibian cell, bacterial cell, yeast cell, insect cell or an immortalized or native mammalian cell.

9. The method according to any one of claims 1 to 8, wherein a first method according to any one of claims 1 to 8 is coupled with a second method according to any one of claims 1 to 8 such that the measurement values and the results of the first method in view of the substance to be measured are compared with the measurement values and results of the second method in view of the substance to be measured, **characterized in that** in one of the two methods, in the following referred to as the main method, in step (a) the substance to be tested is incubated
either
with a biomolecule of Group II: the protein BNPI and/or a protein according to any one of Figures 1b), 1d), 1f) or 1h) and/or a protein having a similarity of at least 90% to any one of the aforementioned proteins, and/or a protein encoded by a nucleic acid according to any one of Figures 1a), 1c), 1e) or 1g) or a polynucleotide having a similarity of at least 90% thereto, and/or a protein encoded by a nucleic acid which binds to polynucleotide according to any one of Figures 1a), 1c), 1e) or 1g) or their antisense polynucleotides under stringent conditions, and/or a cell and/or a preparation of such a cell having synthesized at least one of the aforementioned proteins,
or
with a biomolecule of Group III: the protein DNPI and/or a protein according to any one of Figures 2b), 2d) or 2f) and/or a protein having a similarity of at least 90% to any one of the aforementioned proteins, and/or a protein encoded by a nucleic acid according to any one of Figures 2a), 2c) or 2e) or a polynucleotide having a similarity of at least 90% thereto, and/or a protein encoded by a nucleic acid binding to a polynucleotide according to any one Figures 2a), 2c) or 2e) or their antisense polynucleotides under stringent conditions and/or a cell and/or a preparation of such a cell having synthesized at least one of the aforementioned proteins,
and
that in the other of the two methods, in the following referred to as auxiliary method, in step (a) the substance to be tested is incubated with a biomolecule of Group I or with a biomolecule of that group selected from Group II and Group III from which the biomolecule being used for incubation with the substance in the main method is not selected.

10. The method according to any one of claims 1 to 9, **characterized in that** the pain regulated by the substance to be screened for is selected from:
chronic pain, in particular musculoskeletal pain; neuropathic pain, in particular allodynic pain, mechanical hyperalgesia or diabetic neuropathy, visceral pain, cerebral pain, peripheral pain or inflammation-associated pain, in particular peripheral inflammatory pain; as well as migraine, cluster headache or the pain in trigeminal neuralgia.

## Revendications

1. Méthode de détection de substances régulant la douleur, comprenant les étapes suivantes :
(a) incubation dans des conditions convenables d'une substance à tester, avec une biomolécule du Groupe I : la protéine BNPI ou DNPI et/ou une protéine selon l'une des figures 1b), 1d), 1f), 1h), 2b), 2d) ou 2f) et/ou une protéine semblable à au moins 90 % à l'une des protéines mentionnées ci-dessus et/ou une protéine qui est codée par un polynucléotide selon l'une des figures 1a), 1c), 1e), 1g), 2a), 2c) ou 2e) ou par un polynucléotide qui y est semblable à 90 % au moins, et/ou une protéine qui est codée par un acide nucléique qui se lie dans des conditions stringentes à un polynucléotide selon l'une des figures 1a), 1c), 1e), 1g), 2a), 2c) ou 2e) ou leurs polynucléotides antisens, et/ou une cellule et/ou une préparation d'une telle cellule qui a synthétisé au moins l'une des protéines mentionnées ci-dessus,
(b) mesure de la liaison de la substance testée à la protéine synthétisée par la cellule ou mesure d'au moins l'un des paramètres fonctionnels modifiés par la liaison de la substance testée à la protéine par mesure de la régulation, de l'inhibition et/ou de l'activation de récepteurs, de canaux ioniques et/ou d'enzymes, en particulier par mesure de la variation de l'expression génique, du milieu ionique, du pH ou du potentiel de membrane, d'après la modification de l'activité enzymatique ou de la concentration des seconds messagers, ou par mesure de la liaison d'après le déplacement d'un ligand marqué connu de la protéine et/ou d'après l'activité liée à la protéine d'une substance testée marquée.

2. Méthode suivant la revendication 1, **caractérisée en ce que** la cellule est soumise à une manipulation génétique avant l'étape (a).

3. Méthode suivant la revendication 2, **caractérisée en ce que** la manipulation génétique permet la mesure d'au moins l'un des paramètres fonctionnels modifiés par la substance testée.

4. Méthode suivant la revendication 3, **caractérisée en ce que** la manipulation génétique entraîne l'expression d'une forme d'une protéine G non exprimée de façon endogène dans la cellule ou l'introduction d'un gène rapporteur.

5. Méthode suivant l'une des revendications 2 à 4, **caractérisée en ce que** la cellule est soumise à une manipulation génétique de telle manière qu'elle contienne au moins un polynucléotide selon l'une des figures 1a), 1c), 1e), 1g), 2a), 2c) ou 2e), un polynucléotide qui y soit semblable à 90 % au moins.

6. Méthode suivant la revendication 5, **caractérisée en ce que** le polynucléotide est contenu dans un produit d'assemblage de DNA recombiné.

7. Méthode suivant l'une des revendications 2 à 6, **caractérisée en ce que** la cellule est cultivée après la manipulation génétique selon la revendication 2 et avant l'étape (a) selon la revendication 1 dans des conditions qui permettent une expression, éventuellement sous pression de sélection.

8. Méthode suivant l'une des revendications 1 à 7, **caractérisée en ce que** la cellule est une cellule d'amphibien, une cellule bactérienne, une cellule de levure, une cellule d'insecte ou une cellule immortalisée ou native de mammifère.

9. Méthode suivant l'une des revendications 1 à 8, dans laquelle une première méthode selon l'une des revendications 1 à 8 est couplée à une seconde méthode selon l'une des revendications 1 à 8, de façon telle que les valeurs de mesure et les résultats de la première méthode en ce qui concerne la substance à mesurer sont comparés avec les valeurs de mesure et les résultats de la seconde méthode en ce qui concerne la substance à mesurer, **caractérisée par le fait que** dans l'une des deux méthodes, appelées ci-après méthode principale, dans l'étape (a), la substance à tester est incubée
ou bien
avec une biomolécule du Groupe II, à savoir la protéine BNPI et/ou une protéine selon l'une des figures 1b), 1d), 1f) ou 1h) et/ou une protéine semblable à 90 % au moins à l'une des protéines mentionnées ci-dessus et/ou une protéine codée par un polynucléotide selon l'une des figures 1a), 1c), 1e) ou 1g) ou un polynucléotide qui y est semblable à 90 % au moins, et/ou une protéine qui est codée par un acide nucléique qui se lie dans des conditions stringentes à un polynucléotide selon l'une des figures 1a), 1c), 1e) ou 1g) ou leurs polynucléotides antisens et/ou une cellule et/ou une préparation d'une telle cellule qui a synthétisé au moins l'une des protéines mentionnées ci-dessus,
ou bien
avec une biomolécule du Groupe III, à savoir à la protéine DNPI et/ou une protéine selon l'une des figures 2b), 2d) ou 2f) et/ou une protéine semblable à 90 % au moins à l'une des protéines mentionnées ci-dessus et/ou une protéine codée par un polynucléotide selon l'une des figures 2a), 2c) ou 2e) ou un polynucléotide qui y est semblable à 90 % au moins et/ou une protéine qui est codée par un acide nucléique qui se lie dans des conditions stringentes à un polynucléotide selon l'une des figures 2a), 2c) ou 2e) ou leurs polynucléotides antisens et/ou une cellule et/ou une préparation d'une telle cellule qui a synthétisé au moins l'une des protéines mentionnées ci-dessus,
et en ce que
dans l'autre des deux méthodes, appelées ci-après méthode secondaire, dans l'étape (a), la substance à tester est incubée avec une biomolécule du Groupe I ou avec une biomolécule choisie dans celui des Groupes II et III dans lesquels la biomolécule avec laquelle la substance est incubée dans la méthode principale, n'a pas été choisie.

10. Méthode suivant l'une des revendications 1 à 9, **caractérisée en ce que** la douleur régulée par la substance à détecter est choisie entre :
douleur chronique, en particulier douleur musculo-squelettique ; douleur neuropathique, en particulier douleur allodynique, hyperalgésie mécanique ou neuropathie diabétique ; douleur viscérale, douleur cérébrale, douleur périphérique ou douleur liée à une inflammation, en particulier douleur inflammatoire périphérique ; ainsi que migraine, céphalée vasculaire ou névralgie essentielle du trijumeau.
